(19) Europäisches Patentamt
European Patent Office
Office européen des brevets

(11) **EP 4 395 749 B1**

(12) **EUROPEAN PATENT SPECIFICATION**

(45) Date of publication and mention of the grant of the patent:
**19.11.2025 Bulletin 2025/47**

(21) Application number: **22769279.5**

(22) Date of filing: **29.08.2022**

(51) International Patent Classification (IPC):
***A61K 9/70*** (2006.01) ***A61K 47/12*** (2006.01)
***A61K 31/165*** (2006.01)

(52) Cooperative Patent Classification (CPC):
**A61K 47/12; A61K 9/7061; A61K 9/7069; A61K 31/165**

(86) International application number:
**PCT/EP2022/073922**

(87) International publication number:
**WO 2023/031103 (09.03.2023 Gazette 2023/10)**

(54) **TRANSDERMAL THERAPEUTIC SYSTEM FOR THE TRANSDERMAL ADMINISTRATION OF GUANFACINE COMPRISING GUANFACINE AND A MONO-CARBOXYLIC ACID**

TRANSDERMALES THERAPEUTISCHES SYSTEM ZUR TRANSDERMALEN VERABREICHUNG VON GUANFACIN MIT GUANFACIN UND EINER MONOCARBONSÄURE

SYSTÈME THÉRAPEUTIQUE TRANSDERMIQUE POUR L'ADMINISTRATION TRANSDERMIQUE DE GUANFACINE COMPRENANT DE LA GUANFACINE ET UN ACIDE MONOCARBOXYLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2021 EP 21193889**

(43) Date of publication of application:
**10.07.2024 Bulletin 2024/28**

(73) Proprietor: **LTS Lohmann Therapie-Systeme AG**
**56626 Andernach (DE)**

(72) Inventors:
- **PRINZ, Eva-Marie**
**56575 Weißenthurm (DE)**
- **EMGENBROICH, Marco**
**53359 Rheinbach (DE)**
- **MOHR, Patrick**
**53498 Bad Breisig (DE)**
- **LORSCHEIDT, Stefan**
**53121 Bonn (DE)**

(74) Representative: **Maiwald GmbH**
**Elisenhof**
**Elisenstraße 3**
**80335 München (DE)**

(56) References cited:
**WO-A1-2019/072998** **WO-A1-2019/175096**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD OF THE INVENTION

The present invention relates to a transdermal therapeutic system (TTS) for the transdermal administration of guanfacine to the systemic circulation, and processes of manufacture and uses thereof:

### BACKGROUND OF THE INVENTION

The active agent guanfacine (also known as N-(aminoiminomethyl)-2,6-dichloro-benzeneacetamide, $C_9H_9Cl_2N_3O$, CAS No. 29110-47-2) is a sympatholytic drug used to treat hypertension and attention deficit hyperactivity disorder (ADHD). It is a centrally acting alpha(2)-adrenergic receptor agonist. It has the following chemical formula.

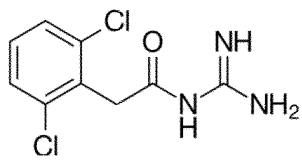

Currently, guanfacine is commercially available, e.g., in the form of immediate or controlled release tablets comprising from 1 to 4 mg guanfacine. The tablets are suitable for once daily administration.

However, the oral administration of active agents has disadvantages, e.g., in terms of patient compliance. Furthermore, it is not possible to quickly terminate the therapy, e.g. in light of overdosing or signs of intolerance, once the prolonged release tablet has been ingested. Patent document WO 2019/072998 discloses transdermal therapeutic systems comprising guanfacine.

Therefore, a need exists for a transdermal therapeutic system for the transdermal administration of guanfacine. In particular, a need exists for a TTS, which is suitable for multi day therapy with a single application thereby improving patient compliance.

### OBJECTS AND SUMMARY OF THE INVENTION

It is therefore an object of the present invention to provide a TTS for the transdermal administration of guanfacine. In particular, it is an object of the present invention to provide a TTS for the transdermal administration of guanfacine providing a skin permeation rate which is sufficient for achieving a therapeutically effective dose.

It is a further object of the present invention to provide a TTS for the transdermal administration of guanfacine providing therapeutically effective amounts of guanfacine for at least 24 hours, preferably at least 72 hours, more preferably about 84 hours. In particular, it is an object of the present invention that the therapeutically effective amounts are provided over the whole time period, wherein the TTS is applied to the skin, allowing an around the clock treatment by exchanging the TTS after a certain application time of, e.g., at least 24 hours, preferably at least 72 hours, more preferably about 84 hours.

It is a further object of the present invention to provide a TTS for the transdermal administration of guanfacine, wherein the fluctuation in guanfacine blood plasma concentration is reduced when compared to oral administration, in particular at steady state.

It is a further object of the present invention to provide a TTS for the transdermal administration of guanfacine with a high active ingredient utilization.

It is another object of the present invention to provide a TTS for the transdermal administration of guanfacine which complies with the needs of a convenient application in view of size and thickness and/or which is easy and cost-efficient to manufacture.

It is a further object of the present invention, to provide a process for the preparation of the TTS.

These objects and others are accomplished by the present invention, which according to one aspect relates to a transdermal therapeutic system for the transdermal administration of guanfacine comprising a guanfacine-containing layer structure, said guanfacine-containing layer structure comprising:

A) a backing layer; and
B) a guanfacine-containing layer comprising guanfacine and a mono-carboxylic acid.

It has been found that the TTS according to the present invention, which comprises guanfacine and a mono-carboxylic acid, provides advantageous properties in terms of the active ingredient utilization and the constant and

continuous guanfacine delivery. In particular, the TTS according to the present invention provides suitable permeation rates and suitable permeated amounts of guanfacine over a time period of at least 24 hours, preferably at least 72 hours, more preferably about 84 hours. According to certain embodiments, the invention also relates to a transdermal therapeutic system for the transdermal administration of guanfacine as described above, wherein the guanfacine-containing layer is a guanfacine-containing matrix layer comprising:

i) guanfacine and a mono-carboxylic acid; and
ii) at least one polymer.

In certain preferred embodiments, the invention relates to a transdermal therapeutic system as described above, wherein the guanfacine and the mono-carboxylic acid in the guanfacine-containing layer are present in the form of a pre-mixture.

In a more preferred embodiment, the invention relates to a transdermal therapeutic system as described above, wherein the guanfacine and the mono-carboxylic acid in the guanfacine-containing layer are present in the form of a pre-mixture, wherein said pre-mixture is obtainable by a dry-grinding method or a slurry method.

In certain preferred embodiments, the at least one polymer is

- a mixture of an acrylic polymer and at least one silicone-based polymer;
- a mixture of two silicone-acrylic hybrid polymers; or
- a mixture of two silicone-based polymers; or
- an acrylic polymer; or
- an acrylic polymer comprising a -OH group.

In another preferred embodiment, the guanfacine-containing layer further comprises at least one additive, preferably at least two additives selected from the group consisting of dispersing agents, permeation enhancers and solubilizers.

According to one specific aspect, the present invention relates to a transdermal therapeutic system for the transdermal administration of guanfacine, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of an acrylic polymer and at least one silicone-based polymer, wherein the acrylic polymer is present in an amount of from 20 to 55 % by weight and the at least one silicone-based polymer is present in an amount of from 20 to 55 % by weight, based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

According to another specific aspect, the present invention relates to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

According to a further specific aspect, the present invention relates to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-based polymers, wherein the first silicone-based polymer is present in an amount of from 20 to 55 % by weight, and the second silicone-based polymer is present in an amount of from 20 to 55 % by weight, based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

According to another specific aspect, the present invention relates to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) an acrylic polymer in an amount of from 65 to 95 % by weight, based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

According to a further specific aspect, the present invention relates to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) an acrylic polymer comprising a -OH group in an amount of from 65 to 95 % by weight, based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

According to certain embodiments of the invention, the transdermal therapeutic system according to the invention is for use in a method of treating a human patient, preferably for use in a method of treating a human patient at the age of from 6 to 17. In particular, the transdermal therapeutic system according to the invention is for use in a method of treating hypertension or attention deficit hyperactivity disorder (ADHD) and/or as adjunctive therapy to stimulant medications in a human patient, preferably in a human patient at the age of from 5 to 17. In connection with these medical uses, the TTS according to the invention is preferably applied to the skin of the patient for at least 24 hours, more preferably at least 72 hours, most preferably about 84 hours.
In connection with these embodiments, it is preferred that the TTS according to the invention is applied to the skin of the patient for at least 24 hours, more preferably at least 72 hours, most preferably about 84 hours.

According to another embodiment, the present invention relates to a transdermal therapeutic system for the transdermal administration of guanfacine as defined above, having an $AUC_{0-24h}$ of about 10 to 600 ng*h/ml, preferably of about 20 to 400 ng*h/ml; and/or having an $AUC_{0-72h}$ of about 30 to 1800 ng*h/ml, preferably of about 60 to 1200 ng*h/ml; and/or having an $AUC_{0-84h}$ of about 35 to 2100 ng*h/ml, preferably of about 70 to 1400 ng*h/ml; and/or having a $C_{max}$ to $C_{84}$ ratio of less than 3.5; and/or having a $C_{max}$ to $C_{72}$ ratio of less than 3.0; and/or having a $C_{max}$ to $C_{24}$ ratio of less than 2.0.

According to a further aspect, the present invention relates to a process for manufacturing an active pharmaceutical-containing layer for use in a transdermal therapeutic system comprising the steps of:

1) combining at least the components

   (i) a pharmaceutically active agent; and
   (ii) at least one mono-carboxylic acid;

   to obtain a pre-mixture;

2) combining

   (i) the pre-mixture of step 1); and
   (ii) at least one polymer;

   to obtain a coating composition

3) coating the coating composition onto a backing layer or a release liner to obtain a coated coating composition; and

4) drying the coated coating composition to form the active pharmaceutical-containing layer, wherein the pharmaceutically active agent is guanfacine.

According to yet another aspect, the present invention relates to a transdermal therapeutic system obtainable by the process according to the invention.

**DEFINITIONS**

Within the meaning of this invention, the term "transdermal therapeutic system" (TTS) refers to a system by which the active agent (guanfacine) is administered to the systemic circulation *via* transdermal delivery and refers to the entire individual dosing unit that is applied, after removing an optionally present release liner, to the skin of a patient, and which comprises a therapeutically effective amount of active agent in an active agent-containing layer structure and optionally an additional adhesive overlay on top of the active agent-containing layer structure. The active agent-containing layer structure may be located on a release liner (a detachable protective layer), thus, the TTS may further comprise a release liner. Within the meaning of this invention, the term "TTS" in particular refers to systems providing transdermal delivery, excluding active delivery for example *via* iontophoresis or microporation. Transdermal therapeutic systems may also be referred to as transdermal drug delivery systems (TDDS) or transdermal delivery systems (TDS).

Within the meaning of this invention, the term "active agent-containing layer structure" or "guanfacine-containing layer structure " refers to the layer structure comprising a backing layer and an active agent-containing layer. The active agent-containing layer structure comprises a therapeutically effective amount of an active agent. Preferably, the active agent-containing layer structure is an active agent-containing self-adhesive layer structure. According to the claims, the active agent is guanfacine.

Within the meaning of this invention, the term "therapeutically effective amount" refers to a quantity of active agent in the TTS sufficient to provide, if administered by the TTS to a patient, the desired pharmacological effect. With regard to guanfacine, the term "therapeutically effective amount" preferably refers to a quantity of active agent in the TTS which is, if administered by the TTS to a patient, sufficient to treat, prevent or reduce hypertension or attention deficit hyperactivity disorder (ADHD) or which is sufficient for adjunctive therapy to stimulant medications in a human patient. A TTS usually contains more active in the system than is in fact provided to the skin and the systemic circulation. This excess amount of active agent is usually necessary to provide enough driving force for the delivery from the TTS to the systemic circulation.

Within the meaning of this invention, the terms "active", "active agent", and the like, as well as the term "guanfacine" refer to the respective active agent in any pharmaceutically acceptable chemical and morphological form and physical state. Preferably, the active agent in the present invention is present in the form of a "co-salt", meaning that the active agent, preferably guanfacine, is present in a pre-mixture together with at least one mono-carboxylic acid, such that a e.g proton transfer or the formation of hydrogen bonds is possible, and preferably at least partly a guanfacine salt of the mono-carboxylic acid is formed. In other words, the active agent, preferably guanfacine, may at least partly be present in protonated from. Further forms include without limitation the active agent in its protonated or partially protonated form,

deprotonated or partially deprotonated form, salts, or cocrystals. Further pharmaceutically acceptable chemical and morphological form and physical states include solvates, hydrates, clathrates, complexes and so on, as well as the active agent in the form of particles, which may be micronized, crystalline and/or amorphous, and any mixtures of the aforementioned forms. The active agent, where contained in a medium such as a solvent, may be dissolved or dispersed or in part dissolved and in part dispersed. However, in connection with the active agent, preferably guanfacine, and the mono-carboxylic acid it is to be understood that any form obtained in the guanfacine-containing layer based on the pre-mixture of guanfacine and the mono-carboxylic acid is covered by the present invention, including, e.g., the option of the guanfacine and the mono-carboxylic acid co-existing without chemical interaction, i.e. proton transfer or the formation of hydrogen bonds, as well as the option of the formation of a co-salt as described above. Preferably, the guanfacine free base and the mono-carboxylic acid may form together a co-salt or any other type of acid addition salt, so that the guanfacine-containing layer preferably comprises at least partly a co-salt or any other type of acid addition salt of guanfacine free base and the mono-carboxylic acid. In other words, the guanfacine-containing layer preferably comprises the guanfacine at least partly in protonated form and the mono-carboxylic acid at least partly in deprotonated form.

When the active agent is mentioned to be used in a particular form in the manufacture of the TTS, this does not exclude interactions between this form of the active agent and other ingredients of the active agent-containing layer structure, e.g., when the active agent is provided in a pre-mixture with the mono-carboxylic acid such that a co-salt, or any other type of acid addition salt is formed, which may still be present in the final TTS. This means that the active agent may be present in the final TTS in protonated or partially protonated / or deprotonated or partially deprotonated form or in the form of an acid addition salt, or, if it is included in the form of a salt, parts of it may be present as free base in the final TTS. Unless otherwise indicated, in particular the amount of the active agent in the layer structure relates to the amount of the active agent included in the TTS during manufacture of the TTS and is calculated based on the active agent itself or the pre-mixture of active agent and the mono-carboxylic acid, but not on other forms thereof.

The active agent starting material included in the TTS during manufacture of the TTS may be in the form of particles. The active agent may e.g. be present in the active agent-containing layer structure in the form of particles and/or dissolved.

Within the meaning of this invention, the term "particles" refers to a solid, particulate material comprising individual particles, the dimensions of which are negligible compared to the material. In particular, the particles are solid, including plastic/deformable solids, including amorphous and crystalline materials.

Within the meaning of this invention, the term "dispersing" refers to a step or a combination of steps wherein a starting material (e.g. guanfacine) is not totally dissolved. Dispersing in the sense of the invention comprises the dissolution of a part of the starting material (e.g. guanfacine particles), depending on the solubility of the starting material (e.g. the solubility of guanfacine in the coating composition).

There are two main types of TTS for active agent delivery, i.e. matrix-type TTS and reservoir-type TTS. The release of the active agent in a matrix-type TTS is mainly controlled by the matrix including the active agent itself. In contrast thereto, a reservoir-type TTS typically needs a rate-controlling membrane controlling the release of the active agent. In principle, also a matrix-type TTS may contain a rate-controlling membrane. However, matrix-type TTS are advantageous in that, compared to reservoir-type TTS, usually no rate determining membranes are necessary and no dose dumping can occur due to membrane rupture. In summary, matrix-type transdermal therapeutic systems (TTS) are less complex in manufacture and easy and convenient to use by patients.

Within the meaning of this invention, "matrix-type TTS" refers to a system or structure wherein the active is homogeneously dissolved and/or dispersed within a polymeric carrier, i.e. the matrix, which forms with the active agent and optionally remaining ingredients a matrix layer. In such a system, the matrix layer controls the release of the active agent from the TTS. Preferably, the matrix layer has sufficient cohesion to be self-supporting so that no sealing between other layers is required. Accordingly, the active agent-containing layer may in one embodiment of the invention be an active agent-containing matrix layer, wherein the active agent is homogeneously distributed within a polymer matrix. In certain embodiments, the active agent-containing matrix layer may comprise two active agent-containing matrix layers, which may be laminated together. Matrix-type TTS may in particular be in the form of a "drug-in-adhesive"-type TTS referring to a system wherein the active is homogeneously dissolved and/or dispersed within a pressure-sensitive adhesive matrix. In this connection, the active agent-containing matrix layer may also be referred to as active agent-containing pressure sensitive adhesive layer or active agent-containing pressure sensitive adhesive matrix layer. A TTS comprising the active agent dissolved and/or dispersed within a polymeric gel, e.g. a hydrogel, is also considered to be of matrix-type in accordance with present invention. It is to be understood that a TTS comprising an active agent-containing matrix layer may additionally also comprise a skin contact layer.

Reservoir-type TTS are not to be understood as being of matrix-type within the meaning of the invention.

Within the meaning of this invention, the term "active agent-containing layer" refers to a layer containing the active agent and providing the area of release. The term covers active agent-containing matrix layers and active agent-containing reservoir layers. If the active agent-containing layer is an active agent-containing matrix layer, said layer is present in a matrix-type TTS. If the polymer is a pressure-sensitive adhesive, the matrix layer may also represent the

adhesive layer of the TTS, so that no additional skin contact layer is present. Alternatively, an additional skin contact layer may be present as adhesive layer, and/or an adhesive overlay is provided. The additional skin contact layer is typically manufactured such that it is active agent-free. However, due to the concentration gradient, the active agent will migrate from the matrix layer to the additional skin contact layer over time, until an equilibrium is reached. The additional skin contact layer may be present on the active agent-containing matrix layer or separated from the active agent-containing matrix layer by a membrane, preferably a rate controlling membrane. Preferably, the active agent-containing matrix layer has sufficient adhesive properties, so that no additional skin contact layer is present. If the active agent-containing layer is an active agent-containing reservoir layer, said layer is present in a reservoir-type TTS, and the layer comprises the active agent in a liquid reservoir. In addition, a skin contact layer may be present, in order to provide adhesive properties. The additional skin contact layer is typically manufactured such that it is active agent-free. If the skin contact layer is free of active agent the active agent will migrate, due to the concentration gradient, from the reservoir layer to the skin contact layer over time, until an equilibrium is reached. Additionally, an adhesive overlay may be provided.

As used herein, the active agent-containing layer is preferably an active agent-containing matrix layer, and it is referred to the final solidified layer. Preferably, an active agent-containing matrix layer is obtained after coating and drying the solvent-containing coating composition as described herein. Alternatively, an active agent-containing matrix layer is obtained after melt-coating and cooling. The active agent-containing matrix layer may also be manufactured by laminating two or more such solidified layers (e.g. dried or cooled layers) of the same composition to provide the desired area weight. Preferably, the matrix layer is a pressure sensitive adhesive matrix layer. Optionally, an adhesive overlay may be present.

Within the meaning of this invention, the term "pressure-sensitive adhesive" (also abbreviated as "PSA") refers to a material that in particular adheres with finger pressure, is permanently tacky, exerts a strong holding force and should be removable from smooth surfaces without leaving a residue. A pressure sensitive adhesive layer, when in contact with the skin, is "self-adhesive", i.e. provides adhesion to the skin so that typically no further aid for fixation on the skin is needed. A "self-adhesive" layer structure according to the present invention includes a pressure sensitive adhesive layer for skin contact which may be provided in the form of a pressure sensitive matrix layer or in the form of an additional layer, i.e. a pressure sensitive adhesive skin contact layer. An adhesive overlay may still be employed to advance adhesion. The pressure-sensitive adhesive properties of a pressure-sensitive adhesive depend on the polymer or polymer composition used.

Within the meaning of this invention, the term "silicone acrylic hybrid polymer" refers to a polymerization product including repeating units of a silicone sub-species and an acrylate-sub species. The silicone acrylic hybrid polymer thus comprises a silicone phase and an acrylic phase. Preferably, the silicone acrylic hybrid polymer comprises a silicone phase and an acrylate phase, i.e. silicone sub-species and acrylate sub-species, in a certain weight ratio, e.g. from 60:40 to 40:60. The term "silicone acrylic hybrid" is intended to denote more than a simple blend of a silicone-based sub-species and an acrylate-based sub-species. Instead, the term denotes a polymerized hybrid species that includes silicone based sub-species and acrylate-based sub-species that have been polymerized together. The silicone acrylic hybrid polymer may also be referred to as a "silicone acrylate hybrid polymer" as the terms acrylate and acrylic are generally used interchangeably in the context of the hybrid polymers used in the present invention.

Within the meaning of this invention, the term "silicone acrylic hybrid pressure-sensitive adhesive" refers to a silicone acrylic hybrid polymer in the form of a pressure-sensitive adhesive. Silicone acrylic hybrid pressure-sensitive adhesives are described, for example, in EP 2 599 847 and WO 2016/130408. Examples of silicone acrylic hybrid pressure-sensitive adhesives include the PSA series 7-6100 and 7-6300 manufactured and supplied in n-heptane or ethyl acetate by DuPont™ (7-610X and 7-630X; X=1 n-heptane-based / X=2 ethyl acetate-based). It was found that, depending on the solvent in which the silicone acrylic hybrid PSA is supplied, the arrangement of the silicone phase and the acrylic phase providing a silicone or acrylic continuous external phase and a corresponding discontinuous internal phase is different. If the silicone acrylic hybrid PSA is supplied in n-heptane, the composition contains a continuous, silicone external phase and a discontinuous, acrylic internal phase. If the silicone acrylic hybrid PSA composition is supplied in ethyl acetate, the composition contains a continuous, acrylic external phase and a discontinuous, silicone internal phase.

Within the meaning of this invention, the term "non-hybrid polymer" is used synonymously for a polymer which does not include a hybrid species. Preferably, the non-hybrid polymer is a pressure-sensitive adhesive (e.g. a silicone- or acrylate-based pressure-sensitive adhesives). A preferred non-hybrid polymer according to the present invention is a "silicone-based polymer", which, as used herein, is a polymer obtainable by polycondensation of silanol endblocked polydimethylsiloxane with a silicate resin. Another preferred non-hybrid polymer is an acrylate-based polymer, i.e. an acrylic polymer, which, as used herein, is a polymer obtainable from one or more monomers selected from acrylic acid, butylacrylate, 2-ethylhexylacrylate, glycidylmethacrylate, 2-hydroxyethylacrylate, methylacrylate, methylmethacrylate, butylmethacrylate, t-octylacrylamide, and vinylacetate.

As used herein, an active agent-containing matrix layer is a layer containing the active agent dissolved or dispersed in at least one polymer, or containing the active agent dissolved in a solvent to form an active agent-solvent mixture that is dispersed in the form of deposits (in particular droplets) in at least one polymer. Preferably, the at least one polymer is a polymer-based pressure-sensitive adhesive (e.g. an acrylic polymer). Within the meaning of this invention,

the term "pressure-sensitive adhesive layer" refers to a pressure-sensitive adhesive layer obtained from a solvent-containing adhesive coating composition after coating on a film and evaporating the solvents.

Within the meaning of this invention, the term "skin contact layer" refers to the layer included in the active agent-containing layer structure to be in direct contact with the skin of the patient during administration. When the TTS comprises a skin contact layer, the other layers of the active agent-containing layer structure do not contact the skin and do not necessarily have self-adhesive properties. As outlined above, an additional skin contact layer attached to the active agent-containing layer may over time absorb parts of the active agent. The sizes of the skin contact layer and the active agent-containing layer are usually coextensive and correspond to the area of release. However, the area of the skin contact layer may also be greater than the area of the active agent-containing layer. In such a case, the area of release still refers to the area of the active agent-containing layer.

Within the meaning of this invention, the term "area weight" refers to the dry weight of a specific layer, e.g. of the matrix layer, provided in g/m$^2$. The area weight values are subject to a tolerance of $\pm$ 10 %, preferably $\pm$ 7.5 %, due to manufacturing variability.

If not indicated otherwise "%" refers to weight-% (% by weight).

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2000, preferably above 5000 and more preferably above 10,000 Dalton. Correspondingly, compounds with a molecular weight below 2000, preferably below 5000 or more preferably below 10,000 Dalton are usually referred to as oligomers.

Within the meaning of this invention, the term "cross-linking agent" refers to a substance which is able to cross-link functional groups contained within the polymer.

Within the meaning of this invention, the term "adhesive overlay" refers to a self-adhesive layer structure that is free of active agent and larger in area than the active agent-containing structure and provides additional area adhering to the skin, but no area of release of the active agent. It enhances thereby the overall adhesive properties of the TTS. The adhesive overlay comprises a backing layer that may provide occlusive or non-occlusive properties and an adhesive layer. Preferably, the backing layer of the adhesive overlay provides non-occlusive properties.

Within the meaning of this invention, the term "backing layer" refers to a layer which supports the active agent-containing layer or forms the backing of the adhesive overlay. At least one backing layer in the TTS and usually the backing layer of the active agent-containing layer is substantially impermeable to the active agent contained in the layer during the period of storage and administration and thus prevents active loss or cross-contamination in accordance with regulatory requirements. Preferably, the backing layer is also occlusive, meaning substantially impermeable to water and water-vapor. Suitable materials for a backing layer include polyethylene terephthalate (PET), polyethylene (PE), ethylene vinyl acetate-copolymer (EVA), polyurethanes, and mixtures thereof. Suitable backing layers are thus for example PET laminates, EVA-PET laminates and PE-PET laminates. Also suitable are woven or non-woven backing materials.

The TTS according to the present invention can be characterized by certain parameters as measured in an *in vitro* skin permeation test.

In general, the *in vitro* permeation test is performed in a Franz diffusion cell, with 0.9 % sodium chloride solution with 0.1 % sodium azide as antibacteriological agent.

Further, *in vitro* permeation test may be performed in a Franz diffusion cell, with human or animal skin and preferably with dermatomed Göttinger minipig with a thickness of around 800 $\mu$m and an intact epidermis, and with 0.9% sodium chloride solution (32 °C with 0.1 % saline azide).

Where not otherwise indicated, the *in vitro* permeation test is performed with dermatomed Göttinger minipig skin with a thickness around 800$\mu$m and an intact epidermis, and with 0.9% Sodium chloride solution as receptor medium (32 °C with 0.1 % saline azide). The amount of active permeated into the receptor medium is determined in regular intervals using a HPLC method) with a UV photometric detector by taking a sample volume. The receptor medium is completely or in part replaced by fresh medium when taking the sample volume, and the measured amount of active permeated relates to the amount permeated between the two last sampling points and not the total amount permeated so far.

Thus, within the meaning of this invention, the parameter "permeated amount" is provided in $\mu$g/cm$^2$ and relates to the amount of active permeated in a sample interval at certain elapsed time. E.g., in an *in vitro* permeation test as described above, wherein the amount of active permeated into the receptor medium has been e.g. measured at hours 0, 4, 8, 16, 24, 32, 40, 48, 56, 64, 72 and 88 the "permeated amount" of active can be given e.g. for the sample interval from hour 8 to hour 16 and corresponds to the measurement at hour 16, wherein the receptor medium has been exchanged completely at hour 8.

The permeated amount can also be given as a "cumulative permeated amount", corresponding to the cumulated amount of active permeated at a certain point in time. E.g., in an *in vitro* permeation test as described above, wherein the

amount of active permeated into the receptor medium has been e.g. measured at hours 0, 4, 8, 16 and 24, the "cumulative permeated amount" of active at hour 16 corresponds to the sum of the permeated amounts from hour 0 to hour 4, hour 4 to hour 8 and hour 8 to hour 16.

Within the meaning of this invention, the parameter "skin permeation rate" for a certain sample interval at certain elapsed time is provided in $\mu g/(cm^2*h)$ and is calculated from the permeated amount in said sample interval as measured by *in vitro* permeation test as described above in $\mu g/cm^2$, divided by the hours of said sample interval. E.g. the skin permeation rate in an *in vitro* permeation test as described above, wherein the amount of active permeated into the receptor medium has been e.g. measured at hours 0, 4, 8, 16 and 24, the "skin permeation rate" at hour 16 is calculated as the permeated amount in the sample interval from hour 8 to hour 16 divided by 8 hours.

A "cumulative skin permeation rate" can be calculated from the respective cumulative permeated amount by dividing the cumulative permeated amount by the elapsed time. E.g. in an *in vitro* permeation test as described above, wherein the amount of active permeated into the receptor medium has been e.g. measured at hours 0, 4, 8, 16 and 24, the "cumulative skin permeation rate" at hour 16 is calculated as the cumulative permeated amount for hour 16 (see above) divided by 16 hours.

Within the meaning of this invention, the above parameters "permeated amount" and "skin permeation rate" (as well as "cumulative permeated amount" and "cumulative skin permeation rate") refer to mean values calculated from at least 2 *in vitro* permeation test experiments. Where not otherwise indicated, the standard deviation (SD) of these mean values refer to a corrected sample standard deviation, calculated using the formula:

$$SD = \sqrt{\frac{1}{n-1}\sum_{i=1}^{n}\left(x_i - \bar{x}\right)^2}$$

wherein n is the sample size, $\{x_1, x_2, ... x_n\}$ are the observed values and $x$ is the mean value of the observed values.

The TTS according to the present invention can also be characterized by certain parameters as measured in an *in vivo* clinical study.

Within the meaning of this invention, the parameter "mean release rate" refers to the mean release rate in $\mu g/h$ ($\mu g/hour$) or in mg/day over the period of administration (e.g., 1 to 7 days) by which the active agent is released through the human skin into the systemic circulation and is based on the AUC obtained over said period of administration in a clinical study.

Within the meaning of this invention, the term "extended period of time" relates to a period of at least or about 24 hours, at least or about 48 hours, at least or about 84 hours, at least or about 168 hours, at least or about 1 day, at least or about 3.5 days, or at least or about 7 days, or to a period of about 24 hours to about 168 hours or 1 to 7 day(s), or about 24 hours to about 84 hours or 1 to 3.5 day(s).

For a continuous drug treatment, the frequency of drug administration is preferably kept sufficiently high so as to maintain therapeutically effective blood plasma concentration. In other words, the interval between two dosage form administrations, also called dosing interval, needs to be adapted accordingly. Within the meaning of the present invention, the term "dosing interval" refers to the period of time between two consecutive TTS administrations, i.e. the interval between two consecutive points in time a TTS is applied to the skin of the patient. Once applied, the TTS is usually maintained on the skin of the patient for the entire dosing interval and only removed at the end of the dosing interval, at which time a new TTS is applied to the skin. E.g., if the dosing interval is 24 hours or 1 day, the TTS is applied to and maintained on the skin of the patient for 24 hours or 1 day. After 24 hours or 1 day, the TTS is removed from the skin and a new TTS is applied. Thus, a dosing interval of 24 hours or 1 day allows a daily TTS exchange mode in an around-the-clock treatment.

Within the meaning of this invention, the term "room temperature" refers to the unmodified temperature found indoors in the laboratory where the experiments are conducted and usually lies within 15 to 35 °C, preferably about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated.

Within the meaning of this invention the term "pharmacokinetic parameters" refers to parameters describing the blood plasma curve, e.g. $C_{max}$, $C_t$ and $AUC_{t1-t2}$ obtained in a clinical study, e.g. by single-dose, multi-dose or steady state administration of the active agent-containing TTS, e.g. the guanfacine-containing TTS to healthy human subjects. The pharmacokinetic parameters of the individual subjects are summarized using arithmetic and geometric means, e.g. a mean $C_{max}$, a mean $AUC_t$ and a mean $AUC_{INF}$, and additional statistics such as the respective standard deviations and standard errors, the minimum value, the maximum value, and the middle value when the list of values is ranked (Median). In the context of the present invention, pharmacokinetic parameters, e.g. the $C_{max}$, $C_t$ and $AUC_{t1-t2}$ refer to geometric

mean values if not indicated otherwise. It cannot be precluded that the absolute mean values obtained for a certain TTS in a clinical study vary to a certain extent from study to study. To allow a comparison of absolute mean values between studies, a reference formulation, e.g. in the future any product based on the invention, may be used as internal standard. A comparison of the AUC per area of release of the respective reference product in the earlier and later study can be used to obtain a correction factor to take into account differences from study to study.

Clinical studies according to the present invention refer to studies performed in full compliance with the International Conference for Harmonization of Clinical Trials (ICH) and all applicable local Good Clinical Practices (GCP) and regulations.

Within the meaning of this invention, the term "healthy human subject" refers to a male or female subject with a body weight ranging from 55 kg to 100 kg and a body mass index (BMI) ranging from 18 to 29.4 and normal physiological parameters, such as blood pressure, etc. Healthy human subjects for the purposes of the present invention are selected according to inclusion and exclusion criteria, which are based on and in accordance with recommendations of the ICH.

Within the meaning of this invention, the term "subject population" refers to at least five, preferably at least ten individual healthy human subjects.

Within the meaning of this invention, the term "geometric mean" refers to the mean of the log transformed data back-transformed to the original scale.

Within the meaning of this invention, the term "arithmetic mean" refers to the sum of all values of observation divided by the total number of observations.

Within the meaning of this invention, the parameter "AUC" corresponds to the area under the plasma concentration-time curve. The AUC value is proportional to the amount of active agent absorbed into the blood circulation in total and is hence a measure for the bioavailability.

Within the meaning of this invention, the parameter "$AUC_{t1-t2}$" is provided in (ng / ml) h and relates to the area under the plasma concentration-time curve from hour t1 to t2 and is calculated by the linear trapezoidal method, unless otherwise indicated. Other calculation methods are e.g. the logarithmic and linear log trapezoidal method.

Within the meaning of this invention, the parameter "$C_{max}$" is provided in (ng / ml) and relates to the maximum observed blood plasma concentration of the active agent.

Within the meaning of this invention, the parameter "Ct" is provided in (ng / ml) and relates to the blood plasma concentration of the active agent observed at hour t.

Within the meaning of this invention, the parameter "$t_{max}$" is provided in hours and relates to the time point at which the $C_{max}$ value is reached. In other words, $t_{max}$ is the time point of the maximum observed plasma concentration.

Within the meaning of this invention, the term "mean plasma concentration" is provided in (ng / ml) and is a mean of the individual plasma concentrations of active agent, e.g. guanfacine, at each point in time.

Within the meaning of this invention, the term "coating composition" refers to a composition comprising all components of the matrix layer in a solvent, which may be coated onto the backing layer or release liner to form the matrix layer upon drying.

Within the meaning of this invention, the term "pressure sensitive adhesive composition" refers to a pressure sensitive adhesive at least in mixture with a solvent (e.g. n-heptane or ethyl acetate).

Within the meaning of this invention, the term "dissolve" refers to the process of obtaining a solution, which is clear and does not contain any particles, as visible to the naked eye.

Within the meaning of this invention, the term "solvent" refers to any liquid substance, which preferably is a volatile organic liquid such as methanol, ethanol, isopropanol, acetone, ethyl acetate, methylene chloride, hexane, n-heptane, toluene and mixtures thereof.

Within the meaning of this invention, the term "mono-carboxylic acid" refers to a carboxylic acid comprising only one -COOH group.

Within the meaning of this invention, the term "equimolar" refers to the amount in which two or more ingredients are present. Preferably, "equimolar" means that the ingredients are present in a ratio of 1:1.

Within the meaning of this invention, the term "pre-mixture" refers to a mixture of guanfacine and the mono-carboxylic acid. The pre-mixture can be obtained by different preparation methods, such as a dry-grinding method or a slurry method. Within the meaning of this invention the pre-mixture obtained by the "slurry method" comprises the process of weighing in guanfacine free base and a mono-carboxylic acid in equimolar amounts. Solvent, e.g. DCM is added and the mixture is stirred with a magnetic stirring bar at room temperature for at least 1 day. The white solid was recovered by filtration under vacuum, washed with solvent and dried under vacuum at 40 °C for 24 hours.

Within the meaning of this invention, the pre-mixture obtained by the "dry-grinding method" refers to guanfacine and the mono-carboxylic acid obtained by the process comprising milling of a mixture of guanfacine base, mono-carboxylic acid and methanol with zirconium oxide milling beads (e.g. bead size 3 mm, in a Retsch Mixer Mill MM 500) at 35 Hz for approx. 10 minutes.

## BRIEF DESCRIPTION OF THE DRAWINGS

Fig.1.1 depicts the guanfacine cumulative permeated amounts of TTS prepared according to comparative Examples 1A and 1B.
Fig.1.2 depicts the guanfacine cumulative permeated amount of a TTS prepared according to comparative Example 1D.
Fig.1.3 depicts the guanfacine cumulative permeated amounts of TTS prepared according to Examples 1A-E and comparative Example 1C.
Fig. 2 depicts the guanfacine cumulative permeated amounts of TTS prepared according to Examples 2A-C and comparative Examples 2A and 2B.
Fig.3 depicts the guanfacine cumulative permeated amounts of TTS prepared according to Examples 3A-E and comparative Example 3.
Fig. 4.1 depicts the guanfacine cumulative permeated amounts of TTS prepared according to Examples 4A-C and comparative Example 4.
Fig. 4.2 depicts the guanfacine cumulative permeated amounts of the TTS prepared according to Example 4D.
Fig. 5.1 depicts the guanfacine cumulative permeated amounts of TTS prepared according to Examples 5A-E.
Fig. 5.2 depicts the guanfacine cumulative permeated amounts of the TTS prepared according to Example 5F.
Fig. 6 depicts the guanfacine cumulative permeated amounts of TTS prepared according to Example 6 and comparative Example 6.

## DETAILED DESCRIPTION

### TTS STRUCTURE

The present invention relates to a transdermal therapeutic system for the transdermal administration of guanfacine comprising a guanfacine-containing layer structure, said guanfacine-containing layer structure comprising a) a backing layer, and b) a guanfacine-containing layer comprising guanfacine and a mono-carboxylic acid. This guanfacine-containing layer structure is preferably a guanfacine-containing self-adhesive layer structure and preferably does not comprise an additional skin contact layer. Preferably, the guanfacine-containing layer is a guanfacine-containing matrix layer comprising guanfacine and a mono-carboxylic acid, and at least one polymer. In particular, the at least one polymer present in the transdermal therapeutic system preferably provides the adhesive properties. Furthermore, the guanfacine-containing layer structure comprises guanfacine and a mono-carboxylic acid. Preferably, the guanfacine, in particular the guanfacine free base and the mono-carboxylic acid are pre-mixed prior to the addition to the guanfacine-containing layer.

The TTS according to the present invention may be a matrix-type TTS or a reservoir-type TTS, and preferably is a matrix-type TTS.

In a matrix-type TTS according to the invention, the pre-mixture of the guanfacine and the mono-carboxylic acid are preferably homogeneously dispersed within a polymeric carrier, i.e. the matrix, which forms with the guanfacine and the mono-carboxylic acid and optionally remaining ingredients a matrix layer. Accordingly, the guanfacine-containing layer may in one embodiment of the invention be a guanfacine-containing matrix layer, wherein the guanfacine and the mono-carboxylic acid are homogeneously dispersed within a polymer matrix. The polymer matrix preferably comprises the at least one polymer as defined herein. Thus, it is preferred according to the invention that the guanfacine-containing matrix layer comprises guanfacine, a mono-carboxylic acid and at least one polymer, which is present in the TTS. The at least one polymer is selected from the group consisting of acrylic polymers, silicone-based polymers, silicone-acrylic hybrid polymers, and mixtures thereof. More preferably, according to the invention the guanfacine-containing matrix layer comprises guanfacine, a mono-carboxylic acid and at least one polymer selected from a mixture of an acrylic polymer and at least one silicone-based polymer, a mixture of two silicone-acrylic hybrid polymers, a mixture of two silicone-based polymers, an acrylic polymer, and an acrylic polymer comprising a -OH group. In this connection, it is also preferred that the guanfacine-containing matrix layer is self-adhesive, so that no additional skin contact layer is present. If a guanfacine-containing matrix layer is prepared by laminating together two guanfacine-containing matrix layers, which are of substantially the same composition, the resulting double layer is to be regarded as one guanfacine-containing matrix layer.

In a preferred embodiment of the invention, the guanfacine-containing layer is a guanfacine-containing matrix layer comprising

i) guanfacine and a mono-carboxylic acid; and
ii) at least one polymer.

Thus, according to one embodiment, the present invention relates to a transdermal therapeutic system for the transdermal administration of guanfacine comprising a guanfacine-containing layer structure comprising:

A) a backing layer; and
B) a guanfacine-containing layer, which is preferably a guanfacine-containing matrix layer, comprising:

i) guanfacine and a mono-carboxylic acid, and
ii) at least one polymer.

The guanfacine-containing layer structure is preferably a guanfacine-containing self-adhesive layer structure. In this connection, it is also preferred that the guanfacine-containing layer structure does not comprise an additional skin contact layer. Instead, it is preferred that the guanfacine-containing layer, which is preferably a guanfacine-containing matrix layer, is self-adhesive. Thus, in a preferred embodiment, the guanfacine-containing layer structure is a guanfacine-containing self-adhesive layer structure and preferably does not comprise an additional skin contact layer. Alternatively or additionally, it is preferred that the guanfacine-containing layer is directly attached to the backing layer, so that there is no additional layer between the backing layer and the guanfacine-containing layer. Consequently, a layer structure of low complexity is obtained, which is advantageous, e.g., in terms of the costs for the manufacture.

In particular, it is preferred that the guanfacine-containing layer structure comprises not more than 3, preferably 2 layers, i.e. preferably only the backing layer and the guanfacine-containing layer. Sufficient adhesion between the guanfacine-containing self-adhesive layer structure and the skin of the patient during administration is then provided by the guanfacine-containing layer, which is preferably a guanfacine-containing matrix layer. If an additional skin contact layer is present, e.g., as the third layer of the guanfacine-containing layer structure, the adhesive properties may be provided by the additional skin contact layer. However, it is preferred according to the invention that no additional skin contact layer is present.

The self-adhesive properties of the guanfacine-containing layer structure are preferably provided by the at least one polymer, which is present in the TTS, preferably in the guanfacine-containing layer, more preferably in the guanfacine-containing matrix layer. Thus, in a preferred embodiment of the invention, the at least one polymer is a pressure sensitive adhesive polymer.

In a preferred embodiment of the invention, the guanfacine and the mono-carboxylic acid in the guanfacine-containing layer are present in the form of a pre-mixture.

In a more preferred embodiment of the invention, the guanfacine and the mono-carboxylic acid in the guanfacine-containing layer are present in the form of a pre-mixture, wherein said pre-mixture is obtainable by a dry-grinding method or a slurry method.

In a preferred embodiment of the invention, the at least one polymer is selected from the group consisting of acrylic polymers, silicone-based polymers, silicone-acrylic hybrid polymers, and mixtures thereof. In a more preferred embodiment of the invention, the at least one polymer is selected from a mixture of an acrylic polymer and at least one silicone-based polymer, a mixture of two silicone-acrylic hybrid polymers, a mixture of two silicone-based polymers, an acrylic polymer, and an acrylic polymer comprising a -OH group. Further details regarding the at least one polymer according to the invention are provided further below.

It is to be understood that the TTS, preferably the guanfacine-containing layer, more preferably the guanfacine-containing matrix layer comprises at least two polymers, wherein the at least two polymers are selected from the group consisting of acrylic polymers, silicone-based polymers, silicone-acrylic hybrid polymers, and mixtures thereof. Preferably, it is to be understood that the TTS, preferably the guanfacine-containing layer, more preferably the guanfacine-containing matrix layer comprises a polymer selected from a mixture of an acrylic polymer and at least one silicone-based polymer, a mixture of two silicone-acrylic hybrid polymers and a mixture of two silicone-based polymers. The first polymer may provide advantages in terms of for example a high flux, while the second polymer can be used for example to reduce and/or optimize the flux in order to obtain a continuous and constant flux. Furthermore, the tackiness of the TTS can be modified by using a combination of at least two polymers. Further details regarding the specific polymers and the mixtures are provided below.

It is to be understood that the TTS according to the invention contains at least a therapeutically effective amount of guanfacine. Thus, in a preferred embodiment of the invention, the guanfacine-containing layer structure contains a therapeutically effective amount of guanfacine. The guanfacine in the guanfacine-containing layer structure according to the invention is present together with the mono-carboxylic acid, preferably in the form of a co-salt as described in more detail above or below. In a more preferred embodiment of the invention, the guanfacine and the mono-carboxylic acid are preferably dispersed in the guanfacine-containing layer. Preferred embodiments regarding the guanfacine and the mono-carboxylic acid in the TTS according to the invention are provided further below. Further, it is to be understood that the presence of guanfacine together with the mono-carboxylic acid enhances and/or optimizes the flux. In a particularly preferred embodiment of the invention, guanfacine and sorbic acid are present in the guanfacine-containing layer

structure.

It is to be understood that the TTS according to the invention optionally comprises at least one additive selected from the group consisting of dispersing agents, permeation enhancers and/or solubilizers. The additives are described in further detail below.

Further, it is to be understood that the additive is present within the TTS, preferably within the guanfacine-containing layer structure, more preferably within the guanfacine-containing layer, in particular within the guanfacine-containing matrix layer.

It is preferred according to the invention that the area of release of the TTS ranges from 1 to 100 $cm^2$, preferably from 2.5 to 50 $cm^2$.

In a preferred embodiment of the invention, the backing layer is substantially impermeable for the pre-mixture of guanfacine and a mono-carboxylic acid. In particular, in a preferred embodiment of the invention, the backing layer is substantially impermeable for guanfacine and/or the mono-carboxylic acid. Furthermore, it is preferred that the backing layer is occlusive as outlined above.

According to certain embodiments of the invention, the TTS may further comprise an adhesive overlay. This adhesive overlay is in particular larger in area than the guanfacine-containing layer structure and is attached thereto for enhancing the adhesive properties of the overall transdermal therapeutic system. Said adhesive overlay comprises a backing layer and an adhesive layer. The adhesive overlay provides additional area adhering to the skin but does not add to the area of release of the guanfacine. The adhesive overlay comprises a self-adhesive polymer or a self-adhesive polymer mixture selected from the group consisting of silicone acrylic hybrid polymers, acrylate polymers, silicone polymers, polyisobutylenes, styreneisoprene-styrene copolymers, and mixtures thereof, which may be identical to or different from any polymer or polymer mixture included in the guanfacine-containing layer structure.

The guanfacine-containing layer structure according to the invention, such as a guanfacine-containing self-adhesive layer structure, is normally located on a detachable protective layer (release liner), from which it is removed immediately before application to the surface of the patient's skin. Thus, the TTS may further comprise a release liner. A TTS protected this way is usually stored in a blister pack or a seam-sealed pouch. The packaging may be child resistant and/or senior friendly.

## GUANFACINE-CONTAINING LAYER

As outlined in more detail above, the TTS according to the present invention comprises a guanfacine-containing layer structure comprising a guanfacine-containing layer. Preferably, the guanfacine-containing layer structure is a guanfacine-containing self-adhesive layer structure. Accordingly, it is also preferred that the guanfacine-containing layer is a self-adhesive guanfacine-containing layer, more preferably a self-adhesive guanfacine-containing matrix layer. In a preferred embodiment, the guanfacine-containing layer comprises a therapeutically effective amount of the guanfacine.

In one embodiment of the invention, the guanfacine-containing layer is a guanfacine-containing matrix layer. In another embodiment, the guanfacine-containing layer is a guanfacine-containing reservoir layer. It is preferred that the guanfacine-containing layer is a guanfacine-containing matrix layer.

In one embodiment, the guanfacine-containing layer comprises:

i) guanfacine and a mono-carboxylic acid; and
ii) at least one polymer.

It is to be understood that the at least one polymer, which is contained in the guanfacine-containing layer, is the at least one polymer, which is contained in the TTS according to the invention.

In a preferred embodiment, the guanfacine-containing layer is a guanfacine-containing matrix layer comprising

i) guanfacine and a mono-carboxylic acid; and
ii) at least one polymer.

It is to be understood that the at least one polymer, which is contained in the guanfacine-containing layer, is the at least one polymer, which is contained in the TTS according to the invention.

In a preferred embodiment, the guanfacine-containing layer comprises at least one polymer selected from the group consisting of acrylic polymers, silicone-based polymers, silicone-acrylic hybrid polymers, and mixtures thereof. Preferably, the at least one polymer is a pressure-sensitive adhesive. Thus, the guanfacine-containing layer is preferably a guanfacine-containing matrix layer, and particularly preferably a guanfacine-containing pressure sensitive adhesive matrix layer.

In one embodiment of the invention, the guanfacine-containing layer comprises guanfacine and a mono-carboxylic acid or a salt formed from the guanfacine and the mono-carboxylic acid. Preferably, the guanfacine and

the mono-carboxylic acid are present in the form of a co-salt. Further, it is to be understood that the guanfacine and the mono-carboxylic acid are pre-mixed prior to the addition to the guanfacine-containing layer, such that a co-salt is formed.

Pre-mixing of the guanfacine and the mono-carboxylic acid according to the invention refers to different types of preparation methods for obtaining a guanfacine - mono-carboxylic acid pre-mixture. In one embodiment according to the present invention the pre-mixture of guanfacine and a mono-carboxylic acid is prepared by the dry-grinding method comprising milling of a mixture of guanfacine base, mono-carboxylic acid and methanol with zirconium oxide milling beads (e.g. bead size 3 mm, in a Retsch Mixer Mill MM 500) at 35 Hz for approx. 10 minutes. In this regard, it is to be understood that the guanfacine base and the mono-carboxylic acid are present in equimolar amounts and methanol act as a catalyst. In another embodiment according to the present invention the guanfacine / mono-carboxylic acid pre-mixture is prepared by the slurry method comprising weighing in guanfacine free base and a mono-carboxylic acid in equimolar amounts. Solvent, e.g. DCM is added and the mixture is stirred with a magnetic stirring bar at room temperature for at least 1 day. The white solid was recovered by filtration under vacuum, washed with solvent and dried under vacuum at 40 °C for 24 hours.

Accordingly, the guanfacine-containing layer is obtainable by dispersing the guanfacine mono-carboxylic acid pre-mixture, wherein preferably the guanfacine and the mono-carboxylic acid are present in the pre-mixture in the form of a co-salt. It is to be understood that the guanfacine - mono-carboxylic acid pre-mixture is obtainable by mixing of the guanfacine free base and the mono-carboxylic acid as described above. Preferably, the guanfacine in the present invention is present in the form of a "co-salt", meaning that the guanfacine is present in a pre-mixture together with at least one mono-carboxylic acid, such that a proton transfer is possible, and preferably at least partly a guanfacine salt of the mono-carboxylic acid is formed. In other words, the active agent, preferably guanfacine, may at least partly be present in protonated from. However, in connection with the active agent, preferably guanfacine and the mono-carboxylic acid it is to be understood that any form obtained in the guanfacine-containing layer based on the pre-mixture of guanfacine and the mono-carboxylic acid is covered by the present invention, including, e.g., the option of the guanfacine and the mono-carboxylic acid co-existing without chemical interaction, i.e. proton transfer, as well as the option of the formation of a co-salt as described above. Preferably, the guanfacine free base and the mono-carboxylic acid may form together a co-salt or any other type of acid addition salt, so that the guanfacine-containing layer preferably comprises at least partly a co-salt or any other type of acid addition salt of guanfacine free base and the mono-carboxylic acid. In other words, the guanfacine-containing layer preferably comprises the guanfacine at least partly in protonated form and the mono-carboxylic acid at least partly in deprotonated form.

Thus, in one embodiment, the guanfacine-containing layer comprises guanfacine and a mono-carboxylic acid or a salt formed from the guanfacine and the mono-carboxylic acid. In a more preferred embodiment of the present invention, the mono-carboxylic acid is sorbic acid. Thus, in a preferred embodiment of the invention, the guanfacine-containing layer comprises guanfacine and sorbic acid or a salt formed from the guanfacine and the sorbic acid. In connection with the above embodiments, it is surprising that the combination of guanfacine with a mono-carboxylic acid, in particular with the mono-carboxylic acid sorbic acid, enhances the flux and permeation of the transdermal therapeutic system. In particular, it has surprisingly been found by the inventors of the present invention that a TTS comprising a combination of guanfacine base and sorbic acid in the guanfacine-containing layer shows an enhanced cumulative permeated amount over 88 h in comparison to a TTS comprising only guanfacine in the form of the free base in the guanfacine-containing layer.

In one embodiment of the invention, the guanfacine-containing layer structure, preferably the guanfacine-containing layer, more preferably the guanfacine-containing matrix layer of a transdermal therapeutic system according to the invention comprises guanfacine in an amount of from 1 to 100 mg/TTS, preferably from 3 to 72 mg/TTS. In a preferred embodiment, the guanfacine-containing layer structure, preferably the guanfacine-containing layer, more preferably the guanfacine-containing matrix layer comprises guanfacine in an amount of from 3 to 50 mg/TTS. In another preferred embodiment, the guanfacine-containing layer structure, preferably the guanfacine-containing layer, more preferably the guanfacine-containing matrix layer comprises guanfacine in an amount of from 3 to 30 mg/TTS. In other words, the total amount of guanfacine in the guanfacine-containing layer structure ranges from 1 to 100 mg/TTS, preferably from 3 to 72 mg/TTS, more preferably from 3 to 50 mg/TTS, even more preferably from 3 to 30 mg/TTS. In this regard, it is to be understood that it is referred to the amount of guanfacine in the TTS and not to the amount of the guanfacine - mono-carboxylic acid pre-mixture.

In another embodiment, the guanfacine loading in the guanfacine-containing layer structure ranges from 0.4 to 2 $mg/cm^2$. In another embodiment, the guanfacine loading in the guanfacine-containing layer structure ranges from 0.4 to 0.85 $mg/cm^2$. Furthermore, it is preferred that the area of release of the TTS ranges from 1 to 100 $cm^2$, preferably from 2.5 to 50 $cm^2$. It is to be understood that it is referred to the loading of guanfacine and not the loading of the guanfacine - mono-carboxylic acid pre-mixture.

In one embodiment of the invention, the guanfacine-containing layer comprises guanfacine in an amount of from 1 to 20 % by weight, preferably from 2 to 16 % by weight, more preferably from 4 to 14 % by weight, most preferably from 5 to 13 % by weight, based on the total weight of the guanfacine-containing layer. Particularly preferably, the guanfacine-containing layer comprises guanfacine in an amount of from 4 to 8 % by weight, preferably from 5 to 7 % by weight, or in an amount of from 10 to 14 % by weight, preferably from 11 to 13 % by weight, based on the total weight of the guanfacine-containing layer, depending on the desired dosing strength of the TTS.

In one embodiment of the invention, the guanfacine-containing layer comprises the mono-carboxylic acid in an amount of from 1% to 20%, more preferably in an amount of from 2% to 16% by weight, based on the total weight of the guanfacine-containing layer. A particularly preferred mono-carboxylic acid, which is comprised together with the guanfacine in the guanfacine-containing layer is sorbic acid.

The guanfacine-containing layer structure, and preferably the guanfacine-containing layer comprises at least one polymer. As explained above, the at least one polymer is selected from the group consisting of acrylic polymers, silicone-based polymers, silicone-acrylic hybrid polymers, and mixtures thereof. Preferably, it is to be understood that the TTS, preferably the guanfacine-containing layer, more preferably the guanfacine-containing matrix layer comprises a polymer selected from a mixture of an acrylic polymer and at least one silicone-based polymer, a mixture of two silicone-acrylic hybrid polymers, a mixture of two silicone-based polymers, an acrylic polymer, and an acrylic polymer comprising a -OH group.

In one embodiment of the invention, the guanfacine-containing layer comprises the at least one polymer in an amount of from 20% to 99%, preferably from 30% to 97%, most preferably from 35% to 94% by weight based on the total weight of the guanfacine-containing layer. It is to be understood that the above indicated amounts of the at least one polymer may refer to one polymer only, but also to a combination of polymers as defined herein.

In one preferred embodiment of the invention, the guanfacine-containing layer comprises a mixture of an acrylic polymer and at least one silicone-based polymer, wherein the acrylic polymer is present in an amount of from 20 to 55 % and the at least one silicone-based polymer is present in an amount of from 20 to 55% by weight, based on the total weight of the active pharmaceutical-containing layer. It is to be understood that the amount of the at least one silicone-based polymer refers to the amount of only one silicone-based polymer or to the overall amount of the silicone-based polymers present, if more than one silicone-based polymer, e.g. two silicone-based polymers are present. Further details regarding the acrylic polymer and the silicone-based polymers are provided below.

In another preferred embodiment of the invention, the guanfacine-containing layer comprises a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer and wherein preferably the first silicone-acrylic hybrid polymer and the second silicone-acrylic hybrid polymer comprise a silicone phase and an acrylate phase in a weight ratio of from 60:40 to 40:60.

The silicone acrylic hybrid polymer as defined above comprises a silicone phase and an acrylate phase, preferably in a weight ratio of from 60:40 to 40:60, most preferably in a weight ratio of 50:50. The silicone acrylic hybrid polymer typically comprises the reaction product of (a) a silicon-containing pressure-sensitive adhesive composition comprising acrylate or methacrylate functionality, (b) an ethylenically unsaturated monomer; and (c) an initiator. Further details regarding components (a), (b) and (c) are provided further below. It is to be understood that component (a) mainly forms the silicone phase, while component (b) mainly forms the acrylate phase of the silicone acrylic hybrid polymer. The acrylate phase influences the tackiness and the viscosity of the silicone acrylic hybrid polymer. It is therefore preferred that the ethylenically unsaturated monomer forming the acrylate phase is a combination of 2-ethylhexyl acrylate and methyl acrylate, preferably in a ratio of from 40:60 to 70:30. Preferred in terms of a high tackiness is a ratio of 60:40, although the viscosity is then lower. Preferred in terms of a higher viscosity is a ratio of 50:50, although the tackiness is then reduced. The silicone acrylic hybrid polymer in the guanfacine-containing layer preferably contains a continuous, acrylic external phase and a discontinuous, silicone internal phase.

In one embodiment of the invention, the guanfacine-containing layer comprises the at least one silicone acrylic hybrid polymer in an amount of from 20 to 99 %, preferably from 30 to 97 %, most preferably from 35 to 94 % by weight, based on the total weight of the guanfacine-containing layer. In a preferred embodiment, the guanfacine-containing layer comprises the at least one silicone acrylic hybrid polymer in an amount of from 74 to 94 % by weight, preferably 74 to 89 % by weight, based on the total weight of the guanfacine-containing layer. It is to be understood that the above indicated amounts of the at least one silicone acrylic hybrid polymer may refer to one silicone acrylic hybrid polymer, but also to a combination of silicone acrylic hybrid polymers. Thus, the given amount refers to the overall amount of silicone acrylic hybrid polymers.

In one preferred embodiment of the invention, the guanfacine-containing layer comprises only one silicone acrylic hybrid polymer in an amount of from 60 to 97 % by weight, preferably in an amount of from 70 to 94 % by weight, based on the total weight of the guanfacine-containing layer.

In another preferred embodiment of the invention, the guanfacine-containing layer comprises a first silicone acrylic hybrid polymer and a second silicone acrylic hybrid polymer, wherein the overall amount of the at least two silicone acrylic hybrid polymers is from 35 to 94 % by weight, preferably from 74 to 94 % by weight, based on the total weight of the guanfacine-containing layer. Preferably, the guanfacine-containing layer comprises a first silicone acrylic hybrid polymer in an amount of from 60 to 90 % by weight, and a second silicone acrylic hybrid polymer in an amount of from 1 to 20 % by weight, based on the total weight of the guanfacine-containing layer. In one particularly preferred embodiment, the

guanfacine-containing layer comprises a first silicone acrylic hybrid polymer in an amount of from 70 to 85 %, preferably from 70 to 78 % by weight, and a second silicone acrylic hybrid polymer in an amount of from 1 to 8 % by weight, preferably from 3 to 5 % by weight, based on the total weight of the guanfacine-containing layer.

It is to be understood that the above-indicated preferences regarding the silicone acrylic hybrid polymers, in particular regarding the weight ratio of the acrylate to the silicone phase, regarding the components from which the silicone acrylic hybrid polymer is obtained, regarding the ethylenically unsaturated monomers from which the silicone acrylic hybrid polymer is formed as well as regarding the acrylic external phase and the silicone internal phase apply to both, the first and the second silicone acrylic hybrid polymer. In particular, it is preferred that the weight ratio of silicone phase to acrylate phase in the first silicone acrylic hybrid polymer is from 55:45 to 45:55, and that the ethylenically unsaturated monomers forming the acrylate comprise 2-ethylhexyl acrylate and methyl acrylate in a ratio of from 55:45 to 45:55. It is more preferred that the weight ratio of silicone phase to acrylate phase in the first silicone acrylic hybrid polymer is 50:50, and that the ethylenically unsaturated monomers forming the acrylate comprise 2-ethylhexyl acrylate and methyl acrylate in a ratio of 50:50. On the other hand, it is preferred that the weight ratio of silicone phase to acrylate phase in the second silicone acrylic hybrid polymer is from 55:45 to 45:55, and that the ethylenically unsaturated monomers forming the acrylate comprise 2-ethylhexyl acrylate and methyl acrylate in a ratio of from 65:35 to 55:45. It is more preferred that the weight ratio of silicone phase to acrylate phase in the second silicone acrylic hybrid polymer is 50:50, and that the ethylenically unsaturated monomers forming the acrylate comprise 2-ethylhexyl acrylate and methyl acrylate in a ratio of 60:40. Furthermore, it is for both silicone acrylic hybrid polymers preferred that the silicone phase is the internal phase and the acrylate phase is the external phase.

In another preferred embodiment of the invention, the guanfacine-containing layer comprises a mixture of two silicone-based polymers, wherein the first silicone-based polymer is present in an amount of from 20 to 55 % by weight, and the second silicone-based polymer is present in an amount of from 20 to 55 % by weight, based on the total weight of the guanfacine-containing layer. Further details regarding the silicone-based polymer are provided below.

In one embodiment of the invention, the TTS according to the invention, and in particular the guanfacine-containing layer, comprises at least one additive. Suitable additives are described in further detail below and are preferably each present in an amount of from 0.5 to 10 % by weight, based on the total weight of the guanfacine-containing layer.

In a preferred embodiment, the guanfacine-containing layer comprises at least one additive selected from the group consisting of dispersing agents, permeation enhancers, and solubilizers. In one preferred embodiment, the at least one additive is a dispersing agent. In another preferred embodiment, the at least one additive is a permeation enhancer. In yet another preferred embodiment, the at least one additive is a solubilizer. In certain preferred embodiments, also combinations of the afore-mentioned additives are preferred. The afore-mentioned additives are of particular advantage for providing the guanfacine in homogeneously dispersed and releasable form. It is to be understood that a dispersing agent may also act as permeation enhancer and vice versa. Similarly, also a solubilizer may additionally act as dispersing agent or permeation enhancer. Furthermore, the solubilizer may stabilize the guanfacine dispersion in the TTS and avoid crystallization. Moreover, the solubilizer can be helpful in optimizing the cohesion of the TTS. In certain preferred embodiments, the guanfacine-containing layer comprises at least one dispersing agent and at least one permeation enhancer, and optionally also at least one solubilizer.

In one preferred embodiment, the at least one additive is a dispersing agent, which is present in an amount of from 1 to 10 % by weight, based on the total weight of the guanfacine-containing layer. Preferably, the dispersing agent is present in an amount of from 2 to 6 % by weight, more preferably 3 to 5 % by weight, based on the total weight of the guanfacine-containing layer.

In another preferred embodiment, the at least one additive is a permeation enhancer, which is present in an amount of from 1 to 10 % by weight, based on the total weight of the guanfacine-containing layer. Preferably, the permeation enhancer is present in an amount of from 2 to 9 % by weight, more preferably in an amount of from 2 to 6 % by weight, even more preferably 3 to 5 % by weight, based on the total weight of the guanfacine-containing layer.

In another embodiment, the at least one additive is a solubilizer, which is present in an amount of from 0.5 to 10 % by weight, based on the total weight of the guanfacine-containing layer. Preferably, the solubilizer is present in an amount of from 0.5 to 5 % by weight, based on the total weight of the guanfacine-containing layer.

In one embodiment, the TTS according to the invention, and in particular the guanfacine-containing layer, more particularly the guanfacine-containing matrix layer, comprises at least two additives selected from the group consisting of dispersing agents, permeation enhancers, and solubilizers.

In one preferred embodiment, the transdermal therapeutic system, and in particular the guanfacine-containing layer, more particularly the guanfacine-containing matrix layer, comprises at least two additives, wherein the first additive is a dispersing agent, which is present in an amount of from 1 to 10 % by weight based on the total weight of the guanfacine-containing layer, and the second additive is a permeation enhancer, which is present in an amount of from 1 to 10 % by weight based on the total weight of the guanfacine-containing layer. Preferably, the dispersing agent is present in an amount of from 1 to 6 % by weight, and the permeation enhancer is present in an amount of from 2 to 9 % by weight. More preferably, the dispersing agent is present in an amount of from 2 to 6 % by weight, and the permeation enhancer is present

in an amount of from 2 to 6 % by weight. Even more preferably, the dispersing agent is present in an amount of from 3 to 5 % by weight, and the permeation enhancer is present in an amount of from 3 to 5 % by weight, based on the total weight of the guanfacine-containing layer.

In another preferred embodiment, the transdermal therapeutic system, and in particular the guanfacine-containing layer, more particularly the guanfacine-containing matrix layer, comprises at least two additives, wherein the first additive is a dispersing agent, which is present in an amount of from 1 to 10 % by weight based on the total weight of the guanfacine-containing layer, and the second additive is a solubilizer, which is present in an amount of from 0.5 to 10 % by weight based on the total weight of the guanfacine-containing layer. Preferably, the dispersing agent is present in an amount of from 1 to 6 % by weight, and the solubilizer is present in an amount of from 0.5 to 5 % by weight. More preferably, the dispersing agent is present in an amount of from 2 to 6 % by weight, and the solubilizer is present in an amount of from 0.5 to 5 % by weight. Even more preferably, the dispersing agent is present in an amount of from 3 to 5 % by weight, and the solubilizer is present in an amount of from 0.5 to 5 % by weight.

In another preferred embodiment, the transdermal therapeutic system, and in particular the guanfacine-containing layer, more particularly the guanfacine-containing matrix layer, comprises at least two additives, wherein the first additive is a permeation enhancer, which is present in an amount of from 1 to 10 % by weight based on the total weight of the guanfacine-containing layer, and the second additive is a solubilizer, which is present in an amount of from 0.5 to 10 % by weight based on the total weight of the guanfacine-containing layer. Preferably, the permeation enhancer is present in an amount of from 2 to 9 % by weight, and the solubilizer is present in an amount of from 0.5 to 5 % by weight. Preferably, the permeation enhancer is present in an amount of from 2 to 6 % by weight, and the solubilizer is present in an amount of from 0.5 to 5 % by weight. More preferably, the permeation enhancer is present in an amount of from 3 to 5 % by weight, and the solubilizer is present in an amount of from 0.5 to 5 % by weight.

In a particularly preferred embodiment of the invention, the TTS according to the invention, and in particular the guanfacine-containing layer, more preferably the guanfacine-containing matrix layer, comprises two additives selected from dispersing agents and permeation enhancers.

Accordingly, the guanfacine-containing layer, preferably the guanfacine-containing matrix layer comprises a dispersing agent, which is present in an amount of from 1 to 10 % by weight based on the total weight of the guanfacine-containing layer, and a permeation enhancer, which is present in an amount of from 1 to 10 % by weight based on the total weight of the guanfacine-containing layer. Preferably, the dispersing agent is present in an amount of from 1 to 6 % by weight, and the permeation enhancer is present in an amount of from 2 to 9 % by weight. More preferably, the dispersing agent is present in an amount of from 2 to 6 % by weight, and the permeation enhancer is present in an amount of from 2 to 6 % by weight. Even more preferably, the dispersing agent is present in an amount of from 3 to 5 % by weight, and the permeation enhancer is present in an amount of from 3 to 5 % by weight, based on the total weight of the guanfacine-containing layer.

In connection with the above embodiments regarding the number of additives and the amounts of additives in the TTS according to the invention, and in particular the guanfacine-containing layer, more particularly the guanfacine-containing matrix layer, the following specific additives are preferred.

In a preferred embodiment, the dispersing agent is selected from the group consisting of esters of fatty acids with polyols, fatty alcohols, polyethylene glycols having a number average molecular weight of from 300 to 400, polyethylene glycol alkyl ethers, and wherein the dispersing agent is preferably polyethylene glycol $C_8$-$C_{20}$-alkyl ether having from 2 to 10 EO units, preferably from 2 to 6 EO units. A particularly preferred dispersing agent is polyoxyethylene (4) lauryl ether ($C_{12}H_{25}(OCH_2CH_2)_4OH$). This dispersing agent is, e.g., available from Merck under the tradename Brij L4®.

In a preferred embodiment, the permeation enhancer is selected from the group consisting of diethylene glycol monoethyl ether (transcutol), oleic acid, levulinic acid, caprylic/capric triglycerides, diisopropyl adipate, isopropyl myristate, isopropyl palmitate, lauryl lactate, triacetin, dimethylpropylene urea, oleyl alcohol, oleoyl macrogol-6 glycerides (labrafil MS 1944), and lauroglycol, and is preferably oleyl alcohol, lauroglycol or oleoyl macrogol-6 glycerides (labrafil MS 1944). Oleylalcohol is, e.g., available from BASF under the tradename Kollicream® OA.

In a preferred embodiment, the solubilizer is selected from the group consisting of copolymers derived from esters of acrylic and methacrylic acid, polyvinylpyrrolidone, vinylpyrrolidone-vinyl acetate copolymers, and polyvinyl caprolactam-polyvinyl acetate-polyethylene glycol graft copolymers and is preferably a polyvinyl caprolactam-polyvinyl acetate-polyethylene glycol graft copolymer. Particularly preferred solubilizers are polyvinyl caprolactam-polyvinyl acetate-polyethylene glycol graft copolymers. Suitable polyvinyl caprolactam-polyvinyl acetate-polyethylene glycol graft copolymers are, e.g., available from BASF under the tradename Soluplus®, and preferably have the following structural formula, wherein 1, m, and n are selected such that an average molecular weight determined by gel permeation chromatography is in the range of 90000 to 140000 g/mol.

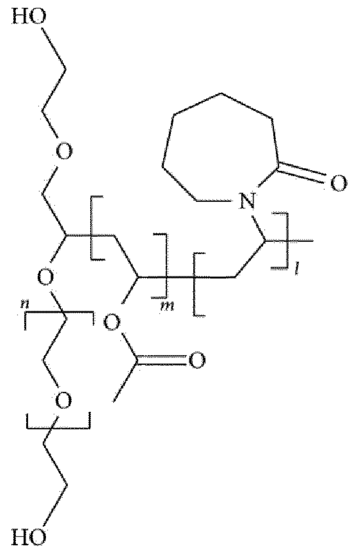

In certain preferred embodiments, the guanfacine-containing layer comprises at least one dispersing agent in an amount of from 2 to 6 % by weight, at least one permeation enhancer in an amount of from 2 to 9 % by weight, and optionally at least one solubilizer in an amount of from 0.5 to 5 % by weight, in each case based on the total weight of the guanfacine-containing layer. Preferably, the guanfacine-containing layer comprises at least one dispersing agent in an amount of from 2 to 6 % by weight, at least one permeation enhancer in an amount of from 2 to 6 % by weight, and optionally at least one solubilizer in an amount of from 0.5 to 5 % by weight, in each case based on the total weight of the guanfacine-containing layer. More preferably, the guanfacine-containing layer comprises at least one dispersing agent in an amount of from 3 to 5 % by weight, at least one permeation enhancer in an amount of from 3 to 5 % by weight, and optionally at least one solubilizer in an amount of from 0.5 to 5 % by weight, in each case based on the total weight of the guanfacine-containing layer. In connection with the above preferred weight-% amounts, the above preferred dispersing agents, permeation enhancers and solubilizers are preferred.

Accordingly, in a particularly preferred embodiment, the guanfacine-containing layer comprises a polyethylene glycol $C_8$-$C_{20}$-alkyl ether having from 2 to 10 EO units, preferably polyoxyethylene (4) lauryl ether, in an amount of from 2 to 6 % by weight, oleyl alcohol in an amount of from 2 to 9 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer. In a more particularly preferred embodiment, the guanfacine-containing layer comprises a polyethylene glycol $C_8$-$C_{20}$-alkyl ether having from 2 to 10 EO units, preferably polyoxyethylene (4) lauryl ether, in an amount of from 2 to 6 % by weight, oleyl alcohol in an amount of from 2 to 6 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer. Most preferably, the guanfacine-containing layer comprises a polyethylene glycol $C_8$-$C_{20}$-alkyl ether having from 2 to 10 EO units, preferably polyoxyethylene (4) lauryl ether, in an amount of from 3 to 5 % by weight, oleyl alcohol in an amount of from 3 to 5 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer.

Accordingly, in another particularly preferred embodiment, the guanfacine-containing layer comprises a polyethylene glycol $C_8$-$C_{20}$-alkyl ether having from 2 to 10 EO units, preferably polyoxyethylene (4) lauryl ether, in an amount of from 2 to 6 % by weight, lauroglycol in an amount of from 2 to 9 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer. In another more particularly preferred embodiment, the guanfacine-containing layer comprises a polyethylene glycol $C_8$-$C_{20}$-alkyl ether having from 2 to 10 EO units, preferably polyoxyethylene (4) lauryl ether, in an amount of from 2 to 6 % by weight, lauroglycol in an amount of from 2 to 6 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer. Most preferably, the guanfacine-containing layer comprises a polyethylene glycol $C_8$-$C_{20}$-alkyl ether having from 2 to 10 EO units, preferably polyoxyethylene (4) lauryl ether, in an amount of from 3 to 5 % by weight, lauroglycol in an amount of from 3 to 5 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer.

Accordingly, in another particularly preferred embodiment, the guanfacine-containing layer comprises a polyethylene glycol $C_8$-$C_{20}$-alkyl ether having from 2 to 10 EO units, preferably polyoxyethylene (4) lauryl ether, in an amount of from 2 to 6 % by weight, oleoyl macrogol-6 glycerides (labrafil MS 1944) in an amount of from 2 to 9 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer. In another more particularly preferred embodiment, the guanfacine-containing layer comprises a polyethylene glycol $C_8$-$C_{20}$-alkyl ether having from 2 to 10 EO units, preferably polyoxyethylene (4) lauryl ether, in an amount of from 2 to 6 % by weight, oleoyl macrogol-6 glycerides (labrafil MS 1944) in an amount of from 2 to 6 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer. Most preferably, the guanfacine-containing layer comprises a polyethylene glycol $C_8$-$C_{20}$-alkyl ether having from 2 to 10 EO units, preferably polyoxyethylene (4) lauryl ether, in an amount of from 3 to 5 % by weight, oleoyl macrogol-6 glycerides (labrafil MS 1944) in an amount of from 3 to 5 % by weight, and optionally a polyvinyl caprolactam-polyvinyl acetate-polyethylene glycol graft copolymer, preferably as specified above, in an amount of from 0.5 to 5 % by weight, in each case based on the total weight of the guanfacine-containing layer.

In one embodiment of the invention, the area weight of the guanfacine-containing layer ranges from 40 to 250 $g/m^2$, preferably from 50 to 180 $g/m^2$, more preferably from 70 to 180 $g/m^2$, e.g. from 75 to 150 $g/m^2$ or from 100 to 150 $g/m^2$. In certain preferred embodiments, the area weight ranges from 80 to 120 $g/m^2$, preferably from 90 to 100 $g/m^2$.

In view of the above, the present invention relates in one embodiment to a transdermal therapeutic system for the transdermal administration of guanfacine comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of an acrylic polymer and at least one silicone-based polymer, wherein the acrylic polymer is present in an amount of from 20 to 55 % by weight and the at least one silicone-based polymer is present in an amount of from 20 to 55 % by weight, based on the total weight of the guanfacine-containing layer;
iii) at least one dispersing agent in an amount of from 2 to 6 % by weight, based on the total weight of the guanfacine-containing layer; and
iv) at least one permeation enhancer in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is a mixture of an acrylic polymer and at least one silicone-based polymer.

In a preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) a mixture of an acrylic polymer and at least one silicone-based polymer, wherein the acrylic polymer is present in an amount of from 20 to 55 % by weight and the at least one silicone-based polymer is present in an amount of from 20 to 55 % by weight, based on the total weight of the guanfacine-containing layer;
iii) polyoxyethylene (4) lauryl ether in an amount of from 2 to 6 % by weight, based on the total weight of the guanfacine-containing layer; and
iv) oleoyl macrogol-6 glycerides in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer,

preferably represents the skin contact layer and has pressure sensitive adhesive properties due to the at least one polymer, which is a mixture of an acrylic polymer and at least one silicone-based polymer.

In a preferred embodiment, the present invention relates in one embodiment to a transdermal therapeutic system for the transdermal administration of guanfacine comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) at least one dispersing agent in an amount of from 2 to 6 % by weight, based on the total weight of the guanfacine-containing layer; and
iv) at least one permeation enhancer in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is a mixture of two silicone-acrylic hybrid polymers.

In a preferred embodiment, the present invention relates in one embodiment to a transdermal therapeutic system for the transdermal administration of guanfacine comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-based polymers, wherein the first silicone-based polymer is present in an amount of from 20 to 55 % by weight, and the second silicone-based polymer is present in an amount of from 20 to 55 % by weight, based on the total weight of the guanfacine-containing layer;
iii) at least one dispersing agent in an amount of from 2 to 6 % by weight, based on the total weight of the guanfacine-containing layer; and
iv) at least one permeation enhancer in an amount of from 2 to 6 % by weight, based on the total weight of the guanfacine-containing layer

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is a mixture of two silicone-based polymers.

In a preferred embodiment, the present invention relates in one embodiment to a transdermal therapeutic system for the transdermal administration of guanfacine comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) an acrylic polymer in an amount of from 65 to 95 % by weight, based on the total weight of the guanfacine-containing layer;
iii) at least one dispersing agent in an amount of from 2 to 6 % by weight, based on the total weight of the guanfacine-containing layer; and
iv) at least one permeation enhancer in an amount of from 2 to 6 % by weight, based on the total weight of the

guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is an acrylic polymer.

In a preferred embodiment, the present invention relates in one embodiment to a transdermal therapeutic system for the transdermal administration of guanfacine comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) an acrylic polymer comprising a -OH group in an amount of from 65 to 95 % by weight, based on the total weight of the guanfacine-containing layer;
iii) at least one dispersing agent in an amount of from 2 to 6 % by weight, based on the total weight of the guanfacine-containing layer; and
iv) at least one permeation enhancer in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is an acrylic polymer comprising a -OH group.

In a more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of an acrylic polymer and at least one silicone-based polymer, wherein the acrylic polymer is present in an amount of from 20 to 55 % by weight and the at least one silicone-based polymer is present in an amount of from 20 to 55 % by weight, based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is a mixture of an acrylic polymer and at least one silicone-based polymer.

In another more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of

from 1 to 20 % by weight, in each case based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is a mixture of two silicone-acrylic hybrid polymers.

In another more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-based polymers, wherein the first silicone-based polymer is present in an amount of from 20 to 55 % by weight, and the second silicone-based polymer is present in an amount of from 20 to 55 % by weight, based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is a mixture of two silicone-based polymers.
In an even more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) a mixture of two silicone-based polymers, wherein the first silicone-based polymer is present in an amount of from 20 to 55 % by weight, and the second silicone-based polymer is present in an amount of from 20 to 55 % by weight, based on the total weight of the guanfacine-containing layer;
iii) polyoxyethylene (4) lauryl ether in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is a mixture of two silicone-based polymers.

In another more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) an acrylic polymer in an amount of from 65 to 95 % by weight, based on the total weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is an acrylic polymer.
In an even more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) an acrylic polymer in an amount of from 65 to 95 % by weight, based on the total weight of the guanfacine-containing layer;
iii) polyoxyethylene (4) lauryl ether in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is an acrylic polymer.
In another more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) an acrylic polymer comprising a -OH group in an amount of from 65 to 95 % by weight, based on the weight of the guanfacine-containing layer;
iii) polyethylene glycol $C_8$-$C_{20}$-alkyl ether having from 2 to 10 EO units in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is an acrylic polymer comprising a -OH group.
In an even more preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and

B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) an acrylic polymer comprising a -OH group in an amount of from 65 to 95 % by weight, based on the total weight of the guanfacine-containing layer;
iii) polyoxyethylene (4) lauryl ether in an amount of from 2 to 6 % by weight, based on the total weight of the guanfacine-containing layer; and
iv) oleyl alcohol in an amount of from 2 to 6 % by weight, based on the total weight of the guanfacine-containing layer.

In connection with this embodiment, it is further preferred that the guanfacine-containing layer structure does not comprise an additional skin-contact layer. Thus, the guanfacine-containing layer, preferably the guanfacine-containing matrix layer, preferably represents the skin contact layer and has pressure sensitive adhesive properties due to the at least one polymer, which is an acrylic polymer comprising a -OH group.

In particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid;
ii) a mixture of two silicone-acrylic hybrid polymers;
iii) polyoxyethylene (4) lauryl ether; and
iv) a permeation enhancer selected from oleyl alcohol, lauroglycol and oleoyl macrogol-6 glycerides.

In particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid;
ii) a mixture of two silicone-acrylic hybrid polymers;
iii) polyoxyethylene (4) lauryl ether; and
iv) oleyl alcohol.

In another particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid;
ii) a mixture of two silicone-acrylic hybrid polymers;
iii) polyoxyethylene (4) lauryl ether; and
iv) lauroglycol.

In another particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid;
ii) a mixture of two silicone-acrylic hybrid polymers;
iii) polyoxyethylene (4) lauryl ether; and
iv) oleoyl macrogol-6 glycerides.

In an even more particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) a permeation enhancer selected from oleyl alcohol, lauroglycol and oleoyl macrogol-6 glycerides in an amount of from 2 to 6 % by weight, based on the total weight of the guanfacine-containing layer.

In another even more particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) a permeation enhancer selected from oleyl alcohol, lauroglycol and oleoyl macrogol-6 glycerides in an amount of from 2 to 6 % by weight, based on the total weight of the guanfacine-containing layer.

In an even more particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) oleyl alcohol in an amount of from 2 to 6 % by weight, based on the total weight of the guanfacine-containing layer.

In another even more particularly preferred embodiment, the present invention relates in one embodiment to a transdermal therapeutic system for the transdermal administration of guanfacine, comprising a guanfacine-containing

layer structure, said guanfacine-containing layer structure comprising:

A) a backing layer; and
B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) oleyl alcohol in an amount of from 2 to 6 % by weight, based on the total weight of the guanfacine-containing layer.

In another even more particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) lauroglycol in an amount of from 2 to 6 % by weight, based on the total weight of the guanfacine-containing layer.

In another even more particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) lauroglycol in an amount of from 2 to 6 % by weight, based on the total weight of the guanfacine-containing layer.

In another even more particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine in an amount of from 3 to 16 % by weight and sorbic acid in an amount of from 1 to 7.2 % by weight, based on the total weight of the guanfacine-containing layer;

ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) oleoyl macrogol-6 glycerides in an amount of from 2 to 6 % by weight, based on the total weight of the guanfacine-containing layer.

In another even more particularly preferred embodiment, the present invention relates in one embodiment to a transdermal therapeutic system for the transdermal administration of guanfacine, comprising a guanfacine-containing layer structure, said guanfacine-containing layer structure comprising:

A) a backing layer; and
B) a guanfacine-containing layer, preferably a guanfacine-containing matrix layer, comprising

i) guanfacine and sorbic acid in an overall amount of from 6 to 9 % by weight, based on the total weight of the guanfacine-containing layer;
ii) a mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of the guanfacine-containing layer;
iii) polyoxyethylene (4) lauryl ether in an amount of from 2 to 6 % by weight, based on the total weight of the guanfacine-containing layer; and
iv) oleoyl macrogol-6 glycerides in an amount of from 2 to 6 % by weight, based on the total weight of the guanfacine-containing layer.

For the above preferred and more preferred embodiments it is further preferred that the area weight of the guanfacine-containing layer ranges from 50 to 180 g/m$^2$, preferably from 75 to 150 g/m$^2$, more preferably from 80 to 120 g/m$^2$.

## GUANFACINE

The TTS according to the invention comprises a guanfacine-containing layer structure, said guanfacine-containing layer structure comprising A) a backing layer; and B) a guanfacine-containing layer comprising guanfacine and a mono-carboxylic acid. The guanfacine-containing layer, which is preferably a guanfacine-containing matrix layer, has been described in detail above.

In one embodiment of the invention, the guanfacine in the guanfacine-containing layer, preferably the guanfacine-containing matrix layer, is present in a pre-mixture with a mono-carboxylic acid. In one embodiment of the invention, the guanfacine-containing layer, preferably the guanfacine-containing matrix layer comprises guanfacine and a mono-carboxylic acid or a salt formed from the guanfacine and the mono-carboxylic acid. Preferably, the guanfacine and the mono-carboxylic acid are present in the form of a co-salt. Further, it is to be understood that the guanfacine and the mono-carboxylic acid are pre-mixed prior to the addition to the guanfacine-containing layer, such that a co-salt is formed.

Preferably, the guanfacine in the present invention is present in the form of a "co-salt", meaning that the guanfacine is present in a pre-mixture together with at least one mono-carboxylic acid, such that a proton transfer is possible, and preferably at least partly a guanfacine salt of the mono-carboxylic acid is formed. In other words, the active agent, preferably guanfacine, may at least partly be present in protonated from. However, in connection with the active agent, preferably guanfacine, and the mono-carboxylic acid it is to be understood that any form obtained in the guanfacine-containing layer based on the pre-mixture of guanfacine and the mono-carboxylic acid is covered by the present invention, including, e.g., the option of the guanfacine and the mono-carboxylic acid co-existing without chemical interaction, i.e. proton transfer, as well as the option of the formation of a co-salt as described above. Preferably, the guanfacine free base and the mono-carboxylic acid may form together a co-salt or any other type of acid addition salt, so that the guanfacine-containing layer preferably comprises at least partly a co-salt or any other type of acid addition salt of guanfacine free base and the mono-carboxylic acid. In other words, the guanfacine-containing layer preferably comprises the guanfacine at least partly in protonated form and the mono-carboxylic acid at least partly in deprotonated form.

In a preferred embodiment of the invention, the guanfacine and the mono-carboxylic acid in the guanfacine-containing layer are present in the form of a pre-mixture as described above.

In a more preferred embodiment of the invention, the guanfacine and the mono-carboxylic acid in the guanfacine-containing layer are present in the form of a pre-mixture, wherein said pre-mixture is obtainable by a dry-grinding method or a slurry method.

In one preferred embodiment of the invention, the guanfacine and the mono-carboxylic acid are present in the guanfacine-containing layer in equimolar amounts. In this connection, it is to be understood that the guanfacine free base as added to the pre-mixture and the mono-carboxylic acid as added to the pre-mixture are also present in equimolar amounts.

In a preferred embodiment of the invention, the guanfacine-containing layer comprises the mono-carboxylic acid in an amount of from 1% to 20%, more preferably in an amount of from 2% to 16% by weight, based on the total weight of the guanfacine-containing layer. A particularly preferred mono-carboxylic acid, which is comprised together with the guanfacine in the guanfacine-containing layer is sorbic acid.

In connection with the above embodiments, it is surprising that the combination of guanfacine with a mono-carboxylic acid, in particular with the mono-carboxylic acid sorbic acid, enhances the flux and permeation of the transdermal therapeutic system. In particular, it has surprisingly been found by the inventors of the present invention that a TTS comprising a combination of guanfacine base and sorbic acid in the guanfacine-containing layer shows an enhanced cumulative permeated amount over 88 h in comparison to a TTS comprising only guanfacine in the form of the free base in the guanfacine-containing layer.

In one preferred embodiment of the invention, the guanfacine-containing layer structure preferably contains a therapeutically effective amount of guanfacine. More preferably, the therapeutically effective amount of guanfacine is present in the guanfacine-containing layer of the guanfacine-containing layer structure.

In certain embodiments, the amount of guanfacine in the guanfacine-containing layer ranges from 1 to 20 % by weight, preferably from 2 to 16 % by weight, most preferably from 5 to 13 % by weight, e.g. from 11 to 13 % by weight or from 5 to 7 % by weight, based on the total weight of the guanfacine-containing layer.

In one embodiment of the invention, the guanfacine-containing layer is obtainable by dispersing the guanfacine - mono-carboxylic acid pre-mixture. If the guanfacine-containing layer is a guanfacine-containing matrix layer, said layer is preferably obtainable by dispersing the guanfacine - mono-carboxylic acid pre-mixture in the polymeric carrier, which particularly preferably comprises at least one polymer selected from the group consisting of acrylic polymers, silicone-based polymers, silicone-acrylic hybrid polymers, and mixtures thereof, and optionally at least one additive as defined above, in particular at least one dispersing agent and at least one permeation enhancer.

In certain embodiments, the guanfacine has a purity of at least 95 %, preferably of at least 98 %, and more preferably of at least 99 % as determined by quantitative HPLC. Quantitative HPLC may be performed with Reversed-Phase-HPLC with UV detection.

## POLYMERS

### SILICONE ACRYLIC HYBRID POLYMER

The TTS according to the present invention comprises at least one polymer, wherein the at least polymer is selected from the group consisting of acrylic polymers, silicone-based polymers, silicone-acrylic hybrid polymers, and mixtures thereof. Further details regarding the mixtures of the polymers are provided below.

In one embodiment of the present invention the TTS according to the present invention comprises a silicone acrylic hybrid polymer. The silicone acrylic hybrid polymer comprises a polymerized hybrid species that includes silicone-based sub-species and acrylate-based sub-species that have been polymerized together. The silicone acrylic hybrid polymer thus comprises a silicone phase and an acrylic phase. Preferably, the silicone acrylic hybrid polymer is a silicone acrylic hybrid pressure-sensitive adhesive.

The silicone acrylic hybrid pressure-sensitive adhesives are usually supplied and used in solvents like n-heptane and ethyl acetate. The solids content of the pressure-sensitive adhesives is usually between 30 % and 80 %. The skilled person is aware that the solids content may be modified by adding a suitable amount of solvent.

Preferably, the weight ratio of silicone to acrylate in the silicone acrylic hybrid pressure-sensitive adhesive is from 5:95 to 95:5, or from 20:80 to 80:20, more preferably from 40:60 to 60:40, and most preferably the ratio of silicone to acrylate is about 50:50. Suitable silicone acrylic hybrid pressure-sensitive adhesives having a weight ratio of silicone to acrylate of 50:50 are, for example, the commercially available silicone acrylic hybrid pressure-sensitive adhesives 7-6102, Silicone/Acrylate Ratio 50/50, and 7-6302, Silicone/Acrylate Ratio 50/50, supplied in ethyl acetate by DuPont™.

The preferred silicone acrylic hybrid pressure-sensitive adhesives in accordance with the invention are characterized by a solution viscosity at 25 °C and about 50 % solids content in ethyl acetate of more than about 400 cP, or from about 500 cP to about 3,500 cP, in particular from about 1,000 cP to about 3,000 cP, more preferred from about 1,200 cP to about 1,800, or most preferred of about 1,500 cP or alternatively more preferred from about 2,200 cP to about 2,800 cP, or most preferred of about 2,500 cP, preferably as measured using a Brookfield RVT viscometer equipped with a spindle number 5 at 50 RPM. It is noted that 1 cP converts to 1 mPa.s.

These silicone acrylic hybrid pressure-sensitive adhesives may also be characterized by a complex viscosity at 0.1 rad/s at 30 °C of less than about 1.0e9 Poise, or from about 1.0e5 Poise to about 9.0e8 Poise, or more preferred from

about 9.0e5 Poise to about 1.0e7 Poise, or most preferred about 4.0e6 Poise, or alternatively more preferred from about 2.0e6 Poise to about 9.0e7 Poise, or most preferred about 1.0e7 Poise, preferably as measured using a Rheometrics ARES rheometer, wherein the rheometer is equipped with 8mm plates and the gap zeroed.

To prepare samples for measuring the rheological behavior using a Rheometrics ARES rheometer, between 2 and 3 grams of adhesive solution can be poured onto a SCOTCH-PAK 1022 fluoropolymer release liner and allow to sit for 60 minutes under ambient conditions. To achieve essentially solvent-free films of the adhesive, they can be placed in an oven at 110°C +/-10°C for 60 minutes. After removing from the oven and letting equilibrate to room temperature. The films can be removed from the release liner and folded over to form a square. To eliminate air bubbles the films can be compressed using a Carver press. The samples can then be loaded between the plates and are compressed to 1.5 +/-0.1 mm at 30°C. The excess adhesive is trimmed, and the final gap recorded. A frequency sweep between 0.01 to 100 rad/s can be performed with the following settings: Temperature = 30°C; strain = 0.5-1% and data collected at 3 points/decade.

Suitable silicone acrylic hybrid pressure-sensitive adhesives which are commercially available include the PSA series 7-6100 and 7-6300 manufactured and supplied in n-heptane or ethyl acetate by DuPont™ (7-610X and 7-630X; X=1 n-heptane-based / X=2 ethyl acetate-based). For example, the 7-6102 silicone acrylic hybrid PSA having a silicone/acrylate ratio of 50/50 is characterized by a solution viscosity at 25°C and about 50% solids content in ethyl acetate of 2,500 cP and a complex viscosity at 0.1 rad/s at 30°C of 1.0e7 Poise. The 7-6302 silicone acrylic hybrid PSA having a silicone/acrylate ratio of 50/50 has a solution viscosity at 25°C and about 50% solids content in ethyl acetate of 1,500 cP and a complex viscosity at 0.1 rad/s at 30°C of 4.0e6 Poise.

Depending on the solvent in which the silicone acrylic hybrid pressure-sensitive adhesive is supplied, the arrangement of the silicone phase and the acrylic phase providing a silicone or acrylic continuous external phase and a corresponding discontinuous internal phase is different. If the silicone acrylic hybrid pressure-sensitive adhesive is provided in n-heptane, the composition contains a continuous, silicone external phase and a discontinuous, acrylic internal phase. If the silicone acrylic hybrid pressure-sensitive adhesive is provided in ethyl acetate, the composition contains a continuous, acrylic external phase and a discontinuous, silicone internal phase. After evaporating the solvent in which the silicone acrylic hybrid pressure-sensitive adhesive is provided, the phase arrangement of the resulting pressure-sensitive adhesive film or layer corresponds to the phase arrangement of the solvent-containing adhesive coating composition. For example, in the absence of any substance that may induce an inversion of the phase arrangement in a silicone acrylic hybrid pressure sensitive adhesive composition, a pressure-sensitive adhesive layer prepared from a silicone acrylic hybrid pressure-sensitive adhesive in n-heptane provides a continuous, silicone external phase and a discontinuous, acrylic internal phase, a pressure-sensitive adhesive layer prepared from a silicone acrylic hybrid pressure-sensitive adhesive in ethyl acetate provides a continuous, acrylic external phase and a discontinuous, silicone internal phase. The phase arrangement of the compositions can, for example, be determined in peel force tests with pressure-sensitive adhesive films or layers prepared from the silicone acrylic hybrid PSA compositions which are attached to a siliconized release liner. The pressure-sensitive adhesive film contains a continuous, silicone external phase if the siliconized release liner cannot or can only hardly be removed from the pressure-sensitive adhesive film (laminated to a backing film) due to the blocking of the two silicone surfaces. Blocking results from the adherence of two silicone layers which comprise a similar surface energy. The silicone adhesive shows a good spreading on the siliconized liner and therefore can create a good adhesion to the liner. If the siliconized release liner can easily be removed the pressure-sensitive adhesive film contains a continuous, acrylic external phase. The acrylic adhesive has no good spreading due to the different surface energies and thus has a low or almost no adhesion to the siliconized liner. Preferably, according to the present invention the silicone acrylic hybrid pressure-sensitive adhesive is provided in ethyl acetate and the composition contains a continuous, acrylic external phase and a discontinuous, silicone internal phase.

According to a preferred embodiment of the invention the silicone acrylic hybrid polymer is a silicone acrylic hybrid pressure-sensitive adhesive obtainable from a silicon-containing pressure-sensitive adhesive composition comprising acrylate or methacrylate functionality. It is to be understood that the silicon-containing pressure-sensitive adhesive composition comprising acrylate or methacrylate functionality can include only acrylate functionality, only methacrylate functionality, or both acrylate functionality and methacrylate functionality.

According to certain embodiments of the invention the silicone acrylic hybrid pressure-sensitive adhesive comprises the reaction product of (a) a silicon-containing pressure-sensitive adhesive composition comprising acrylate or methacrylate functionality, (b) an ethylenically unsaturated monomer, and (c) an initiator. That is, the silicone acrylic hybrid pressure-sensitive adhesive is the product of the chemical reaction between these reactants ((a), (b), and (c)). In particular, the silicone acrylic hybrid pressure-sensitive adhesive includes the reaction product of (a) a silicon-containing pressure-sensitive adhesive composition comprising acrylate or methacrylate functionality, (b) a (meth)acrylate monomer, and (c) an initiator (i.e., in the presence of the initiator). That is, the silicone acrylic hybrid pressure-sensitive adhesive includes the product of the chemical reaction between these reactants ((a), (b), and (c)).

The reaction product of (a) a silicon-containing pressure-sensitive adhesive composition comprising acrylate or methacrylate functionality, (b) an ethylenically unsaturated monomer, and (c) an initiator may contain a continuous, silicone external phase and a discontinuous, acrylic internal phase or the reaction product of (a), (b), and (c) may contain a

continuous, acrylic external phase and a discontinuous, silicone internal phase.

The silicon-containing pressure-sensitive adhesive composition comprising acrylate or methacrylate functionality (a) is typically present in the silicone acrylic hybrid pressure-sensitive adhesive in an amount of from 5 to 95, more typically 25 to 75, parts by weight based on 100 parts by weight of the hybrid pressure-sensitive adhesive.

The ethylenically unsaturated monomer (b) is typically present in the silicone acrylic hybrid pressure-sensitive adhesive in an amount of from 5 to 95, more typically 25 to 75, parts by weight based on 100 parts by weight of the hybrid pressure-sensitive adhesive.

The initiator (c) is typically present in the silicone acrylic hybrid pressure-sensitive adhesive in an amount of from 0.005 to 3, more typically from 0.01 to 2, parts by weight based on 100 parts by weight of the hybrid pressure-sensitive adhesive.

According to certain embodiments of the invention the silicon-containing pressure-sensitive adhesive composition comprising acrylate or methacrylate functionality (a) comprises the condensation reaction product of (a1) a silicone resin, (a2) a silicone polymer, and (a3) a silicon-containing capping agent which provides said acrylate or methacrylate functionality.

According to certain embodiments of the invention the silicon-containing pressure-sensitive adhesive composition comprising acrylate or methacrylate functionality (a) comprises the condensation reaction product of:

(a1) a silicone resin,
(a2) a silicone polymer, and
(a3) a silicon-containing capping agent which provides said acrylate or methacrylate functionality, wherein said silicon-containing capping agent is of the general formula $XYR'_3SiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts *in-situ* with the silicone resin and silicone polymer.

According to certain embodiments of the invention the silicon-containing pressure-sensitive adhesive composition comprising acrylate or methacrylate functionality comprises the condensation reaction product of a pressure sensitive adhesive and a silicon-containing capping agent which provides said acrylate or methacrylate functionality. That is, the silicon-containing pressure sensitive adhesive composition comprising acrylate or methacrylate functionality is essentially a pressure sensitive adhesive that has been capped or endblocked with the silicon-containing capping agent which provides said acrylate or methacrylate functionality, wherein the pressure sensitive adhesive comprises the condensation reaction product of the silicone resin and the silicone polymer. Preferably, the silicone resin reacts in an amount of from 30 to 80 parts by weight to form the pressure sensitive adhesive, and the silicone polymer reacts in an amount of from 20 to 70 parts by weight to form the pressure sensitive adhesive. Both of these parts by weight are based on 100 parts by weight of the pressure sensitive adhesive. Although not required, the pressure sensitive adhesive may comprise a catalytic amount of a condensation catalyst. A wide array of silicone resins and silicone polymers are suitable to make up the pressure sensitive adhesive.

According to certain embodiments of the invention the silicone acrylic hybrid pressure-sensitive adhesive is the reaction product of:

(a) a silicon-containing pressure-sensitive adhesive composition comprising acrylate or methacrylate functionality that comprises the condensation reaction product of:

(a1) a silicone resin,
(a2) a silicone polymer, and
(a3) a silicon-containing capping agent which provides said acrylate or methacrylate functionality, wherein said

silicon-containing capping agent is of the general formula $XYR'_3SiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(b) an ethylenically unsaturated monomer; and
(c) an initiator.

The silicone acrylic hybrid composition used in the present invention may be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality, wherein said silicon-containing capping agent is of the general formula $XYR'_3SiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in the presence of an initiator to form a silicone acrylic hybrid composition, optionally at a temperature of from 50°C to 100°C, or from 65°C to 90°C.

During the polymerization of the ethylenically unsaturated monomer and the silicon-containing pressure-sensitive adhesive composition comprising acrylate or methacrylate functionality, the silicone to acrylic ratio can be controlled and optimized as desired. The silicone to acrylic ratio can be controlled by a wide variety of mechanisms in and during the method. An illustrative example of one such mechanism is the rate controlled addition of the ethylenically unsaturated monomer or monomers to the silicon-containing pressure-sensitive adhesive composition comprising acrylate or methacrylate functionality. In certain applications, it may be desirable to have the silicone-based sub-species, or the overall silicone content, to exceed the acrylate-based sub-species, or the overall acrylic content. In other applications, it may be desirable for the opposite to be true. Independent of the end application, it is generally preferred,

as already described above, that the silicon-containing pressure-sensitive adhesive composition comprising acrylate or methacrylate functionality is preferably present in the silicone acrylic hybrid composition in an amount of from about 5 to about 95, more preferably from about 25 to about 75, and still more preferably from about 40 to about 60 parts by weight based on 100 parts by weight of the silicone acrylic hybrid composition.

According to a certain embodiment of the invention, the silicone acrylic hybrid composition used in the present invention may be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality, wherein said silicon-containing capping agent is of the general formula $XYR'_3SiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in a first solvent in the presence of an initiator at a temperature of from 50°C to 100°C to form a silicone acrylic hybrid composition;
(iii) removing the first solvent; and
(iv) adding a second solvent to form the silicone acrylic hybrid composition, wherein the phase arrangement of the silicone acrylic hybrid composition is selectively controlled by selection of the second solvent.

The silicone acrylic hybrid PSA composition used in the present invention may also be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality, wherein said silicon-containing capping agent is of the general formula $XYR'_3SiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or
after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in a first solvent in the presence of an initiator at a temperature of from 50°C to 100°C to form a silicone acrylic hybrid composition;
(iii) adding a processing solvent, wherein the processing solvent has a higher boiling point than the first solvent, and
(iv) applying heat at a temperature of from 70°C to 150°C such that a majority of the first solvent is selectively removed;
(v) removing the processing solvent; and.
(vi) adding a second solvent to form the silicone acrylic hybrid composition, wherein the phase arrangement of the silicone acrylic hybrid composition is selectively controlled by selection of the second solvent.

The silicone resin according to the previous paragraphs may contain a copolymer comprising triorganosiloxy units of the formula $R^x{}_3SiO_{1/2}$ and tetrafunctional siloxy units of the formula $SiO_{4/2}$ in a ratio of from 0.1 to 0.9, preferably of about 0.6 to 0.9, triorganosiloxy units for each tetrafunctional siloxy unit. Preferably, each $R^X$ independently denotes a monovalent hydrocarbon radical having from 1 to 6 carbon atoms, vinyl, hydroxyl, or phenyl groups.

The silicone polymer according to the previous paragraphs may comprise at least one polydiorganosiloxane and is preferably end-capped (end-blocked) with a functional group selected from the group consisting of hydroxyl groups, alkoxy groups, hydride groups, vinyl groups, or mixtures thereof. The diorganosubstituent may be selected from the group consisting of dimethyl, methylvinyl, methylphenyl, diphenyl, methylethyl, (3,3,3-trifluoropropyl)methyl and mixtures thereof. Preferably, the diorganosubstituents contain only methyl groups. The molecular weight of polydiorganosiloxane will typically range from about 50,000 to about 1,000,000, preferably, from about 80,000 to about 300,000. Preferably, the polydiorganosiloxane comprises $AR^XSiO$ units terminated with endblocking $TR^XASiO_{1/2}$ units, wherein the polydiorganosiloxane has a viscosity of from about 100 centipoise to about 30,000,000 centipoise at 25°C, each A radical is independently selected from $R^X$ or halohydrocarbon radicals having from 1 to 6 carbon atoms, each T radical is independently selected from the group consisting of $R^X$, OH, H or $OR^Y$, and each $R^Y$ is independently an alkyl radical having from 1 to 4 carbon atoms.

As an example, using forms of the preferred silicone resin and the preferred silicone polymer, one type of pressure sensitive adhesive is made by:
mixing (i) from 30 to 80 inclusive parts by weight of at least one resin copolymer containing silicon-bonded hydroxyl radicals and consisting essentially of $R^x{}_3SiO_{1/2}$ units and $SiO_{4/2}$ units in a mole ratio of 0.6 to 0.9 $R^x{}_3SiO_{1/2}$ units for each $SiO_{4/2}$ unit present, (ii) between about 20 and about 70 parts by weight of at least one polydiorganosiloxane comprising $AR^XSiO$ units terminated with endblocking $TR^XASiO_{1/2}$ units, wherein the polydiorganosiloxane has a viscosity of from about 100 centipoise to about 30,000,000 centipoise at 25°C and each $R^X$ is a monovalent organic radical selected from the group consisting of hydrocarbon radicals of from 1 to 6 inclusive carbon atoms, each A radical is independently selected from $R^X$ or halohydrocarbon radicals having from 1 to 6 inclusive carbon atoms, each T radical is independently selected from the group consisting of $R^X$, OH, H or $OR^Y$, and each $R^Y$ is independently an alkyl radical of from 1 to 4 inclusive carbon atoms; a sufficient amount of (iii) at least one of the silicon-containing capping agents, also referred to throughout as endblocking agents, described below and capable of providing a silanol content, or concentration, in the range of 5,000 to 15,000, more typically 8,000 to 13,000, ppm, when desirable an additional catalytic amount of (iv) a mild silanol condensation catalyst in the event that none is provided by (ii), and when necessary, an effective amount of (v) an organic solvent which is inert with respect to (i), (ii), (iii) and (iv) to reduce the viscosity of a mixture of (i), (ii), (iii), and (iv), and condensing the mixture of (i), (ii), (iii) and (iv) at least until a substantial amount of the silicon-containing capping agent or agents have reacted with the silicon-bonded hydroxyl radicals and T radicals of (i) and (ii). Additional organosilicon endblocking agents can be used in conjunction with the silicon-containing capping agent or agents (iii) of the present invention.

The silicon-containing capping agent according to the previous paragraphs may be selected from the group of acrylate functional silanes, acrylate functional silazanes, acrylate functional disilazanes, acrylate functional disiloxanes, methacrylate functional silanes, methacrylate functional silazanes, methacrylate functional disilazanes, meth-acrylate functional disiloxanes, and combinations thereof and may be described as to be of the general formula $XYR'_3SiZ_{3-b}$, wherein X is a monovalent radical of the general formula AE- where E is -O- or - NH- and A is an acryl group or a methacryl group, Y is a divalent alkylene radical having from 1 to 6 carbon atoms, R' is a methyl or a phenyl radical, Z is a monovalent hydrolyzable organic radical or a halogen, and b is 0, 1 or 2. Preferably, the monovalent hydrolyzable organic radical is of the general formula R"0 - where R" is an alkylene radical. Most preferably, this particular endblocking agent is selected from the group of 3-methacryloxypropyldimethylchlorosilane, 3-methacryloxypropyldichlorosilane, 3-methacryloxypropyltrichlorosilane, 3-methacryloxypropyldimethylmethoxysilane, 3-methacryloxypropylmethyldimethoxysilane, 3-methacryloxypropyltrimethoxysilane, 3-methacryloxypropyldimethylethoxysilane, 3-methacryloxypropylmethyldiethoxysilane, 3-methacryloxypropyltriethoxysilane, (methacryloxymethyl)dimethylmethoxysilane, (methacryloxymethyl)methyldimethoxysilane, (methacryloxymethyl)trimethoxysilane, (methacryloxymethyl)dimethylethoxysilane, (methacryloxymethyl)methyldiethoxysilane, methacryloxymethyltriethoxysilane, methacryloxy-propyltriisopropoxysilane, 3-methacryloxypropyldimethylsilazane, 3-acryloxy-propyldimethylchlorosilane, 3-acryloxypropyldichlorosilane, 3-acryloxypropyltrichlorosilane, 3-acryloxypropyldimethylmethoxysilane, 3-acryloxy-propylmethyldimethoxysilane, 3-acryloxypropyltrimethoxysilane, 3-acryloxypropyl-dimethylsilazane, and combinations thereof.

The ethylenically unsaturated monomer according to the previous paragraphs can be any monomer having at least one carbon-carbon double bond. Preferably, the ethylenically unsaturated monomer according to the previous paragraphs may be a compound selected from the group consisting of aliphatic acrylates, aliphatic methacrylates, cycloaliphatic acrylates, cycloaliphatic methacrylates, and combinations thereof. It is to be understood that each of the compounds, the aliphatic acrylates, the aliphatic methacrylates, the cycloaliphatic acrylates, and the cycloaliphatic methacrylates, include an alkyl radical. The alkyl radicals of these compounds can include up to 20 carbon atoms. The aliphatic acrylates that may be selected as one of the ethylenically unsaturated monomers are selected from the group consisting of methyl acrylate, ethyl acrylate, propyl acrylate, n-butyl acrylate, iso-butyl acrylate, tert-butyl acrylate, hexyl acrylate, 2-ethylhexyl acrylate, iso-octyl acrylate, iso-nonyl acrylate, iso-pentyl acrylate, tridecyl acrylate, stearyl acrylate, lauryl acrylate, and mixtures thereof. The aliphatic methacrylates that may be selected as one of the ethylenically unsaturated monomers are selected from the group consisting of methyl methacrylate, ethyl methacrylate, propyl methacrylate, n-butyl methacrylate, iso-butyl methacrylate, tert-butyl methacrylate, hexyl methacrylate, 2-eth-ylhexyl methacrylate, iso-octyl methacrylate, iso-nonyl methacrylate, iso-pentyl methacrylate, tridecyl methacrylate, stearyl methacrylate, lauryl methacrylate, and mixtures thereof. The cycloaliphatic acrylate that may be selected as one of the ethylenically unsaturated monomers is cyclohexyl acrylate, and the cycloaliphatic methacrylate that may be selected as one of the ethylenically unsaturated monomers is cyclohexyl methacrylate.

It is to be understood that the ethylenically unsaturated monomer used for preparing the silicone acrylic hybrid pressure sensitive adhesive may be more than one ethylenically unsaturated monomer. That is, a combination of ethylenically unsaturated monomers may be polymerized, more specifically co-polymerized, along with the silicon-containing pressure sensitive adhesive composition comprising acrylate or methacrylate functionality and the initiator. According to a certain embodiment of the invention, the silicone acrylic hybrid pressure-sensitive adhesive is prepared by using at least two different ethylenically unsaturated monomers, preferably selected from the group of 2-ethylhexyl acrylate and methyl acrylate, more preferably in a ratio of 50% 2-ethylhexyl acrylate and 50% methyl acrylate, or in a ratio of 60% 2-ethylhexyl acrylate and 40% methyl acrylate as the acrylic monomer.

The initiator according to the previous paragraphs may be any substance that is suitable to initiate the polymerization of the silicon-containing pressure sensitive adhesive composition comprising acrylate or methacrylate functionality and the ethylenically unsaturated monomer to form the silicone acrylic hybrid. For example, free radical initiators selected from the group of peroxides, azo compounds, redox initiators, and photo-initiators may be used.

Further suitable silicone resins, silicone polymers, silicon-containing capping agents, ethylenically unsaturated monomers, and initiators that can be used in accordance with the previous paragraphs are detailed in WO 2007/145996, EP 2 599 847 A1, and WO 2016/130408.

According to a certain embodiment of the invention, the silicone acrylic hybrid polymer comprises a reaction product of a silicone polymer, a silicone resin and an acrylic polymer, wherein the acrylic polymer is covalently self-crosslinked and covalently bound to the silicone polymer and/or the silicone resin.

According to a certain other embodiment of the invention, the silicone acrylic hybrid polymer comprises a reaction product of a silicone polymer, a silicone resin and an acrylic polymer, wherein the silicone resin contains triorganosiloxy units $R_3SiO_{1/2}$ where R is an organic group, and tetrafunctional siloxy units $SiO_{4/2}$ in a mole ratio of from 0.1 to 0.9 $R_3SiO_{1/2}$ units for each $SiO_{4/2}$.

The acrylic polymer may comprise at least an alkoxysilyl functional monomer, polysiloxane-containing monomer, halosilyl functional monomer or alkoxy halosilyl functional monomer. Preferably, the acrylic polymer is prepared from alkoxysilyl functional monomers selected from the group consisting of trialkoxylsilyl (meth)acrylates, dialkoxyalkylsilyl (meth)acrylates, and mixtures thereof, or comprises end-capped alkoxysilyl functional groups. The alkoxysilyl functional groups may preferably be selected from the group consisting of trimethoxylsilyl groups, dimethoxymethylsilyl groups, triethoxylsilyl, diethoxymethylsilyl groups and mixtures thereof.

The acrylic polymer may also be prepared from a mixture comprising polysiloxane-containing monomers, preferably from a mixture comprising polydimethylsiloxane mono (meth)acrylate.

The silyl functional monomers will typically be used in amounts of from 0.2 to 20 weight percent of the acrylic polymer, more preferably the amount of silyl functional monomers will range from about 1.5 to about 5 weight percent of the acrylic polymer.

The polysiloxane-containing monomer will typically be used in amounts of from 1.5 to 50 weight percent of the acrylic polymer, more preferably the amount of polysiloxane-containing monomers will range from 5 to 15 weight percent of the acrylic polymer.

Alternatively, the acrylic polymer comprises a block or grafted copolymer of acrylic and polysiloxane. An example

of a polysiloxane block copolymer is polydimethylsiloxane-acrylic block copolymer. The preferred amount of siloxane block is 10 to 50 weight percent of the whole block polymer.

The acrylic polymer comprises alkyl (meth)acrylate monomers. Preferred alkyl (meth)acrylates which may be used have up to about 18 carbon atoms in the alkyl group, preferably from 1 to about 12 carbon atoms in the alkyl group. Preferred low glass transition temperature (Tg) alkyl acrylate with a homopolymer Tg of less than about 0°C have from about 4 to about 10 carbon atoms in the alkyl group and include butyl acrylate, amyl acrylate, hexyl acrylate, 2-ethylhexyl acrylate, octyl acrylate, isooctyl acrylate, decyl acrylate, isomers thereof, and combinations thereof. Particularly preferred are butyl acrylate, 2-ethylhexyl acrylate and isooctyl acrylate. The acrylic polymer components may further comprise (meth)acrylate monomers having a high Tg such as methyl acrylate, ethyl acrylate, methyl methacrylate and isobutyl methacrylate.

The acrylic polymer component may further comprise a polyisobutylene group to improve cold flow properties of the resultant adhesive.

The acrylic polymer components may comprise nitrogen-containing polar monomers. Examples include N-vinyl pyrrolidone, N-vinyl caprolactam, N-tertiary octyl acrylamide, dimethyl acrylamide, diacetone acrylamide, N-tertiary butyl acrylamide, N-isopropyl acrylamide, cyanoethylacrylate, N-vinyl acetamide and N-vinyl formamide.

The acrylic polymer component may comprise one or more hydroxyl containing monomers such as 2-hydroxyethyl acrylate, 2-hydroxyethyl methacrylate, hydroxypropyl acrylate and/or hydroxypropyl methacrylate.

The acrylic polymer components may, if desired, comprise carboxylic acid containing monomers. Useful carboxylic acids preferably contain from about 3 to about 6 carbon atoms and include, among others, acrylic acid, methacrylic acid, itaconic acid, β-carboxyethyl acrylate and the like. Acrylic acid is particularly preferred.

Other useful, well known co-monomers include vinyl acetate, styrene, cyclohexyl acrylate, alkyl di(meth) acrylates, glycidyl methacrylate and allyl glycidyl ether, as well as macromers such as, for example, poly(styryl) methacrylate.

One acrylic polymer component that can be used in the practice of the invention is an acrylic polymer that comprises from about 90 to about 99.5 wt% of butyl acrylate and from about 0.5 to about 10 wt % dimethoxymethylsilyl methacrylate.

According to a certain embodiment of the invention the silicone acrylic hybrid polymer may be prepared by a) reacting silicone polymer with silicone resin to form a resultant product, b) reacting the resultant product of a) with an acrylic polymer containing reactive functionality, wherein the components are reacted in an organic solvent.

According to a certain embodiment of the invention the silicone acrylic hybrid polymer may be prepared by a) reacting a silicone resin with an acrylic polymer containing reactive functionality to form a resultant product, b) reacting the resultant product of a) with silicone polymer, wherein the components are reacted in an organic solvent.

According to a certain embodiment of the invention the silicone acrylic hybrid polymer may be prepared by a) reacting a silicone polymer with an acrylic polymer containing reactive functionality to form a resultant product, b) reacting the resultant product of a) with silicone resin, wherein the components are reacted in an organic solvent.

Further suitable acrylic polymers, silicone resins, and silicone polymers that can be used for chemically reacting together a silicone polymer, a silicone resin and an acrylic polymer to provide a silicone acrylic hybrid polymer in accordance with the previous paragraphs are detailed in WO 2010/124187.

## ACRYLIC POLYMER (NON-HYBRID)

As indicated above, the TTS according to the present invention comprises at least one polymer in the guanfacine-containing layer. According to the present invention, the at least one polymer is selected from the group consisting of acrylic polymers, silicone-based polymers, silicone-acrylic hybrid polymers, and mixtures thereof. Thus, in one embodiment of the present invention, the TTS according to the present invention comprises an acrylic polymer in the guanfacine-containing layer.

As used herein, the terms acrylic polymer and acrylate polymer are synonymously used. Preferably, the acrylic polymers are pressure-sensitive adhesives based on acrylates. Pressure-sensitive adhesives based on acrylates may also be referred to as acrylate-based pressure-sensitive adhesives, or acrylate pressure-sensitive adhesives.

Pressure-sensitive adhesives based on acrylates may be provided in the form of a solution with a solids content preferably between 30 % and 60 %.

Acrylate-based pressure-sensitive adhesives may or may not comprise functional groups such as hydroxy groups, carboxylic acid groups, neutralized carboxylic acid groups and mixtures thereof. Thus, the term "functional groups" in particular refers to hydroxy- and carboxylic acid groups, and deprotonated carboxylic acid groups.

Corresponding commercial products are available e.g. from Henkel under the tradename Duro Tak®. Such acrylate-based pressure-sensitive adhesives are based on monomers selected from one or more of acrylic acid, butylacrylate, 2-ethylhexylacrylate, glycidylmethacrylate, 2-hydroxyethylacrylate, methylacrylate, methylmethacrylate, butylmethacrylate, t-octylacrylamide and vinylacetate, and are provided in ethyl acetate, heptane, n-heptane, hexane,

methanol, ethanol, isopropanol, 2,4-pentanedione, toluene or xylene or mixtures thereof.

Specific acrylate-based pressure-sensitive adhesives are available as:

- Duro-Tak™ 387-2287 or Duro-Tak™ 87-2287 (a copolymer based on vinyl acetate, 2-ethylhexyl-acrylate, 2-hydroxyethyl-acrylate and glycidyl-methacrylate provided as a solution in ethyl acetate without cross-linking agent),
- Duro-Tak™ 387-2516 or Duro-Tak™ 87-2516 (a copolymer based on vinyl acetate, 2-ethylhexyl-acrylate, 2-hydroxyethyl-acrylate and glycidyl-methacrylate provided as a solution in ethyl acetate, ethanol, n-heptane, and methanol with a titanium cross-linking agent),
- Duro-Tak™ 387-2051 or Duro-Tak™ 87-2051 (a copolymer based on acrylic acid, butylacrylate, 2-ethylhexylacrylate and vinyl acetate, provided as a solution in ethyl acetate or heptane without cross-linking agent),
- Duro-Tak™ 387-2353 or Duro-Tak™ 87-2353 (a copolymer based on acrylic acid, 2-ethylhexylacrylate, glycidyl-methacrylate and methylacrylate, provided as a solution in ethyl acetate and hexane),
- Duro-Tak™ 87-4098 (a copolymer based on 2-ethylhexyl-acrylate and vinyl acetate, provided as a solution in ethyl acetate).
- Duro-Tak™ 387-2287 or Duro-Tak™ 87-2054 (a copolymer based on acrylic acid, butylacrylate, 2-ethylhexylacrylate and vinyl acetate, provided as a solution in ethyl acetate or heptane with cross-linking agent).

Preferred acrylate-based pressure sensitive adhesives according to the present invention are Duro-Tak™ 387-2516 or Duro-Tak™ 87-2516 (a copolymer based on vinyl acetate, 2-ethylhexyl-acrylate, 2-hydroxyethyl-acrylate and glycidyl-methacrylate provided as a solution in ethyl acetate, ethanol, n-heptane and methanol with a titanium cross-linking agent) and Duro-Tak™ 87-4098 (a copolymer based on 2-ethylhexyl-acrylate and vinyl acetate, provided as a solution in ethyl acetate).

Additional polymers may also be added to enhance cohesion and/or adhesion.

## SILICONE-BASED POLYMER (NON HYBRID)

As indicated above, the TTS according to the present invention comprises at least one polymer in the guanfacine-containing layer. According to the present invention, the at least one polymer is selected from the group consisting of acrylic polymers, silicone-based polymers, silicone-acrylic hybrid polymers, and mixtures thereof. Thus, in one embodiment of the present invention, the TTS according to the present invention comprises at least one silicone-based polymer in the guanfacine-containing layer, preferably in the guanfacine-containing matrix layer.

As used herein, the silicone-based polymer is a non-hybrid polymer, i.e. a polymer, which does not include a hybrid species. Silicone-based polymers are based on polysiloxanes. They may therefore also be referred to as polymers based on polysiloxanes. Preferably, the silicone-based polymers are silicone-based pressure sensitive adhesives, i.e. pressure sensitive adhesives based on polysiloxanes.

As the silicone-based polymer is preferably a non-curing polymer, it is typically supplied and used in solvents, such as n-heptane and ethyl acetate. The solids content is usually between 30 % and 80 %.

Suitable silicone-based polymers are commercially available under the brand names BIO-PSAs (pressure sensitive adhesives based on polysiloxanes).

Pressure-sensitive adhesives based on polysiloxanes provide for suitable tack and for quick bonding to various skin types, including wet skin, suitable adhesive and cohesive qualities, long lasting adhesion to the skin, a high degree of flexibility, a permeability to moisture, and compatibility to many actives and film-substrates. It is possible to provide the pressure-sensitive adhesives based on polysiloxanes with sufficient amine resistance and therefore enhanced stability in the presence of amines. Such pressure-sensitive adhesives are based on a resin-in-polymer concept wherein, by condensation reaction of silanol end blocked polydimethylsiloxane with a silica resin (also referred to as silicate resin), a pressure-sensitive adhesive based on polysiloxane is prepared wherein for amine stability the residual silanol functionality is additionally capped with trimethylsiloxy groups. The silanol end blocked polydimethylsiloxane content contributes to the viscous component of the visco-elastic behavior and impacts the wetting and the spreadability properties of the adhesive. The resin acts as a tackifying and reinforcing agent and participates in the elastic component. The correct balance between silanol end blocked polydimethylsiloxane and resin provides for the correct adhesive properties.

In view of the above, silicone-based polymers, and in particular silicone-based pressure sensitive adhesives, are generally obtainable by polycondensation of silanol endblocked polydimethylsiloxane with a silicate resin. Amine-compatible silicone-based polymers, and in particular amine-compatible silicone-based pressure sensitive adhesives, can be obtained by reacting the silicone-based polymer, in particular the silicone-based pressure sensitive adhesive, with trimethylsilyl (e.g. hexamethyldisilazane) in order to reduce the silanol content of the polymer. As a result, the residual silanol functionality is at least partly, preferably mostly or fully capped with trimethylsiloxy groups.

As indicated above, the tackiness of the silicone-based polymer may be modified by the resin-to-polymer ratio, i.e. the ratio of the silanol endblocked polydimethylsiloxane to the silicate resin, which is preferably in the range of from

70:30 to 50:50, preferably from 65:35 to 55:45. The tackiness will be increased with increasing amounts of the polydimethylsiloxane relative to the resin. High tack silicone-based polymers preferably have a resin-to-polymer ratio of 55:45, medium tack silicone-based polymers preferably have a resin-to-polymer ratio of 60:40, and low tack silicone-based polymers preferably have a resin-to-polymer ratio of 65:35. High tack silicone-based polymers preferably have a complex viscosity at 0.01 rad/s and 30 °C of about 5 x $10^6$ Poise, medium tack silicone-based polymers preferably have a complex viscosity at 0.01 rad/s and 30 °C of about 5 x $10^7$ Poise, and low tack silicone-based polymers preferably have a complex viscosity at 0.01 rad/s and 30 °C of about 5 x $10^8$ Poise. High tack amine-compatible silicone-based polymers preferably have a complex viscosity at 0.01 rad/s and 30 °C of about 5 x $10^6$ Poise, medium tack amine-compatible silicone-based polymers preferably have a complex viscosity at 0.01 rad/s and 30 °C of about 5 x $10^8$ Poise, and low tack amine-compatible silicone-based polymers preferably have a complex viscosity at 0.01 rad/s and 30 °C of about 5 x $10^9$ Poise.

Examples of silicone-based PSA compositions which are commercially available include the standard BIO-PSA series (7-4400,7-4500 and 7-4600 series) and the amine compatible (endcapped) BIO-PSA series (7-4100, 7-4200 and 7-4300 series) manufactured and typically supplied in n-heptane or ethyl acetate by DuPont™. For example, BIO-PSA 7-4201 is characterized by a solution viscosity at 25 °C and about 60 % solids content in heptane of 450 mPa s and a complex viscosity at 0.01 rad/s at 30 °C of $1\times10^8$ Poise. BIO-PSA 7-4301 has a solution viscosity at 25 °C and about 60 % solids content in heptane of 500 mPa s and a complex viscosity at 0.01 rad/s at 30 °C of $5\times10^6$ Poise. BIO-PSA 7-4202 is characterized by a solution viscosity at 25 °C and about 60 % solids content in ethyl acetate of 800 mPa s and a complex viscosity at 0.01 rad/s at 30 °C of $1\times10^8$ Poise. BIO-PSA 7-4302 has a solution viscosity at 25 °C and about 60 % solids content in ethyl acetate of 1200 mPa s and a complex viscosity at 0.01 rad/s at 30 °C of $5\times 10^6$ Poise. It is noted that 1 cP converts to 1 mPa.s.

The pressure-sensitive adhesives based on polysiloxanes are supplied and used in solvents like n-heptane, ethyl acetate or other volatile silicone fluids. The solids content of pressure-sensitive adhesives based on polysiloxanes in solvents is usually between 60 and 85 %, preferably between 70 and 80 % or between 60 and 75 %. The skilled person is aware that the solids content may be modified by adding a suitable amount of solvent.

Pressure-sensitive adhesives based on polysiloxanes, which are, e.g., available from DuPont™, may be obtained according to the following scheme:

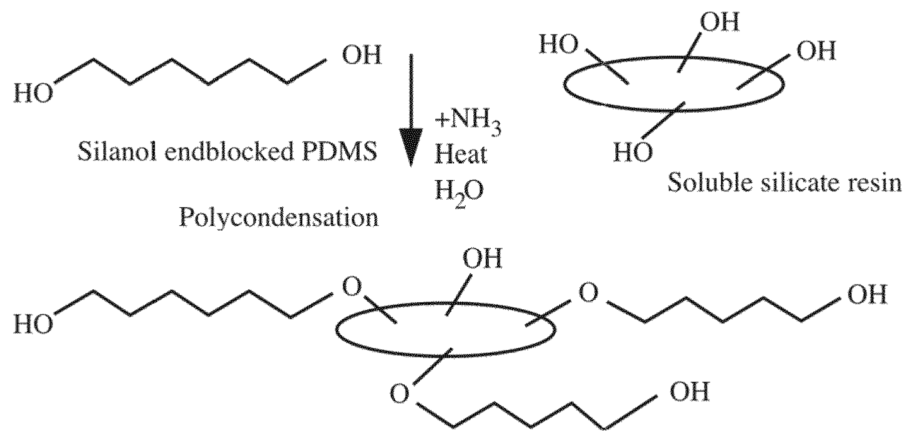


Such pressure-sensitive adhesives based on polysiloxanes are available from DuPont™, e.g., under the tradenames BIO-PSA 7-4401, BIO-PSA-7-4501, or BIO-PSA 7-4601, which are provided in the solvent n-heptane (indicated by the code "01"), or under the tradenames BIO-PSA 7-4402, BIO-PSA 7-4502, and BIO 7-4602, which are provided in the solvent ethyl acetate (indicated by the code "02"). Typical solids contents in the solvent are in the range of from 60 to 75 %. The code "44" indicates a resin-to-polymer ratio of 65:35 resulting in a low tackiness, the code "45" indicates a resin-to-polymer ratio of 60:40 resulting in medium tackiness, the code "46" indicates a resin-to-polymer ratio of 55:45 resulting in high tackiness.

Amine-compatible pressure-sensitive adhesives based on polysiloxanes, which are, e.g., available from DuPont™ may be obtained according to the following scheme:

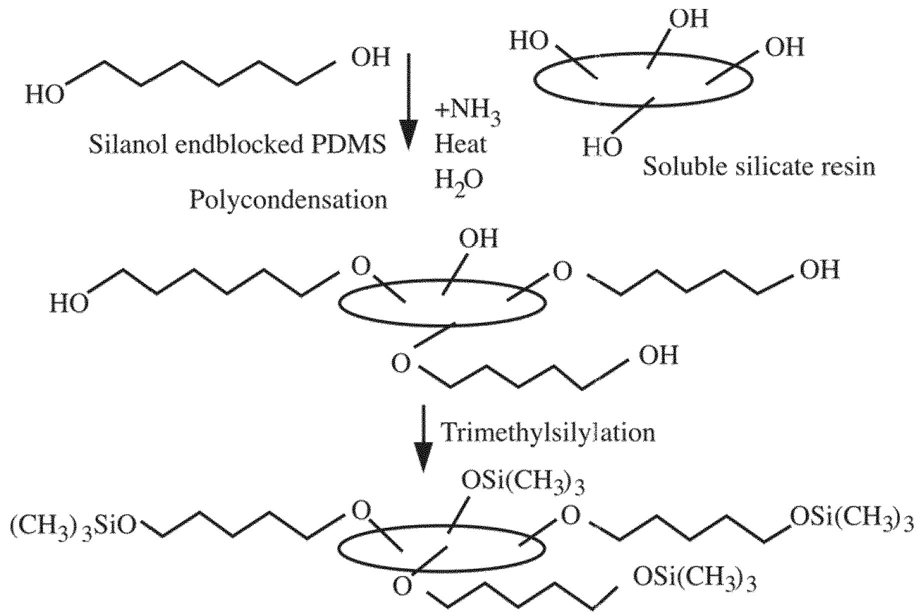


Such amine-compatible pressure-sensitive adhesives based on polysiloxanes are available from DuPont™, e.g., under the tradenames BIO-PSA 7-4101, BIO-PSA-7-4201, or BIO-PSA 7-4301, which are provided in the solvent n-heptane (indicated by the code "01"), or under the tradenames BIO-PSA 7-4102, BIO-PSA 7-4202, and BIO 7-4302, which are provided in the solvent ethyl acetate (indicated by the code "02"). Typical solids contents in the solvent are in the range of from 60 to 75 %. The code "41" indicates a resin-to-polymer ratio of 65:35 resulting in a low tackiness, the code "42" indicates a resin-to-polymer ratio of 60:40 resulting in medium tackiness, the code "43" indicates a resin-to-polymer ratio of 55:45 resulting in high tackiness.

The preferred pressure-sensitive adhesives based on polysiloxanes in accordance with the invention are characterized by a solution viscosity at 25 °C and 60 % solids content in n-heptane of more than about 150 mPa s, or from about 200 mPa s to about 700 mPa s, preferably as measured using a Brookfield RVT viscometer equipped with a spindle number 5 at 50 rpm. These may also be characterized by a complex viscosity at 0.01 rad/s at 30 °C of less than about 1 x $10^9$ Poise or from about 1 x $10^5$ to about 9 x $10^8$ Poise. It is noted that 1 cP converts to 1 mPa.s.

## POLYMER MIXTURES

The TTS according to the invention, and in particular the guanfacine-containing layer, comprises at least one polymer selected from the group consisting of acrylic polymers, silicone-based polymers, silicone-acrylic hybrid polymers, and mixtures thereof.
In one embodiment of the present invention, the guanfacine-containing layer comprises at least one polymer, wherein the at least one polymer is

- a mixture of an acrylic polymer and at least one silicone-based polymer; or
- a mixture of two silicone-acrylic hybrid polymers; or
- a mixture of two silicone-based polymers; or
- an acrylic polymer; or
- an acrylic polymer comprising a -OH group.

In this connection it is to be understood that the acrylic polymers, silicone-based polymers, and silicone-acrylic hybrid polymers are as defined above.

In one preferred embodiment of the present invention, the at least one polymer is present in an amount of from 20% to 99%, preferably from 30% to 97%, most preferably from 35% to 94% by weight based on the total weight of the guanfacine-containing layer.

In a more preferred embodiment of the present invention, the guanfacine-containing layer comprises a mixture of an acrylic polymer and at least one silicone-based polymer, wherein the acrylic polymer is present in an amount of from 20 to 55 % and the at least one silicone-based polymer is present in an amount of from 20 to 55% by weight, based on the total weight of the guanfacine-containing layer.

In another more preferred embodiment of the present invention, the guanfacine-containing layer comprises a

mixture of two silicone-acrylic hybrid polymers, wherein the first silicone-acrylic hybrid polymer is present in an amount of from 60 to 90 % by weight, and the second silicone-acrylic hybrid polymer is present in an amount of from 1 to 20 % by weight, in each case based on the total weight of guanfacine-containing layer and wherein preferably the first silicone-acrylic hybrid polymer and the second silicone-acrylic hybrid polymer comprise a silicone phase and an acrylate phase in a weight ratio of from 60:40 to 40:60.

In a further more preferred embodiment of the present invention, the guanfacine-containing layer comprises a mixture of two silicone-based polymers, wherein the first silicone-based polymer is present in an amount of from 20 to 55 % by weight, and the second silicone-based polymer is present in an amount of from 20 to 55 % by weight, based on the total weight of the guanfacine-containing layer.

In an even more preferred embodiment of the present invention, the guanfacine-containing layer comprises a mixture of an acrylic polymer, wherein the acrylic polymer is a copolymer based on vinyl acetate, 2-ethylhexyl-acrylate, 2-hydroxyethyl-acrylate and glycidyl-methacrylate, and at least one silicone-based polymer, wherein the at least one silicone-based polymer refers to two silicone-based polymers, wherein the first silicone-based polymer is BIO-PSA 7-4202 and the second silicone-based polymer is BIO-PSA 7-4302. In connection with the above more preferred embodiment, it is to be understood that the acrylic polymer is present in an amount of from 20 to 55 % and the two silicone-based polymers are present in an overall amount of from 20 to 55% by weight, based on the total weight of the guanfacine-containing layer. Furthermore, in connection with the above preferred embodiment it is to be understood that the two silicone-based polymers are preferably present in the guanfacine-containing layer in a ratio of 1:1.

In an even more preferred embodiment of the present invention, the guanfacine-containing layer comprises a mixture of an acrylic polymer, wherein the acrylic polymer is a copolymer based on 2-ethylhexyl-acrylate and vinyl acetate, and at least one silicone-based polymer, wherein the at least one silicone-based polymer refers to two silicone-based polymers, wherein the first silicone-based polymer is BIO-PSA 7-4202 and the second silicone-based polymer is BIO-PSA 7-4302. In connection with the above more preferred embodiment, it is to be understood that the acrylic polymer is present in an amount of from 20 to 55 % and the two silicone-based polymers are present in an overall amount of from 20 to 55% by weight, based on the total weight of the guanfacine-containing layer. Furthermore, in connection with the above preferred embodiment it is to be understood that the two silicone-based polymers are preferably present in the guanfacine-containing layer in a ratio of 1:1.

In connection with the above embodiments, it is to be understood that the amount of the at least one silicone-based polymer refers to the overall amount of silicone-based polymers present in the guanfacine-containing layer based on the total weight of the guanfacine-containing layer. In particular, it is to be understood that if it is referred to two silicone-based polymers, the two silicone-based polymers are present in an overall amount as defined above.

In another even more preferred embodiment of the present invention, the guanfacine-containing layer comprises a mixture of two silicone-acrylic hybrid polymers, wherein the first silicone-acrylic hybrid polymer is preferably a 7-6102 silicone acrylic hybrid PSA as defined above and is present in an amount of from 60 to 90 % by weight, and the second silicone-acrylic hybrid polymer is preferably a 7-6302 silicone acrylic hybrid PSA as defined above and is present in an amount of from 1 to 20 % by weight, in each case based on the total weight of guanfacine-containing layer and wherein preferably the first silicone-acrylic hybrid polymer and the second silicone-acrylic hybrid polymer comprise a silicone phase and an acrylate phase in a weight ratio of from 60:40 to 40:60.

In a further even more preferred embodiment of the present invention, the guanfacine-containing layer comprises a mixture of two silicone-based polymers, wherein the first silicone-based polymer is BIO-PSA 7-4202 and is present in an amount of from 20 to 55 % by weight, and the second silicone-based polymer is BIO-PSA 7-4302 and is present in an amount of from 20 to 55 % by weight, based on the total weight of the guanfacine-containing layer. In connection with the above preferred embodiment, it is to be understood that the two silicone-based polymers are preferably present in the guanfacine-containing layer in a ratio of 1:1.

## FURTHER ADDITIVES

The TTS according to the invention, and in particular the guanfacine-containing layer, may further comprise at least one additive or excipient. Particularly preferred additives according to the invention include dispersing agents, permeation enhancers, and solubilizers. Details in this regard are provided above. However, the TTS according to the invention, and in particular the guanfacine-containing layer, may also include further additives or excipients.

In general, additives or excipients are preferably selected from the group consisting of dispersing agents, solubilizers, permeation enhancers, film-forming agents, softeners/plasticizers, tackifiers, substances for skincare, pH regulators, preservatives, stabilizing agents, and fillers. Such additives may be present in the guanfacine-containing layer in an amount of from 0.001 % to 15 % by weight, e.g. from 0.5 to 10 % by weight or from 1 to 10 % by weight or from 0.01 to 6 % by weight, based on the total weight of the guanfacine-containing layer, and wherein the weight % amounts refer to a single additive.

It should be noted that in pharmaceutical formulations, the formulation components are categorized according to

their physicochemical and physiological properties, and in accordance with their function. This means in particular that a substance or a compound falling into one category is not excluded from falling into another category of formulation component. E.g. a certain polymer can be a film-forming agent, but also a tackifier. Some substances may e.g. be a typical softener but at the same time act as a permeation enhancer. The skilled person is able to determine based on his general knowledge in which category or categories of formulation components a certain substance or compound belongs to. In the following, details on the excipients and additives are provided which are, however, not to be understood as being exclusive. Other substances not explicitly listed in the present description may be as well used in accordance with the present invention, and substances and/or compounds explicitly listed for one category of formulation component are not excluded from being used as another formulation component in the sense of the present invention.

In one embodiment, the guanfacine-containing layer comprises a dispersing agent as defined above, preferably a dispersing agent selected from the group consisting of esters of fatty acids with polyols, fatty alcohols, polyethylene glycols having a number average molecular weight of from 300 to 400, polyethylene glycol alkyl ethers. As explained above, the dispersing agent is preferably polyethylene glycol $C_8$-$C_{20}$-alkyl ether having from 2 to 10 EO units, in particular polyoxyethylene (4) lauryl ether. Alternatively or additionally, silicone polyethers may be used as dispersing agents. The dispersing agent is helpful in order to homogeneously disperse the guanfacine within the guanfacine-containing layer, in particular the guanfacine-containing matrix layer, thereby improving the release properties of the TTS.

In one embodiment, the guanfacine-containing layer comprises a solubilizer. The solubilizer preferably improves the dispersibility of the guanfacine in the guanfacine-containing layer and stabilizes the guanfacine-containing layer. Furthermore, the solubilizer may positively influence cohesion. Preferred solubilizers include, e.g., glycerol-, polyglycerol-, propylene glycol- and polyoxyethylene-esters of medium chain and/or long chain fatty acids, such as glyceryl monolinoleate, medium chain glycerides and medium chain triglycerides, non-ionic solubilizers made by reacting castor oil with ethylene oxide, and any mixtures thereof which may further contain fatty acids or fatty alcohols; cellulose and methylcellulose and derivatives thereof such as hydroxypropylcellulose and hypromellose acetate succinate; various cyclodextrins and derivatives thereof; non-ionic tri-block copolymers having a central hydrophobic chain of polyoxypropylene flanked by two hydrophilic chains of polyoxyethylene known as poloxamers; water-soluble derivatives of vitamin E; pharmaceutical graded or agglomerated spherical isomalt; a polyethylene glycol, polyvinyl acetate and polyvinylcaprolactame-based graft copolymer, also abbreviated as PVAc-PVCap- PEG and known as Soluplus®; vinylpyrrolidone-vinyl acetate copolymers such as Kollidon® VA64; purified grades of naturally derived castor oil, of polyethylene glycol 400, of polyoxyethylene sorbitan monooleate (such as polysorbate 80) or of propylene glycols; diethylene glycol monoethyl ether; glucono-delta-lactone; maize and potato starch; as well as any of the below mentioned soluble polyvinylpyrrolidones, but also insoluble / cross-linked polyvinylpyrrolidones such as crospovidones.

However, also the permeation enhancers mentioned below can act as solubilizers. Furthermore, also the film-forming agents described below may act at the same time as solubilizers and vice versa.

In one embodiment, the guanfacine-containing layer comprises a permeation enhancer. Preferences in this regard are provided above. Permeation enhancers are substances, which influence the barrier properties of the stratum corneum in the sense of increasing the active agent permeability. Some examples of permeation enhancers are polyhydric alcohols such as dipropylene glycol, propylene glycol, and polyethylene glycol; oils such as olive oil, squalene, and lanolin; fatty ethers such as cetyl ether and oleyl ether; fatty acid esters such as isopropyl myristate; urea and urea derivatives such as allantoin; polar solvents such as dimethyldecylphosphoxide, methylcetylsulfoxide, dimethylaurylamine, dodecyl pyrrolidone, isosorbitol, dimethylacetonide, dimethylsulfoxide, decylmethylsulfoxide, and dimethylformamide; salicylic acid; amino acids; benzyl nicotinate; and higher molecular weight aliphatic surfactants such as lauryl sulfate salts. Other agents include oleic and linoleic acids, ascorbic acid, panthenol, butylated hydroxytoluene, tocopherol, tocopheryl acetate, tocopheryl linoleate, propyl oleate, and isopropyl palmitate. If the guanfacine-containing layer comprises a permeation enhancer, the permeation enhancer is preferably selected from the group consisting of diethylene glycol monoethyl ether (transcutol), oleic acid, levulinic acid, caprylic/capric triglycerides, diisopropyl adipate, isopropyl myristate, isopropyl palmitate, lauryl lactate, triacetin, dimethylpropylene urea, oleyl alcohol, oleoyl macrogol-6 glycerides (labrafil MS 1944), and lauroglycol, and is preferably oleyl alcohol, lauroglycol or oleoyl macrogol-6 glycerides (labrafil MS 1944).

In one embodiment, the guanfacine-containing layer further comprises a film-forming agent. It is to be understood that the above mentioned solubilizers, such as Soluplus®, may also act as film-forming agents and control cohesion. Suitable examples of further film-forming agents include polyvinylpyrrolidone, vinyl acetate/vinylpyrrolidone copolymers and cellulose derivatives, preferably polyvinylpyrrolidone, more preferably soluble polyvinylpyrrolidone.

If the guanfacine-containing layer is required to have self-adhesive properties and one or more polymers is/are selected, which does/do not provide sufficient self-adhesive properties, a tackifier is added. Preferred tackifiers include Miglyol, which is a liquid wax ester based on long-chain, unsaturated, even-numbered fatty acids and long-chain, unsaturated, even-numbered fatty alcohols of vegetable origin, and polyethylene glycols. In particular, the tackifier may be selected from polyvinylpyrrolidone (which, due to its ability to absorb water, is able to maintain the adhesive properties of the matrix layer and thus can be regarded as a tackifier in a broad sense), triglycerides, polyethylene glycols,

dipropylene glycol, resins, resin esters, terpenes and derivatives thereof, ethylene vinyl acetate adhesives, dimethylpolysiloxanes and polybutenes, preferably polyvinylpyrrolidone and more preferably soluble polyvinylpyrrolidone. Preferably, the tackifier may be selected from polyvinylpyrrolidone, triglycerides, dipropylene glycol, resins, resin esters, terpenes and derivatives thereof, ethylene vinyl acetate adhesives, dimethylpolysiloxanes and polybutenes, preferably polyvinylpyrrolidone and more preferably soluble polyvinylpyrrolidone.

The term "soluble polyvinylpyrrolidone" refers to polyvinylpyrrolidone, also known as povidone, which is soluble with more than 10 % in at least ethanol, preferably also in water, diethylene glycol, methanol, n-propanol, 2 propanol, n-butanol, chloroform, methylene chloride, 2-pyrrolidone, macrogol 400, 1,2 propylene glycol, 1,4 butanediol, glycerol, triethanolamine, propionic acid and acetic acid. Examples of polyvinylpyrrolidones which are commercially available include Kollidon® 12 PF, Kollidon® 17 PF, Kollidon® 25, Kollidon® 30 and Kollidon® 90 F supplied by BASF, or povidone K90F. The different grades of Kollidon® are defined in terms of the K-Value reflecting the average molecular weight of the polyvinylpyrrolidone grades. Kollidon® 12 PF is characterized by a K-Value range of 10.2 to 13.8, corresponding to a nominal K-Value of 12. Kollidon® 17 PF is characterized by a K-Value range of 15.3 to 18.4, corresponding to a nominal K-Value of 17. Kollidon® 25 is characterized by a K-Value range of 22.5 to 27.0, corresponding to a nominal K-Value of 25, Kollidon® 30 is characterized by a K-Value range of 27.0 to 32.4, corresponding to a nominal K-Value of 30. Kollidon® 90 F is characterized by a K-Value range of 81.0 to 97.2, corresponding to a nominal K-Value of 90. Preferred Kollidon® grades are Kollidon® 12 PF, Kollidon® 30 and Kollidon® 90 F. Within the meaning of this invention, the term "K-Value" refers to a value calculated from the relative viscosity of polyvinylpyrrolidone in water according to the European Pharmacopoeia (Ph.Eur.) and USP monographs for "Povidone".

In one embodiment, the guanfacine-containing layer further comprises a softener/ plasticizer. Exemplary softeners/plasticizers include linear or branched, saturated or unsaturated alcohols having 6 to 20 carbon atoms, triglycerides and polyethylene glycols.

In one embodiment, the guanfacine-containing layer further comprises a stabilizing agent. Stabilizing agents include tocopherol and ester derivatives thereof and ascorbic acid and ester derivatives thereof. Further stabilizing agents include sodium metabisulfite, ascorbyl esters of fatty acids such as ascorbyl palmitate, ascorbic acid, butylated hydroxytoluene, tocopherol, tocopheryl acetate and tocopheryl linoleate.

In one embodiment, the guanfacine-containing layer further comprises a pH regulator. Suitable pH regulators include mild acids and bases including amine derivatives, inorganic alkali derivatives, and polymers with basic or acidic functionality.

In one embodiment, the guanfacine-containing layer further comprises a preservative. Suitable preservatives include parabens, formaldehyde releasers, isothiazolinones, and phenoxyethanol.

In one embodiment, the guanfacine-containing layer further comprises a substance for skincare. Such substances may be used to avoid or reduce skin irritation as detectable by the dermal response score. Suitable substances for skincare include sterol compounds such as cholesterol, dexpanthenol, alpha-bisabolol, and antihistamines.

In one embodiment, the guanfacine-containing layer further comprises a filler. Fillers such as silica gels, titanium dioxide and zinc oxide may be used in conjunction with the polymer in order to influence certain physical parameters, such as cohesion and bond strength, in the desired way.

## RELEASE CHARACTERISTICS

The TTS in accordance with the invention are designed for transdermally administering guanfacine to the systemic circulation for a predefined extended period of time, preferably for at least 24 hours, more preferably at least 72 hours, in particular for about 84 hours.

In one embodiment, the TTS according to the invention provides by transdermal delivery at steady state a mean plasma concentration of guanfacine of from 1 to 20 ng/ml, preferably from 1 to 15 ng/ml, more preferably 1 to 10 ng/ml.

Preferably, the TTS provides therapeutically effective plasma concentrations of guanfacine within less than 8 hours, preferably less than 6 hours, more preferably less than 4 hours after application of the TTS to the skin. Furthermore, the therapeutically effective plasma concentrations are preferably maintained over the whole administration period of at least 24 hours, preferably at least 72 hours, more preferably about 84 hours.

In one embodiment, the TTS according to the invention provides an $AUC_{0-24h}$ of 10 to 600 ng*h/ml, preferably of 20 to 400 ng*h/ml. In another embodiment, the TTS according to the invention provides an $AUC_{0-72h}$ of 30 to 1800 ng*h/ml, preferably of 60 to 1200 ng*h/ml. In another embodiment, the TTS according to the invention provides an $AUC_{0-84h}$ of 35 to 2100 ng*h/ml, preferably of 70 to 1400 ng*h/ml. It is to be understood that the AUC values preferably refer to the AUC values obtained at steady state.

In one embodiment, the TTS according to the invention provides a $C_{max}$ to $C_{84}$ ratio of less than 3.5. In another embodiment, the TTS according to the invention provides a $C_{max}$ to $C_{72}$ ratio of less than 3.0. In another embodiment, the TTS according to the invention provides a $C_{max}$ to $C_{24}$ ratio of less than 2.0. These ratios indicate a flat blood plasma curve, which is advantageous in terms of a continuous treatment of the patient.

In one embodiment, the TTS according to the invention provides a skin permeation rate of guanfacine as measured in a Franz diffusion cell with dermatomed human skin of

0.01 μg/(cm$^2$*h) to 8 μg/(cm$^2$*h) in the first 24 hours,
0.05 μg/(cm$^2$*h) to 10 μg/(cm$^2$*h) from hour 24 to hour 88.

In another embodiment, the TTS according to the invention provides a skin permeation rate of guanfacine as measured in a Franz diffusion cell with dermatomed human skin of

0.01 μg/(cm$^2$*h) to 8 μg/(cm$^2$*h) in the first 24 hours,
0.05 μg/(cm$^2$*h) to 8 μg/(cm$^2$*h) from hour 24 to hour 88.

In one embodiment, the TTS according to the invention provides a skin permeation rate of guanfacine as measured in a Franz diffusion cell with dermatomed Göttinger minipig skin of 0.01 μg/(cm$^2$*h) to 8 μg/(cm$^2$*h) in the first 24 hours, 0.05 μg/(cm$^2$*h) to 10 μg/(cm$^2$*h) from hour 24 to hour 88.

In another embodiment, the TTS according to the invention provides a cumulative permeated amount of guanfacine as measured in a Franz diffusion cell with dermatomed Göttinger minipig skin of 0.01 mg/cm$^2$ to 0.7 mg/cm$^2$, preferably 0.05 mg/cm$^2$ to 0.6 mg/cm$^2$, more preferably 0.10 mg/cm$^2$ to 0.5 mg/cm$^2$ over a time period of 88 hours.

In view of the above, the present invention relates in one aspect also to a transdermal therapeutic system for the transdermal administration of guanfacine comprising a guanfacine-containing layer structure,
wherein the transdermal therapeutic system provides by transdermal delivery one or more pharmacokinetic parameter(s) selected from the group consisting of

an $AUC_{0-24}$ from 10 to 600 (ng / mL) h,
an $AUC_{0-72}$ from 30 to 1800 (ng / mL) h,
an $AUC_{0-84}$ from 35 to 2100 (ng / mL) h,
a $C_{max}$ to $C_{24}$ ratio of less than 2.0,
a $C_{max}$ to $C_{72}$ ratio of less than 3.0, and
a $C_{max}$ to $C_{84}$ ratio of less than 3.5.

In a preferred embodiment, the present invention relates to a transdermal therapeutic system for the transdermal administration of guanfacine comprising a guanfacine-containing layer structure, wherein the transdermal therapeutic system provides by transdermal delivery one or more pharmacokinetic parameter(s) selected from the group consisting of

an $AUC_{0-24}$ from 20 to 400 (ng / mL) h,
an $AUC_{0-72}$ from 60 to 1200 (ng / mL) h,
an $AUC_{0-84}$ from 70 to 1400 (ng / mL) h,
a $C_{max}$ to $C_{24}$ ratio of less than 1.5,
a $C_{max}$ to $C_{72}$ ratio of less than 2.5, and
a $C_{max}$ to $C_{84}$ ratio of less than 3.0.

In one particularly preferred embodiment, the present invention relates to a transdermal therapeutic system for the transdermal administration of guanfacine comprising a guanfacine-containing layer structure, wherein the transdermal therapeutic system provides by transdermal delivery an $AUC_{0-84}$ of from 70 to 1400 (ng / mL) h.

**MEDICAL USE**

In one embodiment of the present invention, the TTS according to the invention are suitable for use in a method of treating a human patient, preferably a patient at the age of from 6 to 17. In particular, the TTS according to the invention are suitable for use in a method of treating hypertension or attention deficit hyperactivity disorder (ADHD) and/or as adjunctive therapy to stimulant medications in a human patient, preferably in a human patient at the age of from 6 to 17.

In a preferred embodiment in connection with the above medical use, the TTS is applied to the skin of the patient for at least 24 hours, preferably at least 72 hours, more preferably about 84 hours.

In one embodiment, the present invention relates to a composition for use in a method of treating a human patient, preferably a human patient at the age of from 6 to 17, by applying a transdermal therapeutic system as defined above to the skin of the patient. In particular, the present invention relates to a method of treating hypertension or attention deficit hyperactivity disorder (ADHD) in a human patient, preferably a human patient at the age of from 6 to 17, by applying a transdermal therapeutic system according to the invention to the skin of the patient.

In a preferred embodiment of the above compositions for use in methods of treatment, the transdermal therapeutic system is applied to the skin of the patient for at least 24 hours, preferably at least 72 hours, more preferably about 84 hours.

In view of the above, the present invention relates in one aspect to a transdermal therapeutic system comprising guanfacine and a mono-carboxylic acid for use in a method of treating a human patient, preferably a human patient at the age of from 6 to 17, by transdermal administration of guanfacine, wherein the transdermal therapeutic system is applied to the skin of a patient for at least 24 hours, preferably at least 72 hours, more preferably about 84 hours. In a preferred embodiment, the transdermal therapeutic system is for use in a method of treating hypertension or attention deficit hyperactivity disorder (ADHD) and/or as adjunctive therapy to stimulant medications in a human patient. In a more preferred embodiment, the transdermal therapeutic system is a transdermal therapeutic system according to the invention, in particular a transdermal therapeutic system providing one or more of the pharmacokinetic parameter(s) selected from the group consisting of

an $AUC_{0-24}$ from 10 to 600 (ng / mL) h,
an $AUC_{0-72}$ from 30 to 1800 (ng / mL) h,
an $AUC_{0-84}$ from 35 to 2100 (ng / mL) h,
a $C_{max}$ to $C_{24}$ ratio of less than 2.0,
a $C_{max}$ to $C_{72}$ ratio of less than 3.0, and
a $C_{max}$ to $C_{84}$ ratio of less than 3.5;

and preferably selected from the group consisting of

an $AUC_{0-24}$ from 20 to 400 (ng / mL) h,
an $AUC_{0-72}$ from 60 to 1200 (ng / mL) h,
an $AUC_{0-84}$ from 70 to 1400 (ng / mL) h,
a $C_{max}$ to $C_{24}$ ratio of less than 1.5,
a $C_{max}$ to $C_{72}$ ratio of less than 2.5, and
a $C_{max}$ to $C_{84}$ ratio of less than 3.0.

In connection with the above compositions for use in methods of treatment, the TTS according to the invention is preferably applied to at least one body surface on the subject selected from the upper outer art, upper chest, upper back or the side of the chest for the defined dosing intervals.

The preferred application time of a TTS according to the invention is at least 24 hours (1 day), preferably at least 72 hours (3 days), more preferably about 84 hours (3.5 days). After this time, the TTS may be removed, and optionally a new TTS may be applied, so as to allow an around-the-clock treatment.

**PROCESS OF MANUFACTURE**

The invention further relates to a process for manufacturing an active pharmaceutical-containing layer, preferably an active pharmaceutical-containing matrix layer for use in a transdermal therapeutic system. The pharmaceutically active agent is guanfacine.

In accordance with the invention, the process for manufacturing an active pharmaceutical-containing layer for use in a transdermal therapeutic system according to the invention comprises

1) combining at least the components

   (i) a pharmaceutically active agent; and
   (ii) at least one mono-carboxylic acid;

to obtain a pre-mixture;
2) combining

   (i) the pre-mixture of step 1); and
   (ii) at least one polymer;

to obtain a coating composition
3) coating the coating composition onto a backing layer or a release liner to obtain a coated coating composition; and
4) drying the coated coating composition to form the active pharmaceutical-containing layer; wherein the pharmaceutically active agent is guanfacine.

In one embodiment of the present invention, the pre-mixture according to step 1) of the process is obtained by combining (i) and (ii) in a dry-grinding method and/or in a slurry method.

In one embodiment of the present invention, the slurry method of the above process comprises

- weighing in pharmaceutically active agent (i) and the mono-carboxylic acid (ii) in equimolar amounts;
- adding a solvent, which is selected from the group consisting of dichloromethane, methanol and ethyl acetate;
- stirring the obtained mixture for at least 24 hours, preferably from 24 to 36 hours with a magnetic stirring bar.

Thus, the slurry method of step 1) of the process of manufacture comprises combining equimolar amounts of the components (i) and (ii) in at least one solvent selected from the group consisting of dichloromethane, methanol and ethyl acetate.

In order to obtain the pre-mixture of the pharmaceutically active agent and the mono-carboxylic acid in the slurry method from step 1) the solvent is removed from the above-obtained mixture under reduced pressure and the residue is dried for at least 24 hours under vacuum.

In step 2) of the above process of manufacture, the pre-mixture obtained in step 1) is preferably dispersed in the at least one polymer to obtain a homogenous coating composition. In this regard it is to be understood that the pre-mixture obtained in step 1) can be obtained by the dry-grinding method and/or by the slurry method.

It is to be understood that in step 2), also further ingredients, preferably at least one additive as defined above, may be added.

Preferably, a solvent is added in step 2) of the process, and/or a solvent is present because the one or more polymer(s) are provided in the form of a solution. The solvent is preferably selected from alcoholic solvents, in particular methanol, ethanol, isopropanol and mixtures thereof, and from non-alcoholic solvents, in particular ethyl acetate, hexane, heptane, petroleum ether, toluene, and mixtures thereof. Preferably, the solvent is selected from non-alcoholic solvents and is most preferably ethyl acetate or n-heptane. In a particularly preferred embodiment, the solvent is ethyl acetate.

In a preferred embodiment, the at least on polymer as defined above, in particular the mixtures of polymers as defined above, is provided as a solution, wherein the solvent is ethyl acetate or n-heptane, and is preferably ethyl acetate.

In a preferred embodiment, the at least one polymer as defined above, has a solids content of from 40 % to 70 % by weight.

In step 3) of the process the coating composition is applied to a backing layer or a release liner. As a result, a coated coating composition, i.e. a coating composition being coated on a backing layer or a release liner is obtained.

After the active-pharmaceutical-containing layer is formed in step 4), the process may thus further comprise a step, wherein a release liner or backing layer is applied to the other side of the active-pharmaceutical-containing layer.

In step 4) of the above process of manufacture, drying is performed preferably at a temperature of from 20 to 90 °C, more preferably from 30 to 70 °C.

According to the claims, in the process of manufacture as defined in the above embodiments, the pharmaceutically active agent (i) in the active-pharmaceutical-containing layer is guanfacine.

In another preferred embodiment of the process of manufacture as defined above, the mono-carboxylic acid (ii) is sorbic acid.

In connection with the above embodiments of the process of manufacture it is to be understood that the at least one polymer, the further additives, and the active-pharmaceutical-containing layer, the guanfacine-containing layer are as defined above with regard to the TTS.

## EXAMPLES

The present invention will now be more fully described with reference to the accompanying examples. It should be understood, however, that the following description is illustrative only and should not be taken in any way as a restriction of the invention. Numerical values provided in the examples regarding the amount of ingredients in the composition or the area weight may vary slightly due to manufacturing variability.

### COMPARATIVE EXAMPLE 1A, 1B, 1C AND 1D

#### Coating composition

The formulations of the guanfacine-containing coating compositions of comparative Examples 1A, 1B, 1C and 1C are summarized in Table 1.1 and 1.2 below. The solids %-values refer to the amounts (Amt) in % by weight.

Table 1.1

| Ingredient (Trade Name) | Comp. Ex. 1A | | Comp. Ex. 1B | |
|---|---|---|---|---|
| | Amt [g] | Solids [%] | Amt [g] | Solids [%] |
| Guanfacine Base | 0.81 | 6.03 | - | - |
| Guanfacine Base / Polyoxyethylene (4) lauryl ether (Brij L4) in a ratio of 1:1 | - | - | 1.10 | 8.11 |
| Silicone acrylic hybrid pressure sensitive adhesive in ethyl acetate Solids content of 45.8 % by weight (Dow Corning® SilAc Hybrid PSA 7-6102 from DuPont™) | 23.23 | 79.17 | 24.68 | 83.32 |
| Silicone acrylic hybrid pressure sensitive adhesive in ethyl acetate Solids content of 55.3 % by weight (Dow Corning® SilAc Hybrid PSA 7-6302 from DuPont™) | 1.01 | 4.16 | 1.09 | 4.44 |
| Oleylalcohol | 0.54 | 4.02 | 0.56 | 4.13 |
| Polyoxyethylene (4) lauryl ether (Brij L4) | 0.53 | 3.94 | - | - |
| Sorbic acid | 0.36 | 2.68 | - | - |
| Total | 26.48 | 100.0 | 27.43 | 100.0 |
| Area Weight [g/m$^2$] | 95 | | 96 | |
| Loading API [μg/cm$^2$] | 573 | | 389 | |

Table 1.2

| Ingredient (Trade Name) | Comp. Ex. 1C | | Comp. Ex. 1D | |
|---|---|---|---|---|
| | Amt [g] | Solids [%] | Amt [g] | Solids [%] |
| Guanfacine base | 1.63 | 6.12 | - | - |
| Guanfacine base plus sorbic acid (grinded) | - | - | 1.19 | 8.82 |
| Silicone acrylic hybrid pressure sensitive adhesive in ethyl acetate Solids content of 45.8 or 50.5 % by weight (Dow Corning® SilAc Hybrid PSA 7-6102 from DuPont™) | 42.84 | 81.18 | 23.31 | 79.17 |
| Silicone acrylic hybrid pressure sensitive adhesive in ethyl acetate Solids content of 50.3 or 55.3 % by weight (Dow Corning® SilAc Hybrid PSA 7-6302 from DuPont™) | 2.32 | 4.39 | 1.01 | 4.15 |
| Oleylalcohol | 1.11 | 4.17 | 0.53 | 3.93 |
| Polyoxyethylene (4) lauryl ether (Brij L4) | 1.11 | 4.17 | 0.53 | 3.93 |
| Total | 49.01 | 100.0 | 26.57 | 100.0 |
| Area Weight [g/m$^2$] | 95 | | 89 | |
| Loading API [μg/cm$^2$] | 581 | | 541 | |

**Preparation of the coating composition**

Drug substance, sorbic acid if present and used enhancers were dispersed in the solvent ethyl acetate and optionally ultrasonic treated for approx. 5 min. Then the adhesives were added. These two steps can be done also in reverse order. With a dissolver stirrer the mixture was homogenized at 2000 rpm for 10 minutes.

In case of comparative Example 1B a pre-mixture of guanfacine base and polyoxyethylene (4) lauryl ether (Brij L4) in a ratio of 1:1 was prepared. The pre-mixture was prepared according to the dry-grinding method by milling of a mixture of guanfacine base and polyoxyethylene (4) lauryl ether with zirconium oxide milling beads (bead size 1 mm, in a Retsch Mixer Mill MM 500) at 35 Hz for approx. 1.5 hours to achieve a particle size of approx. 5 μm. For preparing the

coating composition the pre-mixture was stirred and the used enhancers and adhesives were added. With a dissolver stirrer the mixture was homogenized at 2000 rpm for approx. 10 minutes.

In case of comparative Example 1D guanfacine base and sorbic acid in equimolar amounts were grinded in a mortar for 15 minutes. For preparing the coating composition to the guanfacine base and sorbic acid dry grinded and the used enhancers were dissolved in the solvent ethyl acetate and ultrasonic treated for 5 minutes. Then the adhesives were added. With a dissolver stirrer the mixture was homogenized at 2000 rpm for 10 minutes.

**Coating of the coating composition**

The resulting guanfacine-containing coating composition was coated on a polyethylene terephthalate film (Scotchpak 9755, which may function as a release liner) using for example a film applicator from the company Erichsen according to the solid content of the mixture under consideration of the desired coating dry weight and dried at approx. 50 °C for approx. 10 min. Depending on the target area weight the corresponding film applicator gap is between 325 - 350 μm.

The coating thickness was chosen such that removal of the solution results in an area weight of the guanfacine-containing layer of approx. 95 (Comp. Ex. 1A) g/m$^2$, 96 (Comp. Ex. 1B) g/m$^2$, 95 (Comp. Ex. 1C) g/m$^2$ and 89 (Comp. Ex. 1D) g/m$^2$. The dried film was then laminated with a backing layer (PET 15 μm [tsp]) to provide a guanfacine-containing self-adhesive layer structure.

**Preparation of the TTS (concerning all examples)**

The individual systems (TTS) were then punched out from the guanfacine-containing self-adhesive layer structure obtained as described above. Then, the TTS were sealed into pouches of the primary packaging material.

**Measurement of skin permeation**

The permeated amount of TTS prepared according to comparative Examples 1A-D was determined in accordance with the OECD Guideline (adopted April 13, 2004) carried out with a 7.0 mL Franz diffusion cell. Split thickness Goettinger minipig skin (female) was used. A dermatome was used to prepare skin to a thickness of 800 μm, with an intact epidermis for all TTS. Diecuts with an area of release of 1.17 cm$^2$ were punched from the TTS. The guanfacine permeated amount in the receptor medium of the Franz diffusion cell (0.9% sodium chloride solution with 0.1 % sodium azide as antibacteriological agent) at a temperature of 32 ± 1 °C was measured and the corresponding cumulative permeated amount was calculated.

The results are shown in Table 1.3 and 1.4 and Figure 1.1, 1.2 and 1.3.

Table 1.3

| Cumulative permeated amount with SD [μg/cm$^2$] | | | | | | |
|---|---|---|---|---|---|---|
| **Elapsed time [h]** | **Comp. Ex. 1A (n=3)** | | **Comp. Ex. 1B (n=3)** | | **Comp. Ex. 1C (n=3)** | |
| | **Amount** | **SD** | **Amount** | **SD** | **Amount** | **SD** |
| **4** | 0.64 | 0.38 | 0.25 | 0.08 | 0.17 | 0.15 |
| **8** | 1.15 | 0.42 | 0.90 | 0.22 | 0.95 | 0.49 |
| **16** | 6.37 | 0.87 | 5.36 | 0.82 | 5.02 | 1.82 |
| **24** | 19.72 | 1.77 | 13.38 | 1.25 | 12.42 | 3.74 |
| **32** | 39.07 | 2.95 | 23.06 | 1.42 | 21.46 | 5.5 |
| **40** | 62.67 | 4.08 | 33.52 | 1.54 | 31.68 | 6.97 |
| **48** | 86.15 | 5.00 | 44.05 | 1.77 | 42.3 | 8.08 |
| **56** | 110.65 | 5.92 | 54.74 | 1.96 | 52.94 | 8.98 |
| **64** | 134.24 | 7.91 | 65.75 | 2.42 | 64.29 | 9.95 |
| **72** | 156.65 | 7.14 | 76.56 | 2.76 | 74.89 | 11.66 |
| **88** | 193.84 | 6.37 | 95.78 | 3.54 | 95.08 | 13.08 |

Table 1.4

| Cumulative permeated amount with SD [μg/cm²] | | |
|---|---|---|
| Elapsed time [h] | Comp. Ex. 1D (n=3) | |
| | Amount | SD |
| **4** | 0.87 | 0.68 |
| **8** | 5.59 | 3.03 |
| **16** | 24.35 | 7.97 |
| **24** | 50.57 | 11.32 |
| **32** | 78.69 | 13.65 |
| **40** | 106.61 | 15.51 |
| **48** | 133.06 | 16.41 |
| **56** | 157.73 | 15.43 |
| **64** | 180.4 | 13.88 |
| **72** | 201.69 | 13.51 |
| **88** | 234.12 | 12.35 |

**EXAMPLES 1A, 1B, 1C, 1D AND 1E**

**Coating composition**

The formulations of the guanfacine-containing coating compositions of Examples 1A, 1B, 1C, 1D and 1Eare summarized in Table 1.4, Table 1.5 and Table 1.6 below. The solids %-values refer to the amounts (Amt) in % by weight.

Table 1.4

| Ingredient (Trade Name) | Ex. 1A | | Ex. 1B | |
|---|---|---|---|---|
| | Amt [g] | Solids [%] | Amt [g] | Solids [%] |
| Guanfacine base / Sorbic acid / Methanol | 1.96 | 8.68 | 0.98 | 8.67 |
| Silicone acrylic hybrid pressure sensitive adhesive in ethyl acetate Solids content of 45.8 % by weight (Dow Corning® SilAc Hybrid PSA 7-6102 from DuPont™) | 38.92 | 78.93 | - | - |
| Silicone acrylic hybrid pressure sensitive adhesive in ethyl acetate Solids content of 55.3 % by weight (Dow Corning® SilAc Hybrid PSA 7-6302 from DuPont™) | 1.77 | 4.33 | - | - |
| Amine-compatible silicone adhesive (silanol endblocked polydimethylsiloxane polycondensed with a silicate resin and reacted with trimethylsilyl) in ethyl acetate Solids content of 60 % by weight (Liveo™ BIO-PSA Q7-4302 from DuPont™) | - | - | 7.82 | 41.53 |
| Amine-compatible silicone adhesive (silanol endblocked polydimethylsiloxane polycondensed with a silicate resin and reacted with trimethylsilyl) in ethyl acetate Solids content of 60.1 % by weight (Liveo™ BIO-PSA Q7-4202 from DuPont™) | - | - | 7.83 | 41.65 |
| Oleylalcohol | 0.91 | 4.03 | 0.46 | 4.07 |
| Polyoxyethylene (4) lauryl ether (Brij L4) | 0.91 | 4.03 | 0.46 | 4.07 |
| Total | 44.47 | 100.0 | 17.55 | 100.0 |

(continued)

| Ingredient (Trade Name) | Ex. 1A | | Ex. 1B | |
|---|---|---|---|---|
| | Amt [g] | Solids [%] | Amt [g] | Solids [%] |
| Area Weight [g/m2] | 100 | | 94 | |
| Loading API [μg/cm2] | 600 | | 540 | |

Table 1.5

| Ingredient (Trade Name) | Ex. 1C | | Ex. 1D | |
|---|---|---|---|---|
| | Amt [g] | Solids [%] | Amt [g] | Solids [%] |
| Guanfacine Base / Sorbic Acid / Methanol | 0.92 | 8.63 | 0.87 | 8.60 |
| Acrylate-vinylacetate adhesive in ethyl acetate Solids content of 38.6 % by weight (DURO-TAK® 87-4098) | 22.97 | 83.21 | 11.83 | 45.25 |
| Amine-compatible silicone adhesive (silanol endblocked polydimethylsiloxane polycondensed with a silicate resin and reacted with trimethylsilyl) in ethyl acetate Solids content of 60 % by weight (Liveo™ BIO-PSA Q7-4302 from DuPont™) | - | - | 3.88 | 23.07 |
| Amine-compatible silicone adhesive (silanol endblocked polydimethylsiloxane polycondensed with a silicate resin and reacted with trimethylsilyl) in ethyl acetate Solids content of 60.1 % by weight (Liveo™ BIO-PSA Q7-4202 from DuPont™) | - | - | 3.87 | 23.07 |
| Oleylalcohol | 0.42 | 3.94 | - | - |
| Polyoxyethylene (4) lauryl ether (Brij L4) | 0.45 | 4.22 | - | - |
| Total | 24.76 | 100.0 | 20.45 | 100.0 |
| Area Weight [g/m2] | 91 | | 98 | |
| Loading API [μg/cm2] | 542 | | 581 | |

Table 1.6

| Ingredient (Trade Name) | Ex. 1E | |
|---|---|---|
| | Amt [g] | Solids [%] |
| Guanfacine Base / Sorbic Acid / Methanol | 0.98 | 8.70 |
| Acrylate-vinylacetate adhesive in ethyl acetate / ethanol / heptane / methanol Solids content of 42 % by weight (DURO-TAK® 387-2516) | 22.32 | 83.22 |
| Oleylalcohol | 0.45 | 3.99 |
| Polyoxyethylene (4) lauryl ether (Brij L4) | 0.46 | 4.08 |
| Total | 24.21 | 100.0 |
| Area Weight [g/m2] | 81 | |
| Loading API [μg/cm2] | 486 | |

**Preparation of the coating composition**

A pre-mixture of guanfacine base, sorbic acid and methanol was prepared. The pre-mixture was prepared according to the dry-grinding method by milling of a mixture of guanfacine base, sorbic acid and methanol as a catalyst with

zirconium oxide milling beads (bead size 3 mm, in a Retsch Mixer Mill MM 500) at 35 Hz for approx. 10 minutes. Guanfacine base and sorbic acid were present in a ratio of 1:1. For preparing the coating composition the pre-mixture and the used enhancers were dispersed in the solvent ethyl acetate and ultrasonic treated for approx. 5 min. Then the adhesives were added. These two steps can be done also in reverse order. With a dissolver stirrer the mixture was homogenized at 2000 rpm for 10 minutes.

**Coating of the coating composition**

The resulting guanfacine-containing coating composition was coated on a polyethylene terephthalate film (Scotchpak 9755, which may function as a release liner) using for example a film applicator from the company Erichsen according to the solid content of the mixture under consideration of the desired coating dry weight and dried at approx. 50 °C for approx. 10 min. Depending on the target area weight the corresponding film applicator gap is between 300 - 400 μm.

The coating thickness was chosen such that removal of the solution results in an area weight of the guanfacine-containing layer of approx. 100 (Ex. 1A) g/m$^2$, 94 (Ex. 1B) g/m$^2$, 91 (Ex. 1C) g/m$^2$, 98 (Ex. 1D) g/m$^2$ and 81 (Ex. 1E) g/m$^2$. The dried film was then laminated with a backing layer (PET 15 μm [tsp] or MN 19 AB I) to provide a guanfacine-containing self-adhesive layer structure.

**Preparation of the TTS**

See comparative Examples 1A-1D.

**Measurement of skin permeation**

The permeated amount of TTS prepared according to Examples 1A-E was determined in accordance with the OECD Guideline (adopted April 13, 2004) carried out with a 7.0 mL Franz diffusion cell. Split thickness Goettinger minipig skin (female) was used. A dermatome was used to prepare skin to a thickness of 800 μm, with an intact epidermis for all TTS. Diecuts with an area of release of 1.17 cm$^2$ were punched from the TTS. The guanfacine permeated amount in the receptor medium of the Franz diffusion cell (0.9 % sodium chloride solution with 0.1 % sodium azide as antibacteriological agent) at a temperature of 32 ± 1 °C was measured and the corresponding cumulative permeated amount was calculated.

The results are shown in Tables 1.7 and 1.8 and Figure 1.3.

Table 1.7

| Cumulative permeated amount with SD [μg/cm$^2$] | | | | |
|---|---|---|---|---|
| **Elapsed time [h]** | **Ex. 1A (n=3)** | | **Ex. 1B (n=3)** | |
| | **Amount** | **SD** | **Amount** | **SD** |
| **4** | 0.2 | 0.13 | 0.67 | 0.55 |
| **8** | 0.83 | 0.44 | 2.56 | 2.24 |
| **16** | 6.08 | 1.41 | 11.79 | 7.71 |
| **24** | 18.24 | 2.46 | 29.06 | 14.43 |
| **32** | 34.96 | 3.2 | 51.06 | 20.74 |
| **40** | 54.22 | 3.59 | 75.78 | 26.22 |
| **48** | 74.24 | 3.81 | 100.55 | 29.75 |
| **56** | 94.13 | 4.75 | 123.96 | 31.35 |
| **64** | 113.8 | 5.02 | 145.85 | 31.83 |
| **72** | 132.41 | 5.86 | 163.99 | 32.16 |
| **88** | 163 | 6.65 | 191.64 | 31.5 |

Table 1.8

| Cumulative permeated amount with SD [µg/cm²] | | | | | | |
|---|---|---|---|---|---|---|
| **Elapsed time [h]** | **Ex. 1C (n=3)** | | **Ex. 1D (n=3)** | | **Ex. 1E (n=3)** | |
| | **Amount** | **SD** | **Amount** | **SD** | **Amount** | **SD** |
| **4** | 0.32 | 0.52 | 0 | 0 | 0.3 | 0.49 |
| **8** | 1.62 | 1.99 | 0.02 | 0.02 | 1.22 | 1.75 |
| **16** | 7.86 | 7.5 | 0.31 | 0.05 | 6.24 | 6.41 |
| **24** | 18.9 | 15.44 | 0.99 | 0.08 | 17.02 | 13.11 |
| **32** | 32.92 | 23.73 | 2.3 | 0.3 | 32.64 | 19.87 |
| **40** | 49.09 | 31.86 | 4.11 | 0.71 | 52.73 | 27.02 |
| **48** | 65.36 | 38.49 | 6.7 | 1.18 | 74.77 | 32.89 |
| **56** | 81.15 | 43.37 | 9.98 | 1.55 | 97.93 | 37.87 |
| **64** | 96.54 | 46.86 | 13.65 | 2.13 | 121.53 | 40.76 |
| **72** | 110.5 | 48.85 | 17.51 | 3.21 | 145.16 | 44.41 |
| **88** | 134.41 | 51.1 | 26.26 | 4.93 | 186.01 | 45.28 |

**EXAMPLE 2A, 2B, 2C AND COMPARATIVE EXAMPLE 2A AND 2B**

**Coating composition**

The formulations of the guanfacine-containing coating compositions of Examples 2A, 2B and 2C and comparative Example 2A and 2B are summarized in Table 2.1, 2.2 and 2.3 below. The solids %-values refer to the amounts (Amt) in % by weight.

Table 2.1

| **Ingredient (Trade Name)** | **Ex. 2A** | | **Ex.2B** | |
|---|---|---|---|---|
| | **Amt [g]** | **Solids [%]** | **Amt [g]** | **Solids [%]** |
| Guanfacine / Sorbic acid | 0.21 | 8.74 | 0.20 | 8.46 |
| Amine-compatible silicone adhesive (silanol endblocked polydimethylsiloxane polycondensed with a silicate resin and reacted with trimethylsilyl) in ethyl acetate<br>Solids content of 60 % by weight (Liveo™ BIO-PSA Q7-4302 from DuPont™ ) | 1.68 | 41.85 | - | - |
| Amine-compatible silicone adhesive (silanol endblocked polydimethylsiloxane polycondensed with a silicate resin and reacted with trimethylsilyl) in ethyl acetate<br>Solids content of 60.1 % by weight (Liveo™ BIO-PSA Q7-4202 from DuPont™) | 1.68 | 41.92 | - | - |
| Acrylate-vinylacetate adhesive in ethyl acetate<br>Solids content of 38.6 % by weight (DURO-TAK® 87-4098) | - | - | 5.06 | 82.65 |
| Oleylalcohol | 0.09 | 3.75 | 0.09 | 3.81 |
| Polyoxyethylene (4) lauryl ether (Brij L4) | 0.09 | 3.75 | 0.12 | 5.08 |
| Total | 3.74 | 100.0 | 5.47 | 100.0 |
| Area Weight [g/m²] | 137 | | 104 | |
| Loading API [µg/cm²] | 826 | | 607 | |

Table 2.2

| Ingredient (Trade Name) | Ex. 2C | |
|---|---|---|
| | Amt [g] | Solids [%] |
| Guanfacine / Sorbic acid | 0.2 | 8.60 |
| Acrylate-vinylacetate adhesive in ethyl acetate Solids content of 38.6 % by weight (DURO-TAK® 87-4098) | 2.55 | 42.33 |
| Amine-compatible silicone adhesive (silanol endblocked polydimethylsiloxane polycondensed with a silicate resin and reacted with trimethylsilyl) in ethyl acetate Solids content of 60 % by weight (Liveo™ BIO-PSA Q7-4302 from DuPont™) | 0.8 | 20.64 |
| Amine-compatible silicone adhesive (silanol endblocked polydimethylsiloxane polycondensed with a silicate resin and reacted with trimethylsilyl) in ethyl acetate Solids content of 60.1 % by weight (Liveo™ BIO-PSA Q7-4202 from DuPont™) | 0.8 | 20.68 |
| Labrafil M1994 | 0.09 | 3.87 |
| Polyoxyethylene (4) lauryl ether (Brij L4) | 0.09 | 3.87 |
| Total | 4.53 | 100.0 |
| Area Weight [g/m$^2$] | 102 | |
| Loading API [μg/cm$^2$] | 606 | |

Table 2.3

| Ingredient (Trade Name) | Comp. Ex. 2A | | Comp. Ex. 2B | |
|---|---|---|---|---|
| | Amt [g] | Solids [%] | Amt [g] | Solids [%] |
| Guanfacine base | 1.63 | 6.12 | 4.28 | 5.88 |
| Silicone acrylic hybrid pressure sensitive adhesive in ethyl acetate Solids content of 50.5 or 45.8 % by weight (Dow Corning® SilAc Hybrid PSA 7-6102 from DuPont™) | 42.84 | 81.18 | 139.73 | 87.86 |
| Silicone acrylic hybrid pressure sensitive adhesive in ethyl acetate Solids content of 50.3 or 55.3 % by weight (Dow Corning® SilAc Hybrid PSA 7-6302 from DuPont™) | 2.32 | 4.39 | 8.24 | 6.26 |
| Oleylacohol | 1.11 | 4.17 | - | - |
| Polyoxyethylene (4) lauryl ether (Brij L4) | 1.11 | 4.17 | - | - |
| Total | 49.01 | 100.0 | 152.25 | 100.0 |
| Area Weight [g/m$^2$] | 95 | | 95 | |
| Loading API [μg/cm$^2$] | 581 | | 559 | |

**Preparation of the coating composition**

The guanfacine / sorbic acid pre-mixture was prepared according to the slurry method, i.e. according to the following general procedure.
Guanfacine free base and sorbic acid were weighed in in equimolar amounts. Solvent, e.g. DCM 1mL was added and the mixture was stirred with a magnetic stirring bar at room temperature for at least 1 day. The white solid was recovered by filtration under vacuum, washed with solvent (at least 4 mL) and dried under vacuum at 40 °C for 24 hours. In case of comparative Example 2 no pre-mixture was prepared, but the guanfacine was used in the form of the free base.

For preparing the coating composition the guanfacine / sorbic acid pre-mixture or the guanfacine free base and the used enhancers were dispersed in the solvent ethyl acetate and ultrasonic treated for approx. 5 min. Then the adhesives were added. These two steps can be done also in reverse order. With a dissolver stirrer the mixture was homogenized at 2000 rpm for 10 minutes.

For preparing Comparative Examples 2A and 2B guanfacine free base and the used enhancers if present, were dispersed in the solvent ethyl acetate and if necessary ultrasonic treated for approx. 5 min. Then the adhesives were added. These two steps can be done also in reverse order. With a dissolver stirrer the mixture was homogenized at around 1500 rpm for 15 minutes.

## Coating of the coating composition

The resulting guanfacine-containing coating composition was coated on a polyethylene terephthalate film (Scotchpak 9755, which may function as a release liner) using for example a film applicator from the company Erichsen according to the solid content of the mixture under consideration of the desired coating dry weight and dried at approx. 50 °C for approx. 10 min. Depending on the target area weight the corresponding film applicator gap is between 325 - 450 μm.

The coating thickness was chosen such that removal of the solution results in an area weight of the guanfacine-containing layer of approx. 137 (Ex. 2A) g/m$^2$, 104 (Ex. 2B) g/m$^2$, 102 (Ex. 2C) g/m$^2$, 95 (Comp. Ex. 2A) g/m$^2$, and 95 (Comp. Ex. 2B) g/m$^2$. The dried film was then laminated with a backing layer (MN 19 SIL, PET RN15 or PET 15 μm) to provide a guanfacine-containing self-adhesive layer structure.

## Preparation of the TTS

See comparative Examples 1A-1D.

## Measurement of skin permeation

The permeated amount of TTS prepared according to Examples 2A-C and Comparative Examples 2A and 2B was determined in accordance with the OECD Guideline (adopted April 13, 2004) carried out with a 7.0 mL Franz diffusion cell. Split thickness Goettinger minipig skin (female) was used. A dermatome was used to prepare skin to a thickness of 800 μm, with an intact epidermis for all TTS. Diecuts with an area of release of 1.17 cm$^2$ were punched from the TTS. The guanfacine permeated amount in the receptor medium of the Franz diffusion cell (0.9 % sodium chloride solution with 0.1 % sodium azide as antibacteriological agent) at a temperature of 32 ± 1 °C was measured and the corresponding cumulative permeated amount was calculated.

The results are shown in Tables 2.4 and 2.5 and Figure 2.

Table 2.4

| Cumulative permeated amount with SD [μg/cm$^2$] | | | | | | |
|---|---|---|---|---|---|---|
| **Elapsed time [h]** | **Ex. 2A (n=3)** | | **Ex. 2B (n=3)** | | **Ex. 2C (n=3)** | |
| | **Amount** | **SD** | **Amount** | **SD** | **Amount** | **SD** |
| **4** | 1.16 | 0.8 | 0.78 | 0.68 | 0.19 | 0.05 |
| **8** | 3.52 | 2.28 | 2.7 | 2.34 | 0.74 | 0.25 |
| **16** | 11.94 | 6.8 | 11.45 | 7.47 | 5.01 | 1.14 |
| **24** | 23.32 | 10.4 | 27.64 | 13.2 | - | - |
| **32** | 37.87 | 16.79 | 49.26 | 18.22 | 31.89 | 6.04 |
| **40** | 51.81 | 21.41 | 73.09 | 22.44 | 53.01 | 7.67 |
| **48** | 64.21 | 26.28 | 97.82 | 25.38 | 75.75 | 12.23 |
| **56** | 78.08 | 30.97 | 121.73 | 27.05 | 99.3 | 15.73 |
| **64** | 89.93 | 37.04 | 145.65 | 28.54 | 122.58 | 22.53 |
| **72** | 101.56 | 41.05 | 168.61 | 29.84 | 147.39 | 24.61 |
| **88** | 120.77 | 46.36 | 207.8 | 30.97 | 194.65 | 27.42 |

Table 2.5

| Cumulative permeated amount with SD [µg/cm²] | | | | |
|---|---|---|---|---|
| Elapsed time [h] | Comp. Ex. 2A (n=3) | | Comp. Ex. 2B | |
| | Amount | SD | Amount | SD |
| **4** | 0.18 | 0.12 | 0.09 | 0.05 |
| **8** | 0.81 | 0.58 | 0.18 | 0.09 |
| **16** | 4.98 | 2.31 | 0.28 | 0.11 |
| **24** | 12.36 | 3.73 | 0.53 | 0.2 |
| **32** | 21.9 | 4.39 | 1.13 | 0.62 |
| **40** | 32.38 | 5.24 | 2.16 | 1.35 |
| **48** | 43.72 | 6.71 | 3.49 | 2.2 |
| **56** | 54.85 | 8.19 | 5.24 | 3.14 |
| **64** | 66.51 | 9.21 | 7.38 | 4.07 |
| **72** | 77.59 | 11.23 | 9.82 | 5 |
| **88** | 99.51 | 13.49 | 15.13 | 6.42 |

**EXAMPLES 3A, 3B, 3C, 3D, 3E AND COMPARATIVE EXAMPLE 3**

**Coating composition**

The formulations of the guanfacine-containing coating compositions of Examples 3A, 3B, 3C, 3D and 3E and comparative Example 3 are summarized in Table 3.1, 3.2, 3.3 and 3.4 below. The solids %-values refer to the amounts (Amt) in % by weight.

Table 3.1

| Ingredient (Trade Name) | Ex. 3A | | Ex. 3B | |
|---|---|---|---|---|
| | Amt [g] | Solids [%] | Amt [g] | Solids [%] |
| Guanfacine / Sorbic acid | 0.19 | 8.70 | 0.19 | 7.92 |
| Amine-compatible silicone adhesive (silanol endblocked polydimethylsiloxane polycondensed with a silicate resin and reacted with trimethylsilyl) in ethyl acetate<br>Solids content of 60.1 % by weight (Liveo™ BIO-PSA Q7-4202 from DuPont™) | 1.66 | 45.69 | - | - |
| Amine-compatible silicone adhesive (silanol endblocked polydimethylsiloxane polycondensed with a silicate resin and reacted with trimethylsilyl) in ethyl acetate<br>Solids content of 60 % by weight (Liveo™ BIO-PSA Q7-4302 from DuPont™) | 1.66 | 45.61 | - | - |
| Acrylate-vinylacetate adhesive in ethyl acetate<br>Solids content of 38.6 % by weight (DURO-TAK® 87-4098) | - | - | 5.72 | 92.08 |
| Total | 3.51 | 100.0 | 5.91 | 100.0 |
| Area Weight [g/m²] | 114 | | 87 | |
| Loading API [µg/cm²] | 684 | | 475 | |

Table 3.2

| Ingredient (Trade Name) | Ex. 3C | | Ex. 3D | |
|---|---|---|---|---|
| | Amt [g] | Solids [%] | Amt [g] | Solids [%] |
| Guanfacine / Sorbic acid | 0.2 | 8.42 | 0.2 | 8.68 |
| Acrylate-vinylacetate adhesive in ethyl acetate / ethanol / heptane / methanol<br>Solids content of 42 % by weight (DURO-TAK® 387-2516) | 5.18 | 91.58 | - | - |
| Acrylate-vinylacetate adhesive in ethyl acetate / heptane / isopropanol<br>Solids content of 47.5 % by weight (DURO-TAK® 2054) | - | - | 4.43 | 91.32 |
| Total | 5.38 | 100.0 | 4.63 | 100.0 |
| Area Weight [g/m$^2$] | 102 | | 90 | |
| Loading API [μg/cm$^2$] | 612 | | 540 | |

Table 3.3

| Ingredient (Trade Name) | Ex. 3E | |
|---|---|---|
| | Amt [g] | Solids [%] |
| Guanfacine / Sorbic acid | 0.2 | 8.58 |
| Acrylate-vinylacetate adhesive in ethyl acetate<br>Solids content of 38.6 % by weight (DURO-TAK® 87-4098) | 2.78 | 46.06 |
| Amine-compatible silicone adhesive (silanol endblocked polydimethylsiloxane polycondensed with a silicate resin and reacted with trimethylsilyl) in ethyl acetate<br>Solids content of 60.1 % by weight (Liveo™ BIO-PSA Q7-4202 from DuPont™) | 0.88 | 22.66 |
| Amine-compatible silicone adhesive (silanol endblocked polydimethylsiloxane polycondensed with a silicate resin and reacted with trimethylsilyl) in ethyl acetate<br>Solids content of 60 % by weight (Liveo™ BIO-PSA Q7-4302 from DuPont™) | 0.88 | 22.70 |
| Total | 4.74 | 100.0 |
| Area Weight [g/m$^2$] | 96 | |
| Loading API [μg/cm$^2$] | 568 | |

Table 3.4

| Ingredient (Trade Name) | Comp. Ex. 3 | |
|---|---|---|
| | Amt [g] | Solids [%] |
| Guanfacine base | 4.28 | 5.88 |
| Silicone acrylic hybrid pressure sensitive adhesive in ethyl acetate<br>Solids content of 45.8 % by weight (Dow Corning® SilAc Hybrid PSA 7-6102 from DuPont™) | 139.73 | 87.87 |
| Silicone acrylic hybrid pressure sensitive adhesive in ethyl acetate<br>Solids content of 55.3 % by weight (Dow Corning® SilAc Hybrid PSA 7-6302 from DuPont™) | 8.24 | 6.26 |
| Total | 152.25 | 100.0 |
| Area Weight [g/m$^2$] | 95 | |
| Loading API [μg/cm$^2$] | 559 | |

## Preparation of the coating composition

The guanfacine / sorbic acid pre-mixture was prepared according to the slurry method, i.e. according to the following general procedure.
Guanfacine free base and sorbic acid were weighed in in equimolar amounts. Solvent, e.g. DCM 1mL was added and the mixture was stirred with a magnetic stirring bar at room temperature for at least 1 day. The white solid was recovered by filtration under vacuum, washed with solvent (at least 4 mL) and dried under vacuum at 40 °C for 24 hours. In case of comparative Example 3 no pre-mixture was prepared, but the guanfacine was used in the form of the free base.

For preparing the coating composition the guanfacine / sorbic acid pre-mixture or the guanfacine free base and the adhesives were dispersed in the solvent ethyl acetate and the mixture was homogenized with a dissolver stirrer at 2000 rpm for approx.10 minutes. In case of Ex. 3A and if BIO-PSA 4202 and BIO-PSA 4302 are used together the adhesives were prior to mixing with the guanfacine / sorbic acid pre-mixture mixed together and stirred at 2000 rpm for approx. 10 min until homogenized.

## Coating of the coating composition

The resulting guanfacine-containing coating composition was coated on a polyethylene terephthalate film (Scotchpak 9755, which may function as a release liner) using for example a film applicator from the company Erichsen according to the solid content of the mixture under consideration of the desired coating dry weight and dried at approx. 50 °C for approx. 10 min. Depending on the target area weight the corresponding film applicator gap is between 300 - 400 μm.

The coating thickness was chosen such that removal of the solution results in an area weight of the guanfacine-containing layer of approx. 114 (Ex. 3A) g/m$^2$, 87 (Ex. 3B) g/m$^2$, 102 (Ex. 3C) g/m$^2$, 90 (Ex. 3D) g/m$^2$, 96 (Ex. 3E) g/m$^2$ and 95 (Comp. Ex. 3) g/m$^2$. The dried film was then laminated with a backing layer (MN 19 SIL or PET 15 μm) to provide a guanfacine-containing self-adhesive layer structure.

## Preparation of the TTS

See comparative Examples 1A-1D.

## Measurement of skin permeation

The permeated amount of TTS prepared according to Examples 3A-E and Comparative Example 3 was determined in accordance with the OECD Guideline (adopted April 13, 2004) carried out with a 7.0 mL Franz diffusion cell. Split thickness Goettinger minipig skin (female) was used. A dermatome was used to prepare skin to a thickness of 800 μm, with an intact epidermis for all TTS. Diecuts with an area of release of 1.17 cm$^2$ were punched from the TTS. The guanfacine permeated amount in the receptor medium of the Franz diffusion cell (0.9 % sodium chloride solution with 0.1 % sodium azide as antibacteriological agent) at a temperature of 32 ± 1 °C was measured and the corresponding cumulative permeated amount was calculated.

The results are shown in Tables 3.5 and 3.6 and Figure 3.

Table 3.5

| Cumulative permeated amount with SD [μg/cm$^2$] | | | | | | |
|---|---|---|---|---|---|---|
| **Elapsed time [h]** | **Ex. 3A (n=3)** | | **Ex. 3B (n=3)** | | **Ex. 3C (n=3)** | |
| | **Amount** | **SD** | **Amount** | **SD** | **Amount** | **SD** |
| **4** | 0.01 | 0 | 0.04 | 0.05 | 0.08 | 0.12 |
| **8** | 0.05 | 0.02 | 0.2 | 0.17 | 0.25 | 0.35 |
| **16** | 0.12 | 0.08 | 0.66 | 0.45 | 0.67 | 0.91 |
| **24** | 0.38 | 0.19 | 1.64 | 0.78 | 1.48 | 1.53 |
| **32** | 1 | 0.36 | 3.28 | 1.21 | 2.93 | 2.13 |
| **40** | 2.09 | 0.59 | 5.55 | 1.69 | 5.2 | 2.62 |
| **48** | 3.79 | 0.84 | 8.5 | 2.25 | 8.44 | 3.03 |
| **56** | 5.95 | 1.08 | 12.1 | 2.92 | 12.45 | 3.49 |
| **64** | 8.48 | 1.27 | 16.1 | 3.6 | 17.02 | 3.52 |

(continued)

| Cumulative permeated amount with SD [μg/cm²] | | | | | | |
|---|---|---|---|---|---|---|
| **Elapsed time [h]** | **Ex. 3A (n=3)** | | **Ex. 3B (n=3)** | | **Ex. 3C (n=3)** | |
| | **Amount** | **SD** | **Amount** | **SD** | **Amount** | **SD** |
| **72** | 11.44 | 1.53 | 20.6 | 4.36 | 22.58 | 3.95 |
| **88** | 16.58 | 1.65 | 28.65 | 5.52 | 32.32 | 4.18 |

Table 3.6

| Cumulative permeated amount with SD [μg/cm²] | | | | | | |
|---|---|---|---|---|---|---|
| **Elapsed time [h]** | **Ex. 3D (n=3)** | | **Ex. 3E (n=3)** | | **Comp. Ex. 3 (n=3)** | |
| | **Amount** | **SD** | **Amount** | **SD** | **Amount** | **SD** |
| **4** | 0.02 | 0.03 | 0.16 | 0.24 | 0.05 | 0.04 |
| **8** | 0.08 | 0.08 | 0.45 | 0.63 | 0.13 | 0.11 |
| **16** | 0.17 | 0.16 | 1.13 | 1.56 | 0.39 | 0.38 |
| **24** | 0.32 | 0.24 | 2.12 | 2.76 | 1.04 | 1 |
| **32** | 0.47 | 0.32 | 3.59 | 4.26 | 2.31 | 1.95 |
| **40** | 0.65 | 0.37 | 5.47 | 5.79 | 4.23 | 3.14 |
| **48** | 0.86 | 0.4 | 7.77 | 7.24 | 6.79 | 4.42 |
| **56** | 1.07 | 0.47 | 10.59 | 8.81 | 9.94 | 5.65 |
| **64** | 1.28 | 0.52 | 13.92 | 10.55 | 13.55 | 6.82 |
| **72** | 1.54 | 0.57 | 17.93 | 12.6 | 17.78 | 7.96 |
| **88** | 2.05 | 0.64 | 25.03 | 15.66 | 25.36 | 9.33 |

**EXAMPLES 4A, 4B, 4C, 4D AND COMPARATIVE EXAMPLE 4**

**Coating composition**

The formulations of the guanfacine-containing coating compositions of Examples 4A, 4B, 4C and 4D and Comparative Example 4 are summarized in Table 4.1, 4.2 and 4.3 below. The solids %-values refer to the amounts (Amt) in % by weight.

Table 4.1

| **Ingredient (Trade Name)** | **Ex. 4A** | | **Ex. 4B** | |
|---|---|---|---|---|
| | **Amt [g]** | **Solids [%]** | **Amt [g]** | **Solids [%]** |
| Guanfacine / Sorbic acid | 0.2 | 8.73 | 0.20 | 8.97 |
| Silicone acrylic hybrid pressure sensitive adhesive in ethyl acetate Solids content of 50 % by weight (Dow Corning® SilAc Hybrid PSA 7-6102 from DuPont™) | 3.62 | 79.04 | 3.48 | 78.03 |
| Silicone acrylic hybrid pressure sensitive adhesive in ethyl acetate Solids content of 50 % by weight (Dow Corning® SilAc Hybrid PSA 7-6302 from DuPont™) | 0.2 | 4.37 | 0.18 | 4.04 |
| Labrafil M1944 | 0.100 | 3.93 | - | - |
| Oleylalcohol | - | - | 0.091 | 4.04 |
| Polyoxyethylene (4) lauryl ether (Brij L4) | 0.097 | 3.93 | 0.11 | 4.93 |

(continued)

| Ingredient (Trade Name) | Ex. 4A | | Ex. 4B | |
|---|---|---|---|---|
| | Amt [g] | Solids [%] | Amt [g] | Solids [%] |
| Total | 4.22 | 100.0 | 4.06 | 100.0 |
| Area Weight [g/m$^2$] | 103 | | 93 | |
| Loading API [μg/cm$^2$] | 620 | | 575 | |

Table 4.2

| Ingredient (Trade Name) | Ex. 4C | | Ex. 4D | |
|---|---|---|---|---|
| | Amt [g] | Solids [%] | Amt [g] | Solids [%] |
| Guanfacine / Sorbic acid | 0.22 | 8.66 | 0.2 | 8.47 |
| Silicone acrylic hybrid pressure sensitive adhesive in ethyl acetate Solids content of 50 % by weight (Dow Corning® SilAc Hybrid PSA 7-6102 from DuPont™) | 4.00 | 78.74 | 3.71 | 78.39 |
| Silicone acrylic hybrid pressure sensitive adhesive in ethyl acetate Solids content of 50 % by weight (Dow Corning® SilAc Hybrid PSA 7-6302 from DuPont™) | 0.22 | 4.33 | 0.19 | 4.24 |
| Transcutol | - | - | 0.10 | 4.24 |
| Lauroglycol 90 | 0.11 | 4.33 | - | - |
| Polyoxyethylene (4) lauryl ether (Brij L4) | 0.10 | 3.94 | 0.11 | 4.66 |
| Total | 4.65 | 100.0 | 4.31 | 100.0 |
| Area Weight [g/m$^2$] | 96 | | 100 | |
| Loading API [μg/cm$^2$] | 573 | | 584 | |

Table 4.3

| Ingredient (Trade Name) | Comp. Ex. 4 | |
|---|---|---|
| | Amt [g] | Solids [%] |
| Guanfacine base | 1.63 | 6.12 |
| Silicone acrylic hybrid pressure sensitive adhesive in ethyl acetate Solids content of 50.5% by weight (Dow Corning® SilAc Hybrid PSA 7-6102 from DuPont™) | 42.84 | 81.18 |
| Silicone acrylic hybrid pressure sensitive adhesive in ethyl acetate Solids content of 50.3 % by weight (Dow Corning® SilAc Hybrid PSA 7-6302 from DuPont™) | 2.32 | 4.38 |
| Oleylacohol | 1.11 | 4.16 |
| Polyoxyethylene (4) lauryl ether (Brij L4) | 1.11 | 4.16 |
| Total | 49.01 | 100.0 |
| Area Weight [g/m$^2$] | 95 | |
| Loading API [μg/cm$^2$] | 581 | |

**Preparation of the coating composition**

The guanfacine / sorbic acid pre-mixture was prepared according to the slurry method, i.e. according to the following general procedure.

Guanfacine free base and sorbic acid were weighed in in equimolar amounts. Solvent, e.g. DCM 1mL was added and the

mixture was stirred with a magnetic stirring bar at room temperature for at least 1 day. The white solid was recovered by filtration under vacuum, washed with solvent (at least 4 mL) and dried under vacuum at 40 °C for 24 hours. In case of comparative Example 4 no pre-mixture was prepared, but the guanfacine was used in the form of the free base.

For preparing the coating composition the guanfacine / sorbic acid pre-mixture or the guanfacine free base, the used enhancers and the adhesives were dispersed in the solvent ethyl acetate and the mixture was homogenized with a dissolver stirrer at 2000 rpm for approx. 10 minutes. The adhesives SilAc 6102 and SilAc 6302 when used together were prior to mixing with the guanfacine / sorbic acid pre-mixture mixed together and stirred at 2000 rpm for approx. 10 min until homogenized.

For Comparative Example 4 the guanfacine free base and the used enhancers were dissolved in the solvent ethyl acetate and the adhesives were added. The mixture was homogenized with a dissolver stirrer at 2000 rpm for approx. 10 minutes.

**Coating of the coating composition**

The resulting guanfacine-containing coating composition was coated on a polyethylene terephthalate film (Scotchpak 9755, which may function as a release liner) using for example a film applicator from the company Erichsen according to the solid content of the mixture under consideration of the desired coating dry weight and dried at approx. 50 °C for approx. 10 min. Depending on the target area weight the corresponding film applicator gap is between 325 - 350 μm.

The coating thickness was chosen such that removal of the solution results in an area weight of the guanfacine-containing layer of approx. 103 (Ex. 4A) g/m$^2$, 93 (Ex. 4B) g/m$^2$, 96 (Ex. 4C) g/m$^2$, 100 (Ex. 4D) g/m$^2$ and 95 (Comp. Ex. 4) g/m$^2$. The dried film was then laminated with a backing layer (PET 15 μm) to provide a guanfacine-containing self-adhesive layer structure.

**Preparation of the TTS**

See comparative Examples 1A-1D.

**Measurement of skin permeation**

The permeated amount of TTS prepared according to Examples 4A-D and Comparative Example 4 was determined in accordance with the OECD Guideline (adopted April 13, 2004) carried out with a 7.0 mL Franz diffusion cell. Split thickness Goettinger minipig skin (female) was used. A dermatome was used to prepare skin to a thickness of 800 μm, with an intact epidermis for all TTS. Diecuts with an area of release of 1.17 cm$^2$ were punched from the TTS. The guanfacine permeated amount in the receptor medium of the Franz diffusion cell (0.9 % sodium chloride solution with 0.1 % sodium azide as antibacteriological agent) at a temperature of 32 ± 1 °C was measured and the corresponding cumulative permeated amount was calculated.

The results are shown in Tables 4.3 and 4.4 and Figure 4.1 and 4.2.

Table 4.3

| Cumulative permeated amount with SD [μg/cm$^2$] | | | | |
|---|---|---|---|---|
| **Elapsed time [h]** | **Ex. 4A (n=3)** | | **Ex. 4B (n=3)** | |
| | **Amount** | **SD** | **Amount** | **SD** |
| **4** | 0.52 | 0.39 | 0.74 | 0.4 |
| **8** | 2.98 | 1.81 | 3.38 | 1.26 |
| **16** | 16.75 | 7.24 | 18.65 | 3.61 |
| **24** | 38.5 | 12.71 | 43.91 | 5.25 |
| **32** | 64.85 | 17.02 | 75.78 | 6.08 |
| **40** | 93.12 | 19.42 | 110.99 | 5.56 |
| **48** | 120.89 | 20.26 | 143.5 | 7.2 |
| **56** | 147.47 | 20.02 | 176.85 | 5.77 |
| **64** | 174 | 19.27 | 207.33 | 6.26 |
| **72** | 199.51 | 17.86 | 236.58 | 4.8 |

(continued)

| Cumulative permeated amount with SD [µg/cm²] | | | | |
|---|---|---|---|---|
| Elapsed time [h] | Ex. 4A (n=3) | | Ex. 4B (n=3) | |
| | Amount | SD | Amount | SD |
| **88** | 242.24 | 13.78 | 281.16 | 9.25 |

Table 4.4

| Cumulative permeated amount with SD [µg/cm²] | | | | | | |
|---|---|---|---|---|---|---|
| Elapsed time [h] | Ex. 4C (n=3) | | Ex. 4D (n=3) | | Comp. Ex. 4 (n=2) | |
| | Amount | SD | Amount | SD | Amount | SD |
| **4** | 1.01 | 0.99 | -0.11 | 0.01 | 0.6 | 0.47 |
| **8** | 4.51 | 2.93 | -0.05 | 0.05 | 2.76 | 1.35 |
| **16** | 21.47 | 8.61 | 0.44 | 0.21 | 10.6 | 2.84 |
| **24** | 47.17 | 14.17 | 1.89 | 0.34 | 21.35 | 3.92 |
| **32** | 78.92 | 19.57 | 5.09 | 0.91 | 34.08 | 4.82 |
| **40** | 113.46 | 23.09 | 8.94 | 1.63 | 48.07 | 5.86 |
| **48** | 147.86 | 26.13 | 13.81 | 2.09 | 62.74 | 7.99 |
| **56** | 181.37 | 28.56 | 18.69 | 2.69 | 77.03 | 9.71 |
| **64** | 215.46 | 31.38 | 24.28 | 3.35 | 91.84 | 11.07 |
| **72** | 247.41 | 32.92 | 28.74 | 2.97 | 106.28 | 12.61 |
| **88** | 302.19 | 33 | 36.36 | 2.94 | 132.5 | 14.25 |

**COMPARATIVE EXAMPLES 5A, 5B, 5C, 5D, 5E AND 5F**

**Coating composition**

The formulations of the guanfacine-containing coating compositions of comparative Examples 5A, 5B, 5C, 5D, 5E and 5F are summarized in Tables 5.1, 5.2, 5.3 and 5.4 below. The solids %-values refer to the amounts (Amt) in % by weight.

Table 5.1

| Ingredient (Trade Name) | Comp. Ex. 5A | | Comp. Ex. 5B | |
|---|---|---|---|---|
| | Amt [g] | Solids [%] | Amt [g] | Solids [%] |
| Guanfacine Base / Pimelic acid (dry grinding) | 1.11 | 9.84 | 1.12 | 9.93 |
| Amine-compatible silicone adhesive (silanol endblocked polydimethylsiloxane polycondensed with a silicate resin and reacted with trimethylsilyl) in ethyl acetate<br>Solids content of 60.1 % by weight (Liveo™ BIO-PSA Q7-4202 from DuPont™) | 7.70 | 41.05 | - | - |
| Amine-compatible silicone adhesive (silanol endblocked polydimethylsiloxane polycondensed with a silicate resin and reacted with trimethylsilyl) in ethyl acetate<br>Solids content of 60 % by weight (Liveo™ BIO-PSA Q7-4302 from DuPont™) | 7.70 | 40.96 | - | - |

(continued)

| Ingredient (Trade Name) | Comp. Ex. 5A | | Comp. Ex. 5B | |
|---|---|---|---|---|
| | Amt [g] | Solids [%] | Amt [g] | Solids [%] |
| Acrylate-vinylacetate adhesive in ethyl acetate / ethanol / heptane / methanol<br>Solids content of 42 % by weight (DURO-TAK® 387-2516) | - | - | 22.05 | 82.09 |
| Oleylalcohol | 0.46 | 4.08 | 0.45 | 3.99 |
| Polyoxyethylene (4) lauryl ether (Brij L4) | 0.46 | 4.08 | 0.45 | 3.99 |
| Total | 17.43 | 100.0 | 24.07 | 100.0 |
| Area Weight [g/m$^2$] | 90 | | 96 | |
| Loading API [$\mu$g/cm$^2$] | 537 | | 578 | |

Table 5.2

| Ingredient (Trade Name) | Comp. Ex. 5C | | Comp. Ex. 5D | |
|---|---|---|---|---|
| | Amt [g] | Solids [%] | Amt [g] | Solids [%] |
| Guanfacine Base / Pimelic Acid (dry grinding) | 1.05 | 9.78 | 0.99 | 9.86 |
| Amine-compatible silicone adhesive (silanol endblocked polydimethylsiloxane polycondensed with a silicate resin and reacted with trimethylsilyl) in ethyl acetate<br>Solids content of 60 % by weight (Liveo™ BIO-PSA Q7-4302 from DuPont™) | - | - | 3.79 | 22.71 |
| Amine-compatible silicone adhesive (silanol endblocked polydimethylsiloxane polycondensed with a silicate resin and reacted with trimethylsilyl) in ethyl acetate<br>Solids content of 60.1 % by weight (Liveo™ BIO-PSA Q7-4202 from DuPont™) | - | - | 3.77 | 22.51 |
| Acrylate-vinylacetate adhesive in ethyl acetate<br>Solids content of 38.6 % by weight (DURO-TAK® 87-4098) | 22.87 | 82.22 | 11.69 | 44.92 |
| Oleylalcohol | 0.43 | 4.00 | - | - |
| Polyoxyethylene (4) lauryl ether (Brij L4) | 0.43 | 4.00 | - | - |
| Total | 24.78 | 100.0 | 20.24 | 100.0 |
| Area Weight [g/m$^2$] | 112 | | 107 | |
| Loading API [$\mu$g/cm$^2$] | 664 | | 639 | |

Table 5.3

| Ingredient (Trade Name) | Comp. Ex. 5E | |
|---|---|---|
| | Amt [g] | Solids [%] |
| Guanfacine Base / Pimelic Acid (dry grinding) | 2.23 | 9.88 |
| Silicone acrylic hybrid pressure sensitive adhesive in ethyl acetate<br>Solids content of 50 % by weight (Dow Corning® SilAc Hybrid PSA 7-6102 from DuPont™) | 38.34 | 77.77 |
| Silicone acrylic hybrid pressure sensitive adhesive in ethyl acetate<br>Solids content of 50 % by weight (Dow Corning® SilAc Hybrid PSA 7-6302 from DuPont™) | 1.7 | 4.16 |
| Oleylalcohol | 0.91 | 4.03 |

(continued)

| Ingredient (Trade Name) | Comp. Ex. 5E | |
|---|---|---|
| | Amt [g] | Solids [%] |
| Polyoxyethylene (4) lauryl ether (Brij L4) | 0.94 | 4.16 |
| Total | 44.12 | 100.0 |
| Area Weight [g/m$^2$] | 99 | |
| Loading API [µg/cm$^2$] | 593 | |

Table 5.4

| Ingredient (Trade Name) | Comp. Ex. 5F | |
|---|---|---|
| | Amt [g] | Solids [%] |
| Guanfacine Base | 0.80 | 5.99 |
| Silicone acrylic hybrid pressure sensitive adhesive in ethyl acetate Solids content of 50 % by weight (Dow Corning® SilAc Hybrid PSA 7-6102 from DuPont™) | 22.70 | 77.85- |
| Silicone acrylic hybrid pressure sensitive adhesive in ethyl acetate Solids content of 50 % by weight (Dow Corning® SilAc Hybrid PSA 7-6302 from DuPont™) | 1.01 | 4.19 |
| Pimelic Acid | 0.52 | 3.89 |
| Oleylalcohol | 0.53 | 3.97 |
| Polyoxyethylene (4) lauryl ether (Brij L4) | 0.55 | 4.12 |
| Total | 26.11 | 100.0 |
| Area Weight [g/m$^2$] | 104 | |
| Loading API [µg/cm$^2$] | 623 | |

**Preparation of the coating composition**

A pre-mixture of guanfacine base and pimelic acid was prepared. The pre-mixture was prepared according to the grinding method by milling of a mixture of guanfacine base and pimelic acid with zirconium oxide milling beads (bead size 3 mm, in a Retsch Mixer Mill MM 500) at 35 Hz for approx. 10 minutes. Guanfacine base and pimelic acid were present in a ratio of 1:1. For preparing the coating composition the pre-mixture and the used enhancers were dispersed in the solvent ethyl acetate and ultrasonic treated for approx. 5 min. Then the adhesives were added. These two steps can be done also in reverse order. With a dissolver stirrer the mixture was homogenized at 2000 rpm for 10 minutes. In case of comparative Ex. 5F no pre-mixture of guanfacine base and pimelic acid was prepared. Instead, guanfacine base, pimelic acid and the used enhancers were weighed in directly for preparing the coating composition.

**Coating of the coating composition**

The resulting guanfacine-containing coating composition was coated on a polyethylene terephthalate film (Scotchpak 9755, which may function as a release liner) using for example a film applicator from the company Erichsen according to the solid content of the mixture under consideration of the desired coating dry weight and dried at approx. 50 °C for approx. 10 min. Depending on the target area weight the corresponding film applicator gap is between 300 - 525 µm.

The coating thickness was chosen such that removal of the solution results in an area weight of the guanfacine-containing layer of approx. 90 (Comp. Ex. 5A) g/m$^2$, 96 (Comp. Ex. 5B) g/m$^2$, 112 (Comp. Ex. 5C) g/m$^2$, 107 (Comp. Ex. 5D) g/m$^2$, 99 (Comp. Ex. 5E) g/m$^2$ and 104 (Comp. Ex. 5F) g/m$^2$. The dried film was then laminated with a backing layer (PET MN 19 AB 1 or PET 15 µm [tsp]) to provide a guanfacine-containing self-adhesive layer structure.

**Preparation of the TTS**

See comparative Examples 1A-1D.

**Measurement of skin permeation**

The permeated amount of TTS prepared according to comparative Examples 5A-F was determined in accordance with the OECD Guideline (adopted April 13, 2004) carried out with a 7.0 mL Franz diffusion cell. Split thickness Goettinger minipig skin (female) was used. A dermatome was used to prepare skin to a thickness of 800 μm, with an intact epidermis for all TTS. Diecuts with an area of release of 1.17 $cm^2$ were punched from the TTS. The guanfacine permeated amount in the receptor medium of the Franz diffusion cell (0.9 % sodium chloride solution with 0.1 % sodium azide as antibacteriological agent) at a temperature of 32 ± 1 °C was measured and the corresponding cumulative permeated amount was calculated.

The results are shown in Tables 5.5 and 5.6 and Figure 5.1 and 5.2.

Table 5.5

| | Cumulative permeated amount with SD [μg/$cm^2$] | | | | | |
|---|---|---|---|---|---|---|
| **Elapsed time [h]** | **Comp. Ex. 5A (n=3)** | | **Comp. Ex. 5B (n=3)** | | **Comp. Ex. 5C (n=3)** | |
| | **Amount** | **SD** | **Amount** | **SD** | **Amount** | **SD** |
| **4** | 0.04 | 0.07 | 0.03 | 0.05 | 0.02 | 0.04 |
| **8** | 0.22 | 0.32 | 0.21 | 0.32 | 0.18 | 0.32 |
| **16** | 1.25 | 0.95 | 1.31 | 1.13 | 0.93 | 1.17 |
| **24** | 3.74 | 1.83 | 3.58 | 2.53 | 2.85 | 2.39 |
| **32** | 7.93 | 2.99 | 7.26 | 4.35 | 5.91 | 4.07 |
| **40** | 13.69 | 4.31 | 12.15 | 6.26 | 9.93 | 6.04 |
| **48** | 20.55 | 5.53 | 18.12 | 7.88 | 14.83 | 8.08 |
| **56** | 27.94 | 6.48 | 24.64 | 9.03 | 20.13 | 9.9 |
| **64** | 35.75 | 7.24 | 31.61 | 9.72 | 25.72 | 11.37 |
| **72** | 44.14 | 8.31 | 38.84 | 10.22 | 31.78 | 12.49 |
| **88** | 59.52 | 10.13 | 50.82 | 10.19 | 42.27 | 13.25 |

Table 5.6

| | Cumulative permeated amount with SD [μg/$cm^2$] | | | | | |
|---|---|---|---|---|---|---|
| **Elapsed time [h]** | **Comp. Ex. 5D (n=2)** | | **Comp. Ex. 5E (n=2)** | | **Comp. Ex. 5F (n=3)** | |
| | **Amount** | **SD** | **Amount** | **SD** | **Amount** | **SD** |
| **4** | 0 | 0 | 0 | 0 | 0.13 | 0.2 |
| **8** | 0.13 | 0.18 | 0.09 | 0.13 | 0.36 | 0.28 |
| **16** | 0.5 | 0.61 | 1.19 | 0.57 | 1.95 | 0.33 |
| **24** | 0.98 | 1.1 | 4.06 | 0.9 | 6.36 | 0.67 |
| **32** | 1.56 | 1.71 | 8.96 | 1.18 | 14.23 | 1.41 |
| **40** | 2.24 | 2.39 | 15.69 | 1.27 | 25.32 | 1.83 |
| **48** | 3.05 | 3.2 | 24.04 | 1.41 | 38.32 | 1.1 |
| **56** | 3.99 | 4.09 | 33.29 | 1.12 | 51.71 | 0.66 |
| **64** | 5.04 | 5.09 | 43.3 | 0.73 | 65.52 | 2.75 |
| **72** | 6.27 | 6.18 | 53.77 | 0.19 | 78.38 | 4.96 |
| **88** | 8.64 | 8.17 | 71.76 | 0.9 | 99.47 | 8.8 |

**EXAMPLE 6 AND COMPARATIVE EXAMPLE 6**

**Coating composition**

The formulations of the guanfacine-containing coating compositions of Examples and Comparative Example 6 are summarized in Tables 6.1 below. The solids %-values refer to the amounts (Amt) in % by weight.

Table 6.1

| Ingredient (Trade Name) | Ex. 6 | | Comp. Ex. 6 | |
|---|---|---|---|---|
| | Amt [g] | Solids [%] | Amt [g] | Solids [%] |
| Guanfacine / Sorbic acid | 0.84 | 8.42 | - | - |
| Guanfacine / Glutaric acid | - | - | 0.96 | 10.23 |
| Silicone acrylic hybrid pressure sensitive adhesive in ethyl acetate Solids content of 45.8 % by weight (Dow Corning® SilAc Hybrid PSA 7-6102 from DuPont™) | 17.36 | 79.66 | 15.72 | 76.76 |
| Silicone acrylic hybrid pressure sensitive adhesive in ethyl acetate Solids content of 55.3 % by weight (Dow Corning® SilAc Hybrid 7-6302 from DuPont™) | 0.78 | 4.31 | 0.72 | 4.26 |
| Oleylalcohol | 0.37 | 3.71 | 0.42 | 4.48 |
| Polyoxyethylene (4) lauryl ether (Brij L4) | 0.39 | 3.91 | 0.40 | 4.26 |
| Total | 19.74 | 100.0 | 18.22 | 100.0 |
| Area Weight [g/m$^2$] | 89 | | 94 | |
| Loading API [μg/cm$^2$] | 517 | | 578 | |

**Preparation of the coating composition**

The guanfacine / sorbic acid pre-mixture of Example 6 and the guanfacine / glutaric acid pre-mixture of Comparative Example 6 were prepared according to the slurry method, i.e. according to the following general procedure. Guanfacine free base and the respective acid were weighed in in equimolar amounts. Solvent, e.g. DCM 1mL or ethyl acetate 2mL was added and the mixture was stirred with a magnetic stirring bar at room temperature for at least 1 day. The white solid was recovered by filtration under vacuum, washed with solvent (at least 4 mL) and dried under vacuum at 40 °C for 24 hours.

For preparing the coating composition the guanfacine / sorbic acid pre-mixture or the guanfacine / glutaric acid pre-mixture, the used enhancers and the adhesives were dispersed in the solvent ethyl acetate and the mixture was homogenized with a dissolver stirrer at 1400 rpm for approx. 15 minutes.

**Coating of the coating composition**

The resulting guanfacine-containing coating composition was coated on a polyethylene terephthalate film (Scotchpak 9755, which may function as a release liner) using for example a film applicator from the company Erichsen according to the solid content of the mixture under consideration of the desired coating dry weight and dried at approx. 50 °C for approx. 15 min. Depending on the target area weight the corresponding film applicator gap is between 275 - 350 μm.

The coating thickness was chosen such that removal of the solution results in an area weight of the guanfacine-containing layer of approx. 89 (Ex. 6) g/m$^2$ and 94 (Comp. Ex. 6) g/m$^2$. The dried film was then laminated with a backing layer (PET 15 μm [tsp.]) to provide a guanfacine-containing self-adhesive layer structure.

**Preparation of the TTS**

See comparative Examples 1A-1D.

**Measurement of skin permeation**

The permeated amount of TTS prepared according to Example 6 and Comparative Example 6 was determined in accordance with the OECD Guideline (adopted April 13, 2004) carried out with a 7.0 mL Franz diffusion cell. Split thickness

Goettinger minipig skin was used. A dermatome was used to prepare skin to a thickness of 800 μm, with an intact epidermis for all TTS. Diecuts with an area of release of 1.17 $cm^2$ were punched from the TTS. The guanfacine permeated amount in the receptor medium of the Franz diffusion cell (0.9 % sodium chloride solution with 0.1 % sodium azide as antibacteriological agent) at a temperature of 32 ± 1 °C was measured and the corresponding cumulative permeated amount was calculated.

The results are shown in Table 6.1 and Figure 6.

Table 6.1

| Cumulative permeated amount with SD [μg/$cm^2$] | | | | |
|---|---|---|---|---|
| **Elapsed time [h]** | **Ex. 6 (n=3)** | | **Comp. Ex. 6 (n=3)** | |
| | **Amount** | **SD** | **Amount** | **SD** |
| **4** | - | - | - | - |
| **8** | - | - | - | - |
| **16** | 19.39 | 8.35 | 8.34 | 4.77 |
| **24** | 39.82 | 13.72 | 18.02 | 6.81 |
| **32** | 58.96 | 19.97 | 24.92 | 7.7 |
| **40** | 82.32 | 23.81 | 34.88 | 9.14 |
| **48** | 106.66 | 25.49 | 42.2 | 10.16 |
| **56** | 127.34 | 26.35 | 50.64 | 11.19 |
| **64** | 149.24 | 28.53 | 59.3 | 12.8 |
| **72** | 170.43 | 29.3 | 67.03 | 14.52 |
| **80** | 188.66 | 31.54 | 74.13 | 16.55 |
| **88** | 206.52 | 31.06 | 80.92 | 18.23 |

## Claims

**1.** Transdermal therapeutic system for the transdermal administration of guanfacine comprising a guanfacine-containing layer structure, said guanfacine-containing layer structure comprising:

A) a backing layer; and
B) a guanfacine-containing layer comprising guanfacine and a mono-carboxylic acid.

**2.** Transdermal therapeutic system according to claim 1,
wherein the guanfacine-containing layer is a guanfacine-containing matrix layer comprising:

i) guanfacine and a mono-carboxylic acid; and
ii) at least one polymer.

**3.** Transdermal therapeutic system according to claim 1 or 2, wherein the mono-carboxylic acid is sorbic acid.

**4.** Transdermal therapeutic system according to any one of claims 1 to 3, wherein the guanfacine and the mono-carboxylic acid in the guanfacine-containing layer are present in the form of a pre-mixture.

**5.** Transdermal therapeutic system according to any one of claims 1 to 4, wherein the guanfacine and the mono-carboxylic acid in the guanfacine-containing layer are present in the form of a pre-mixture, wherein said pre-mixture is obtainable by a dry-grinding method or a slurry method.

**6.** Transdermal therapeutic system according to any one of claims 1 to 5, wherein the guanfacine-containing layer structure is self-adhesive and preferably does not comprise an additional skin contact layer.

**7.** Transdermal therapeutic system according to any one of claims 1 to 6, wherein the at least one polymer is selected from the group consisting of acrylic polymers, silicone-based polymers, silicone-acrylic hybrid polymers, and mixtures thereof.

**8.** Transdermal therapeutic system according to any one of claims 1 to 7, wherein the at least one polymer is

- a mixture of an acrylic polymer and at least one silicone-based polymer;
- a mixture of two silicone-acrylic hybrid polymers; or
- a mixture of two silicone-based polymers; or
- an acrylic polymer; or
- an acrylic polymer comprising a -OH group.

**9.** Transdermal therapeutic system according to any one of claims 7 or 8, wherein the silicone-based polymer is obtainable by polycondensation of silanol endblocked polydimethylsiloxane with a silicate resin.

**10.** Transdermal therapeutic system according to any one of claims 7 to 9, wherein the acrylic polymer is selected from a copolymer based on vinyl acetate, 2-ethylhexyl-acrylate, 2-hydroxyethyl-acrylate and glycidyl-methacrylate and a copolymer based on 2-ethylhexyl-acrylate and vinyl acetate.

**11.** Transdermal therapeutic system according to any one of claims 7 to 10, wherein the silicone-acrylic hybrid polymer is a silicone acrylic hybrid pressure-sensitive adhesive comprising the reaction product of

(a) a silicon-containing pressure-sensitive adhesive composition comprising acrylate or methacrylate functionality:
(b) an ethylenically unsaturated monomer; and
(c) an initiator.

**12.** Transdermal therapeutic system according to claim 11,

wherein the silicon-containing pressure-sensitive adhesive composition comprising acrylate or methacrylate functionality comprises the condensation reaction product of (a1) a silicone resin, and
(a2) a silicone polymer, and
(a3) a silicon-containing capping agent comprising acrylate or methacrylate functionality, wherein said silicon-containing capping agent is of the general formula $XYR'_bSiZ_{3-b}$, wherein X is a monovalent radical of the general formula AE, where E is - O- or -NH- and A is an acryl group or methacryl group, Y is a divalent alkylene radical having from 1 to 6 carbon atoms, R' is a methyl or phenyl radical, Z is a monovalent hydrolysable organic radical or halogen, and b is 0 or 1;
wherein the silicone resin and silicone polymer are reacted to form a pressure-sensitive adhesive, wherein the silicon-containing capping agent is introduced prior to, during, or after the silicone resin and silicone polymer are reacted,
and wherein the silicon-containing capping agent reacts with the pressure-sensitive adhesive after the silicone resin and silicone polymer have been condensation reacted to form the pressure-sensitive adhesive, or the silicon-containing capping agent reacts in situ with the silicone resin and silicone polymer.

**13.** Transdermal therapeutic system according to any one of claims 11 or 12, wherein the ethylenically unsaturated monomer is selected from the group consisting of aliphatic acrylates, aliphatic methacrylates, cycloaliphatic acrylates, cycloaliphatic methacrylates, and combinations thereof, each of said compounds having up to 20 carbon atoms in the alkyl radical, and wherein the ethylenically unsaturated monomer is preferably a combination of 2-ethylhexyl acrylate and methyl acrylate, particularly preferably in a ratio of from 40:60 to 70:30.

**14.** The transdermal therapeutic system according to any one of claims 1 to 13, wherein the guanfacine-containing layer structure comprises guanfacine in an amount of from 1 to 100 mg/TTS, preferably from 3 to 72 mg/TTS.

**15.** Transdermal therapeutic system according to any one of claims 1 to 14, wherein the guanfacine-containing layer comprises guanfacine in an amount of from 1% to 20%, more preferably in an amount of from 3% to 16% by weight, based on the total weight of the guanfacine-containing layer.

**16.** Transdermal therapeutic system according to any one of claims 1 to 15 comprising guanfacine and the mono-carboxylic acid in equimolar amounts.

**17.** Transdermal therapeutic system according to any one of claims 1 to 16, wherein the guanfacine-containing layer comprises the at least one polymer in an amount of from 20% to 99%, preferably from 30% to 97%, most preferably from 35% to 94% by weight based on the total weight of the guanfacine-containing layer.

**18.** Transdermal therapeutic system according to any one of claims 1 to 17, wherein the guanfacine-containing layer further comprises at least one additive, preferably at least two additives selected from the group consisting of dispersing agents, permeation enhancers and solubilizers.

**19.** Transdermal therapeutic system according to any one of claims 1 to 18, wherein the area weight of the guanfacine-containing layer ranges from 40 to 250 g/$m^2$, preferably from 50 to 180 g/$m^2$, and/or wherein the area of release ranges from 1 to 100 $cm^2$, preferably from 2.5 to 50 $cm^2$.

**20.** Transdermal therapeutic system according to any one of claims 1 to 19 for use in a method of treating a human patient, preferably a human patient at the age of from 6 to 17.

**21.** Transdermal therapeutic system according to any one of claims 1 to 19 for use in a method of treating hypertension or attention deficit hyperactivity disorder (ADHD) and/or as adjunctive therapy to stimulant medications in a human patient, preferably in a human patient at the age of from 6 to 17.

**22.** Transdermal therapeutic system for use according to claim 20 or 21, wherein the transdermal therapeutic system is applied to the skin of the patient for at least 24 hours, preferably at least 72 hours, more preferably about 84 hours.

**23.** Process for manufacturing an active pharmaceutical-containing layer for use in a transdermal therapeutic system comprising the steps of:

1) combining at least the components

(i) a pharmaceutically active agent; and
(ii) at least one mono-carboxylic acid;

to obtain a pre-mixture;
2) combining

(i) the pre-mixture of step 1); and
(ii) at least one polymer;

to obtain a coating composition
3) coating the coating composition onto a backing layer or a release liner to obtain a coated coating composition; and
4) drying the coated coating composition to form the active pharmaceutical-containing layer;

wherein the pharmaceutically active agent is guanfacine.

**24.** The process according to claim 23, wherein the pre-mixture according to step 1) of the process is obtained by combining (i) and (ii) in a dry-grinding method and/or in a slurry method.

## Patentansprüche

**1.** Transdermales therapeutisches System zur transdermalen Verabreichung von Guanfacin mit einer Guanfacin-haltigen Schichtstruktur, wobei die Guanfacin-haltige Schichtstruktur umfasst:

A) eine Trägerschicht; und
B) eine Guanfacin-haltige Schicht, die Guanfacin und eine Monocarbonsäure umfasst.

**2.** Transdermales therapeutisches System nach Anspruch 1,

wobei die Guanfacin-haltige Schicht eine Guanfacin-haltige Matrixschicht ist, umfassend

i) Guanfacin und eine Monocarbonsäure; und
ii) mindestens ein Polymer.

**3.** Transdermales therapeutisches System nach Anspruch 1 oder 2, wobei die Monocarbonsäure Sorbinsäure ist.

**4.** Transdermales therapeutisches System nach einem der Ansprüche 1 bis 3, wobei das Guanfacin und die Monocarbonsäure in der Guanfacin-haltigen Schicht in Form eines Vorgemischs vorliegen.

**5.** Transdermales therapeutisches System nach einem der Ansprüche 1 bis 4, wobei das Guanfacin und die Monocarbonsäure in der Guanfacin-haltigen Schicht in Form eines Vorgemischs vorliegen, wobei das Vorgemisch durch ein Trockenmahlverfahren oder ein Aufschlämmungsverfahren erhältlich ist.

**6.** Transdermales therapeutisches System nach einem der Ansprüche 1 bis 5, wobei die Guanfacin-haltige Schichtstruktur selbstklebend ist und vorzugsweise keine zusätzliche Hautkontaktschicht umfasst.

**7.** Transdermales therapeutisches System nach einem der Ansprüche 1 bis 6, wobei das mindestens eine Polymer ausgewählt ist aus der Gruppe bestehend aus Acrylpolymeren, Polymeren auf Silikonbasis, Silikon-Acryl-Hybridpolymeren und Mischungen davon.

**8.** Transdermales therapeutisches System nach einem der Ansprüche 1 bis 7, wobei es sich bei dem mindestens einen Polymer um

- ein Gemisch aus einem Acrylpolymer und mindestens einem Polymer auf Silikonbasis;
- ein Gemisch aus zwei Silikon-Acryl-Hybridpolymeren; oder
- ein Gemisch aus zwei Polymeren auf Silikonbasis; oder
- ein Acrylpolymer; oder
- ein Acrylpolymer, das eine -OH-Gruppe umfasst.

**9.** Transdermales therapeutisches System nach einem der Ansprüche 7 oder 8, wobei das Polymer auf Silikonbasis durch Polykondensation von silanolendblockiertem Polydimethylsiloxan mit einem Silikatharz erhältlich ist.

**10.** Transdermales therapeutisches System nach einem der Ansprüche 7 bis 9, wobei das Acrylpolymer ausgewählt ist aus einem Copolymer auf Basis von Vinylacetat, 2-Ethylhexylacrylat, 2-Hydroxyethylacrylat und Glycidylmethacrylat und einem Copolymer auf Basis von 2-Ethylhexylacrylat und Vinylacetat.

**11.** Transdermales therapeutisches System nach einem der Ansprüche 7 bis 10, wobei das Silikon-Acryl-Hybridpolymer ein Silikon-Acryl-Hybrid-Haftkleber ist, umfassend das Reaktionsprodukt von

(a) einer siliziumhaltigen druckempfindlichen Klebstoffzusammensetzung mit Acrylat- oder Methacrylatfunktionalität:
(b) einem ethylenisch ungesättigten Monomer; und
(c) einem Initiator.

**12.** Transdermales therapeutisches System nach Anspruch 11,

wobei die siliziumhaltige Haftklebstoffzusammensetzung mit Acrylat- oder Methacrylatfunktionalität das Kondensationsreaktionsprodukt umfasst von (a1) einem Silikonharz und
(a2) einem Silikonpolymer, und
(a3) ein siliziumhaltiges Verkappungsmittel mit Acrylat- oder Methacrylatfunktionalität, wobei das siliziumhaltige Verkappungsmittel die allgemeine Formel $XYR'_bSiZ_{3-b}$hat, wobei X ein einwertiger Rest der allgemeinen Formel AE ist, wobei E -O- oder -NH- ist und A eine Acrylgruppe oder Methacrylgruppe ist, Y ein zweiwertiger Alkylenrest mit 1 bis 6 Kohlenstoffatomen ist, R' ein Methyl- oder Phenylrest ist, Z ein einwertiger hydrolysierbarer organischer Rest oder Halogen ist und b 0 oder 1 ist;
wobei das Silikonharz und das Silikonpolymer unter Bildung eines druckempfindlichen Klebstoffs umgesetzt werden, wobei das siliziumhaltige Verkappungsmittel vor, während oder nach der Umsetzung des Silikonharzes und des Silikonpolymers eingeführt wird, und wobei das siliziumhaltige Verkappungsmittel mit dem Haftklebstoff

reagiert, nachdem das Silikonharz und das Silikonpolymer unter Bildung des Haftklebstoffs kondensationsreagiert wurden, oder das siliziumhaltige Verkappungsmittel in situ mit dem Silikonharz und dem Silikonpolymer reagiert.

**13.** Transdermales therapeutisches System nach einem der Ansprüche 11 oder 12, wobei das ethylenisch ungesättigte Monomer ausgewählt ist aus der Gruppe bestehend aus aliphatischen Acrylaten, aliphatischen Methacrylaten, cycloaliphatischen Acrylaten, cycloaliphatischen Methacrylaten und Kombinationen davon, wobei jede dieser Verbindungen bis zu 20 Kohlenstoffatome im Alkylrest aufweist, und wobei das ethylenisch ungesättigte Monomer vorzugsweise eine Kombination aus 2-Ethylhexylacrylat und Methylacrylat ist, besonders bevorzugt in einem Verhältnis von 40:60 bis 70:30.

**14.** Transdermales therapeutisches System nach einem der Ansprüche 1 bis 13, wobei die Guanfacin-haltige Schichtstruktur Guanfacin in einer Menge von 1 bis 100 mg/TTS, vorzugsweise von 3 bis 72 mg/TTS, umfasst.

**15.** Transdermales therapeutisches System nach einem der Ansprüche 1 bis 14, wobei die Guanfacin-haltige Schicht Guanfacin in einer Menge von 1 bis 20 Gew.-%, bevorzugter in einer Menge von 3 bis 16 Gew.-%, bezogen auf das Gesamtgewicht der Guanfacin-haltigen Schicht, umfasst.

**16.** Transdermales therapeutisches System nach einem der Ansprüche 1 bis 15, umfassend Guanfacin und die Monocarbonsäure in äquimolaren Mengen.

**17.** Transdermales therapeutisches System nach einem der Ansprüche 1 bis 16, wobei die Guanfacin-haltige Schicht das mindestens eine Polymer in einer Menge von 20 bis 99 Gew.-%, vorzugsweise von 30 bis 97 Gew.-%, am meisten bevorzugt von 35 bis 94 Gew.-%, bezogen auf das Gesamtgewicht der Guanfacin-haltigen Schicht, umfasst.

**18.** Transdermales therapeutisches System nach einem der Ansprüche 1 bis 17, wobei die Guanfacin-haltige Schicht weiterhin mindestens ein Additiv, vorzugsweise mindestens zwei Additive, ausgewählt aus der Gruppe bestehend aus Dispergiermitteln, Permeationsverstärkern und Lösungsvermittlern, umfasst.

**19.** Transdermales therapeutisches System nach einem der Ansprüche 1 bis 18, wobei das Flächengewicht der Guanfacin-haltigen Schicht im Bereich von 40 bis 250 g/m$^2$, vorzugsweise von 50 bis 180 g/m$^2$, liegt und/oder wobei die Freisetzungsfläche im Bereich von 1 bis 100 cm$^2$, vorzugsweise von 2,5 bis 50 cm$^2$, liegt.

**20.** Transdermales therapeutisches System nach einem der Ansprüche 1 bis 19 zur Verwendung in einem Verfahren zur Behandlung eines menschlichen Patienten, vorzugsweise eines menschlichen Patienten im Alter von 6 bis 17 Jahren.

**21.** Transdermales therapeutisches System nach einem der Ansprüche 1 bis 19 zur Verwendung in einem Verfahren zur Behandlung von Bluthochdruck oder Aufmerksamkeitsdefizit-Hyperaktivitätsstörung (ADHD) und/oder als Zusatztherapie zu stimulierenden Medikamenten bei einem menschlichen Patienten, vorzugsweise bei einem menschlichen Patienten im Alter von 6 bis 17 Jahren.

**22.** Transdermales therapeutisches System zur Verwendung nach Anspruch 20 oder 21, wobei das transdermale therapeutische System für mindestens 24 Stunden, vorzugsweise mindestens 72 Stunden, noch bevorzugter etwa 84 Stunden, auf die Haut des Patienten aufgetragen wird.

**23.** Verfahren zur Herstellung einer ein aktives Arzneimittel enthaltenden Schicht zur Verwendung in einem transdermalen therapeutischen System, das die folgenden Schritte umfasst:

1) Kombinieren mindestens der Komponenten

(i) eines pharmazeutischen Wirkstoffs; und
(ii) mindestens einer Monocarbonsäure;

um eine Vormischung zu erhalten;
2) Kombinieren

(i) des Vorgemischs aus Schritt 1); und

(ii) mindestens einem Polymer;

um eine Beschichtungszusammensetzung zu erhalten
3) Auftragen der Beschichtungszusammensetzung auf eine Trägerschicht oder eine Trennfolie, um eine beschichtete Beschichtungszusammensetzung zu erhalten; und
4) Trocknen der beschichteten Beschichtungszusammensetzung, um die ein aktives Arzneimittel enthaltende Schicht zu bilden;

wobei das pharmazeutisch aktive Mittel Guanfacin ist.

**24.** Verfahren nach Anspruch 23, wobei die Vormischung gemäß Schritt 1) des Verfahrens durch Kombination von (i) und (ii) in einem Trockenmahlverfahren und/oder in einem Aufschlämmungsverfahren erhalten wird.

## Revendications

**1.** Système thérapeutique transdermique pour l'administration transdermique de guanfacine, comprenant une structure de couches contenant de la guanfacine, ladite structure de couches contenant de la guanfacine comprenant :

A) une couche de support ; et
B) une couche contenant de la guanfacine et un acide monocarboxylique.

**2.** Système thérapeutique transdermique selon la revendication 1, dans lequel la couche contenant de la guanfacine est une couche matricielle contenant de la guanfacine comprenant :

i) de la guanfacine et un acide monocarboxylique ; et
ii) au moins un polymère.

**3.** Système thérapeutique transdermique selon la revendication 1 ou 2, dans lequel l'acide monocarboxylique est l'acide sorbique.

**4.** Système thérapeutique transdermique selon l'une quelconque des revendications 1 à 3, dans lequel la guanfacine et l'acide monocarboxylique de la couche contenant de la guanfacine sont présents sous forme de prémélange.

**5.** Système thérapeutique transdermique selon l'une quelconque des revendications 1 à 4, dans lequel la guanfacine et l'acide monocarboxylique dans la couche contenant de la guanfacine sont présents sous forme d'un prémélange, dans lequel ledit prémélange peut être obtenu par un procédé de broyage à sec ou par un procédé de mise en suspension.

**6.** Système thérapeutique transdermique selon l'une quelconque des revendications 1 à 5, dans lequel la structure de la couche contenant de la guanfacine est auto-adhésive et de préférence ne comprend pas de couche de contact cutané supplémentaire.

**7.** Système thérapeutique transdermique selon l'une quelconque des revendications 1 à 6, dans lequel le au moins un polymère est sélectionné dans le groupe constitué par les polymères acryliques, les polymères à base de silicone, les polymères hybrides silicone-acryliques et leurs mélanges.

**8.** Système thérapeutique transdermique selon l'une quelconque des revendications 1 à 7, dans lequel le au moins un polymère est :

- un mélange d'un polymère acrylique et d'au moins un polymère à base de silicone ;
- un mélange de deux polymères hybrides silicone-acrylique ; ou
- un mélange de deux polymères à base de silicone ; ou
- un polymère acrylique ; ou
- un polymère acrylique comprenant un groupe -OH.

**9.** Système thérapeutique transdermique selon l'une quelconque des revendications 7 ou 8, dans lequel le polymère à base de silicone peut être obtenu par polycondensation de polydiméthylsiloxane à terminaison silanol avec une résine

de silicate.

**10.** Système thérapeutique transdermique selon l'une quelconque des revendications 7 à 9, dans lequel le polymère acrylique est sélectionné parmi un copolymère à base d'acétate de vinyle, 2-éthylhexylacrylate, 2-hydroxyéthyl-acrylate et glycidyl-méthacrylate et un copolymère à base de 2-éthylhexyl-acrylate et d'acétate de vinyle.

**11.** Système thérapeutique transdermique selon l'une quelconque des revendications 7 à 10, dans lequel le polymère hybride silicone-acrylique est un adhésif hybride silicone-acrylique sensible à la pression comprenant le produit de la réaction de :

(a) une composition adhésive sensible à la pression contenant du silicium et comprenant une fonctionnalité acrylate ou méthacrylate ;
(b) un monomère éthyléniquement insaturé ; et
(c) un initiateur.

**12.** Système thérapeutique transdermique selon la revendication 11, dans lequel la composition adhésive sensible à la pression contenant du silicium comprenant une fonctionnalité acrylate ou méthacrylate comprend le produit de la réaction de condensation de :

(a1) une résine de silicone, et
(a2) un polymère de silicone, et
(a3) un agent de coiffage contenant du silicium comprenant une fonctionnalité acrylate ou méthacrylate, dans lequel ledit agent de coiffage contenant du silicium présente la formule générale XYR'bSiZ3-b, où X est un radical monovalent de formule générale AE, où E est -O- ou -NH- et A est un groupe acrylique ou méthacrylique, Y est un radical alkylène divalent comportant de 1 à 6 atomes de carbone, R' est un radical méthyle ou phényle, Z est un radical organique monovalent hydrolysable ou un halogène et b est égal à 0 ou 1 ;
dans lequel la résine de silicone et le polymère de silicone réagissent entre eux pour former un adhésif sensible à la pression, dans lequel l'agent de coiffage contenant du silicium est introduit avant, pendant ou après que la résine de silicone et du polymère de silicone aient réagi et dans lequel l'agent de coiffage contenant du silicium réagisse avec l'adhésif sensible à la pression après que la résine de silicone et le polymère de silicone aient réagi par condensation pour former l'adhésif sensible à la pression, ou l'agent de coiffage contenant du silicium réagisse in situ avec la résine de silicone et le polymère de silicone.

**13.** Système thérapeutique transdermique selon l'une quelconque des revendications 11 ou 12, dans lequel le monomère éthyléniquement insaturé est sélectionné dans le groupe constitué par les acrylates aliphatiques, les méthacrylates aliphatiques, les acrylates cycloaliphatiques, les méthacrylates cycloaliphatiques et leurs combinaisons, chacun de ces composés comportant jusqu'à 20 atomes de carbone dans le radical alkyle et dans lequel le monomère éthyléniquement insaturé est de préférence une combinaison d'acrylate de 2-éthylhexyle et d'acrylate de méthyle, particulièrement préférable selon un rapport compris entre 40/60 et 70/30.

**14.** Système thérapeutique transdermique selon l'une quelconque des revendications 1 à 13, dans lequel la structure de couches contenant la guanfacine comprend de la guanfacine en une quantité comprise entre 1 et 100 mg/TTS, de préférence entre 3 et 72 mg/TTS.

**15.** Système thérapeutique transdermique selon l'une quelconque des revendications 1 à 14, dans lequel la couche contenant de la guanfacine comprend de la guanfacine en une proportion comprise entre 1% et 20%, plus préférablement en une proportion comprise entre 3 % et 16 % en poids, exprimés par rapport au poids total de la couche contenant de la guanfacine.

**16.** Système thérapeutique transdermique selon l'une quelconque des revendications 1 à 15, comprenant de la guanfacine et de l'acide monocarboxylique en quantités équimolaires.

**17.** Système thérapeutique transdermique selon l'une quelconque des revendications 1 à 16, dans lequel la couche contenant de la guanfacine comprend le au moins un polymère en une proportion comprise entre 20 % et 99 %, de préférence entre 30 % et 97 %, idéalement entre 35 % et 94 % en poids, exprimés par rapport au poids total de la couche contenant de la guanfacine.

**18.** Système thérapeutique transdermique selon l'une quelconque des revendications 1 à 17, dans lequel la couche

contenant de la guanfacine comprend en outre au moins un additif, de préférence au moins deux additifs sélectionnés parmi les agents dispersants, les agents améliorant la perméation et les agents solubilisants.

**19.** Système thérapeutique transdermique selon l'une quelconque des revendications 1 à 18, dans lequel le poids par unité de surface de la couche contenant de la guanfacine est compris entre 40 et 250 $g/m^2$, de préférence entre 50 et 180 $g/m^2$, et/ou dans lequel la surface de libération varie entre 1 et 100 $cm^2$, de préférence entre 2,5 et 50 $cm^2$.

**20.** Système thérapeutique transdermique selon l'une quelconque des revendications 1 à 19, destiné à être utilisé dans un procédé de traitement d'un patient humain, de préférence âgé de 6 à 17 ans.

**21.** Système thérapeutique transdermique selon l'une quelconque des revendications 1 à 19, pour une utilisation dans un procédé de traitement de l'hypertension ou de troubles de déficit de l'attention avec hyperactivité (TDAH) et/ou comme traitement d'appoint à des médicaments stimulants chez un patient humain, de préférence âgé de 6 à 17 ans.

**22.** Système thérapeutique transdermique destiné à être utilisé selon la revendication 20 ou 21, dans lequel le système thérapeutique transdermique est appliqué sur la peau du patient pendant au moins 24 heures, de préférence au moins 72 heures, plus préférablement environ 84 heures.

**23.** Procédé de fabrication d'une couche pharmaceutique active pour une utilisation contenant un principe actif pharmaceutique destiné à être utilisée dans un système thérapeutique transdermique, comprenant les étapes suivantes :

1) combinaison d'au moins les composants

(i) un agent pharmaceutiquement actif ; et
(ii) au moins un acide monocarboxylique ;

pour obtenir un prémélange ;
2) combinaison

(i) du prémélange de l'étape 1 ; et
(ii) d'au moins un polymère ;

pour obtenir une composition de revêtement
3) revêtement de la composition de revêtement sur une couche de support ou un film amovible pour obtenir le revêtement de la composition de revêtement; et
4) séchage de la composition de revêtement revêtue pour former la couche contenant le principe pharmaceutique actif ; dans lequel l'agent pharmaceutiquement actif est la guanfacine.

**24.** Procédé selon la revendication 23, dans lequel le prémélange selon l'étape 1 du procédé est obtenu par combinaison de (i) et (ii) par un procédé de broyage à sec et/ou par un procédé de mise en suspension.

Fig. 1.1

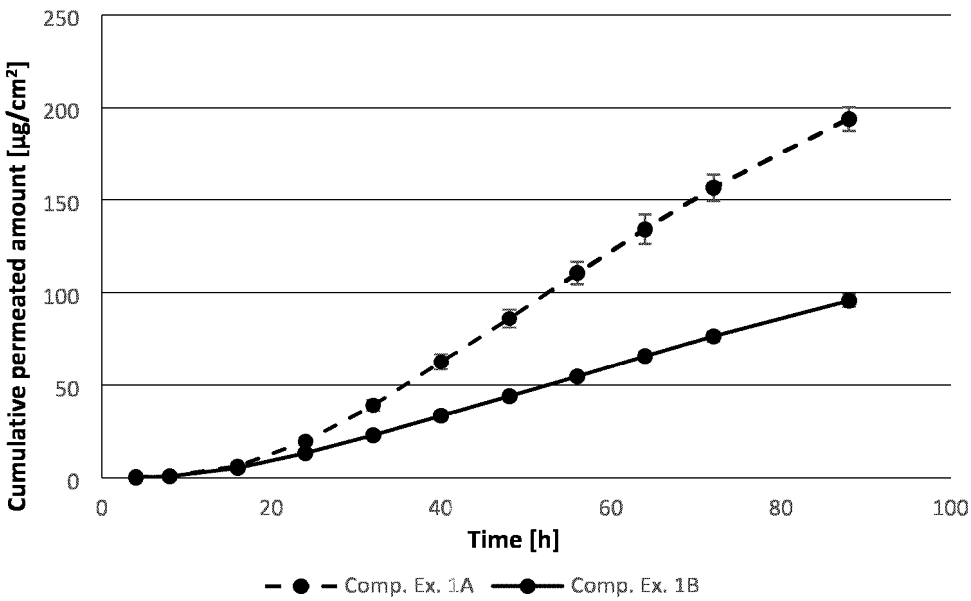
Cumulative permeated amount [µg/cm²]
250
200
150
100
50
0
0
20
40
60
80
100
Time [h]
Comp. Ex. 1A
Comp. Ex. 1B

Fig. 1.2

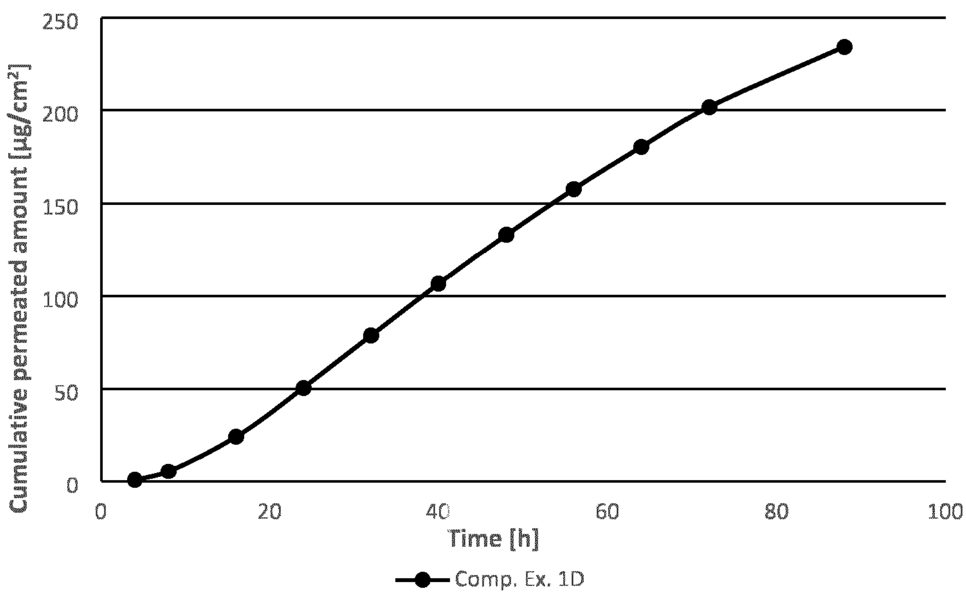
Cumulative permeated amount [µg/cm²]
250
200
150
100
50
0
0
20
40
60
80
100
Time [h]
Comp. Ex. 1D

**Fig. 1.3**

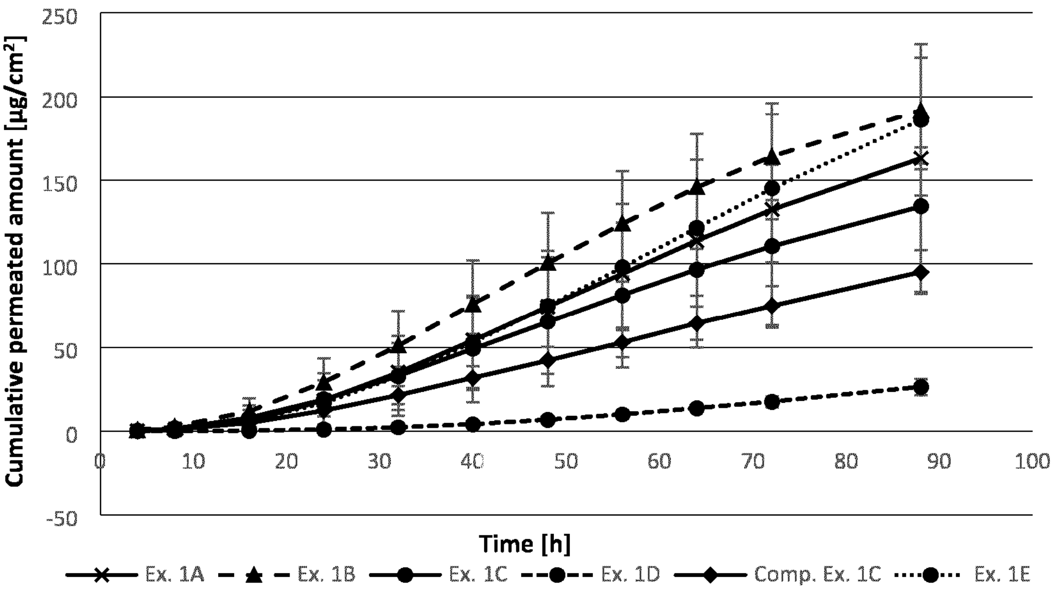
Cumulative permeated amount [µg/cm²]
250
200
150
100
50
0
-50
0
10
20
30
40
50
60
70
80
90
100
Time [h]
Ex. 1A
Ex. 1B
Ex. 1C
Ex. 1D
Comp. Ex. 1C
Ex. 1E

Fig. 2

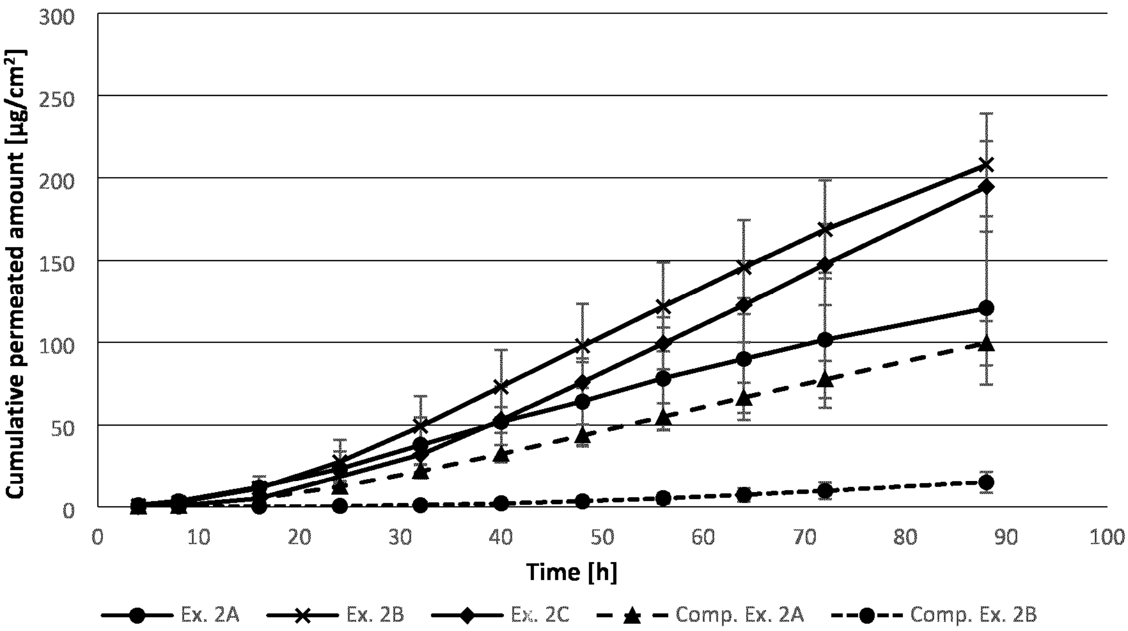
Cumulative permeated amount [µg/cm²]
300
250
200
150
100
50
0
0
10
20
30
40
50
60
70
80
90
100
Time [h]
Ex. 2A
Ex. 2B
Ex. 2C
Comp. Ex. 2A
Comp. Ex. 2B

Fig. 3

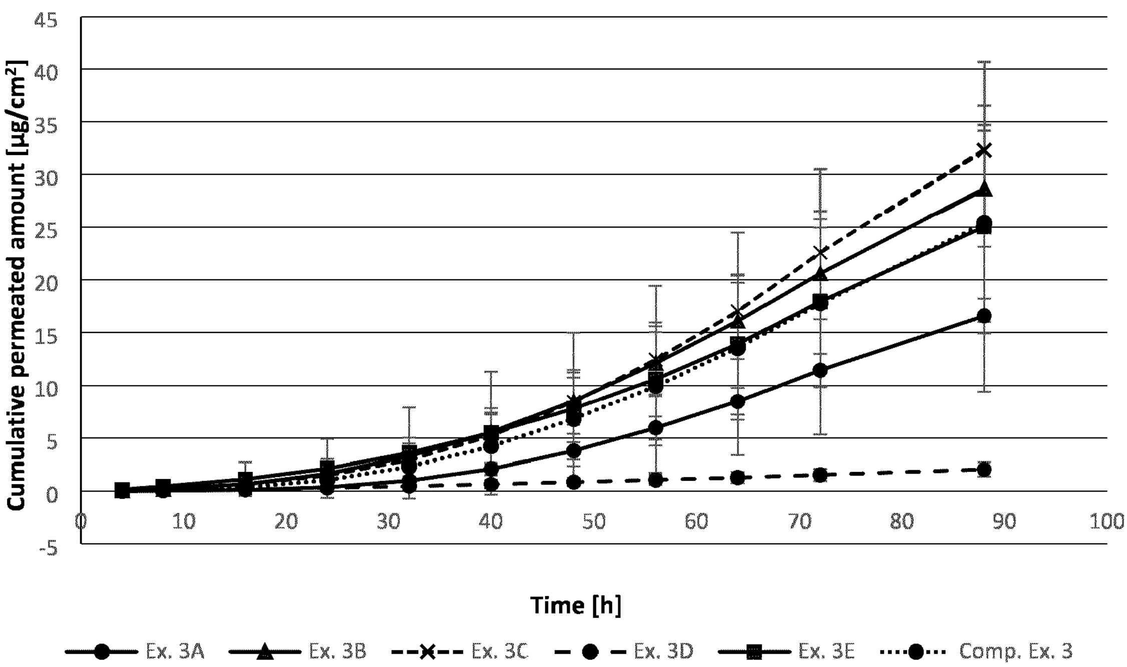
Cumulative permeated amount [µg/cm²]
45
40
35
30
25
20
15
10
5
0
-5
0
10
20
30
40
50
60
70
80
90
100
Time [h]
Ex. 3A
Ex. 3B
Ex. 3C
Ex. 3D
Ex. 3E
Comp. Ex. 3

Fig. 4.1

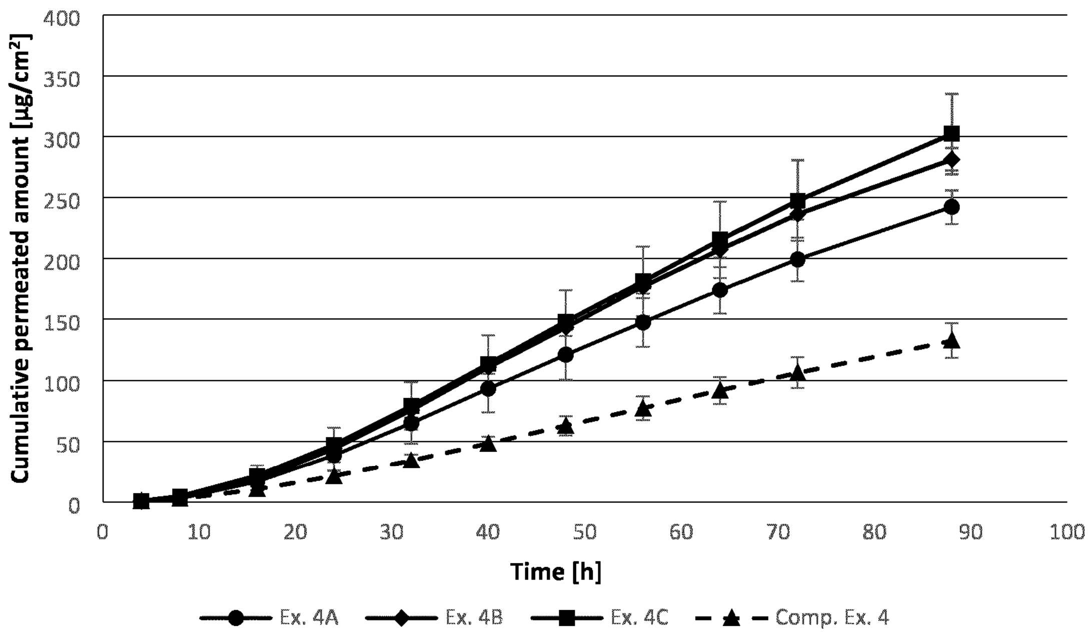
Cumulative permeated amount [µg/cm²]
400
350
300
250
200
150
100
50
0
0
10
20
30
40
50
60
70
80
90
100
Time [h]
Ex. 4A
Ex. 4B
Ex. 4C
Comp. Ex. 4

Fig. 4.2

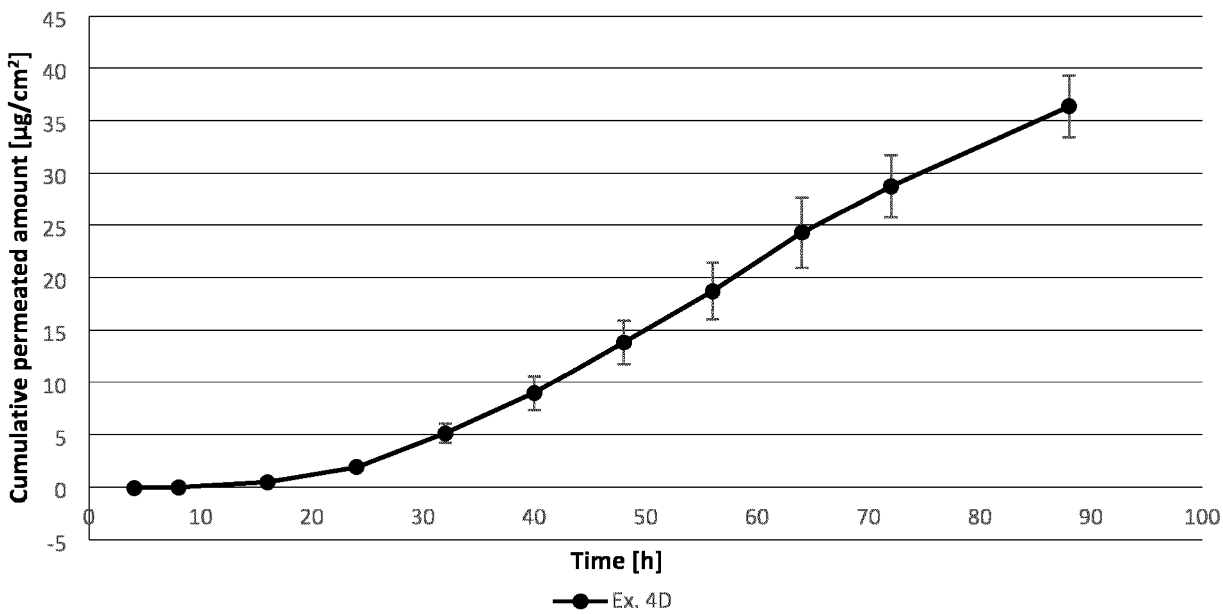
Cumulative permeated amount [µg/cm²]
45
40
35
30
25
20
15
10
5
0
-5
0
10
20
30
40
50
60
70
80
90
100
Time [h]
Ex. 4D

Fig. 5.1

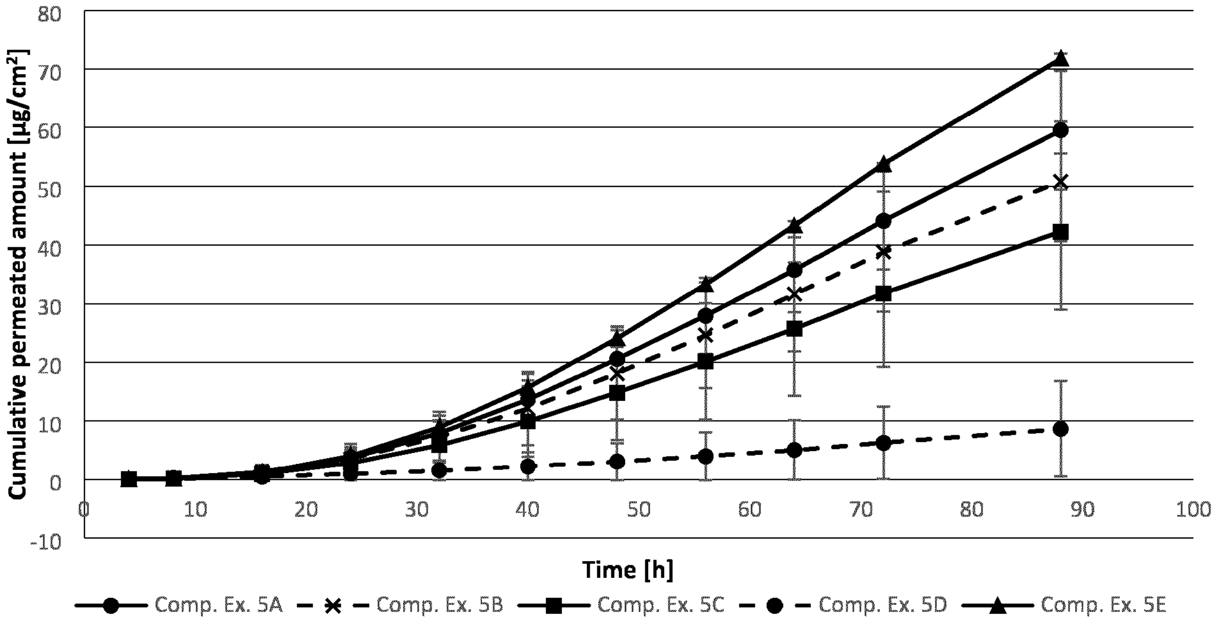
Cumulative permeated amount [µg/cm²]
80
70
60
50
40
30
20
10
0
-10
0
10
20
30
40
50
60
70
80
90
100
Time [h]
Comp. Ex. 5A
Comp. Ex. 5B
Comp. Ex. 5C
Comp. Ex. 5D
Comp. Ex. 5E

Fig. 5.2

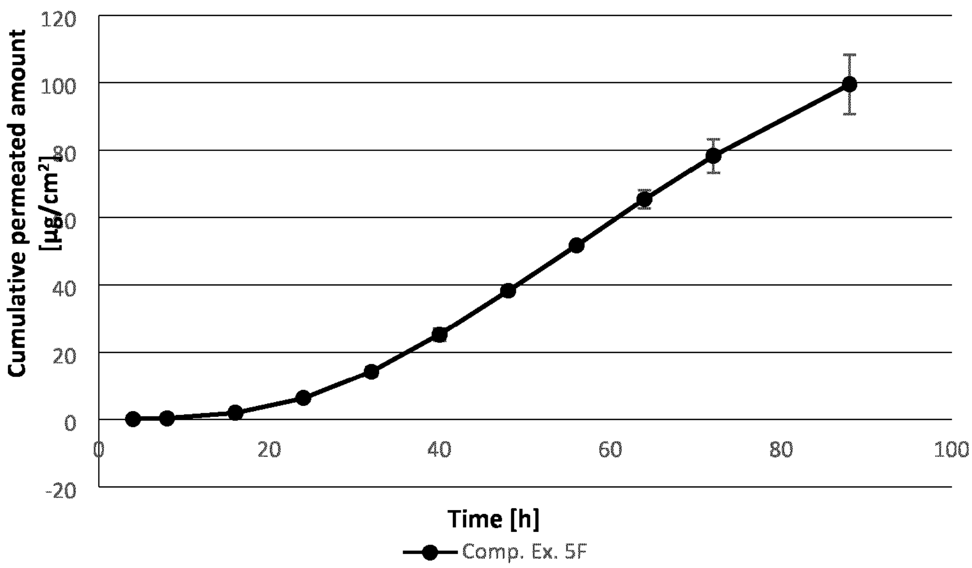
Cumulative permeated amount [µg/cm²]
120
100
80
60
40
20
0
-20
0
20
40
60
80
100
Time [h]
Comp. Ex. 5F

**Fig. 6**

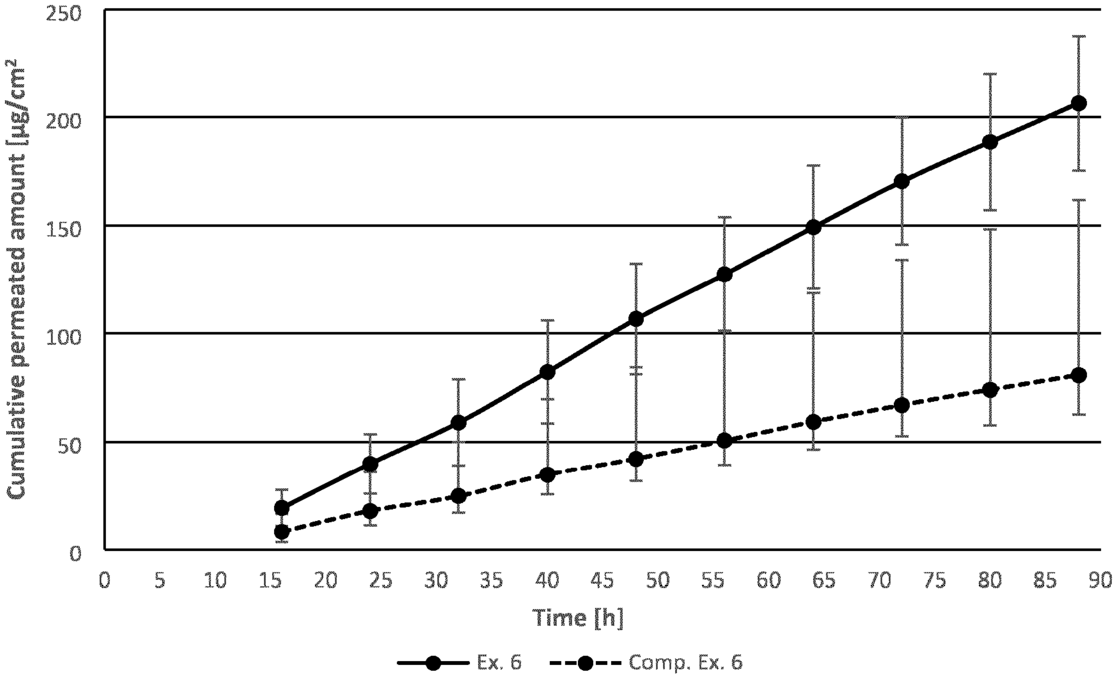
250
200
150
100
50
0
Cumulative permeated amount [µg/cm²]
0 5 10 15 20 25 30 35 40 45 50 55 60 65 70 75 80 85 90
Time [h]
Ex. 6
Comp. Ex. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019072998 A
- EP 2599847 A
- WO 2016130408 A
- WO 2007145996 A
- EP 2599847 A1
- WO 2010124187 A